# EUROPEAN PATENT APPLICATION

(11) **EP 4 610 287 A1**
(43) Date of publication of application: **03.09.2025**
(21) Application number: 23882614.3
(22) Date of filing: 24.10.2023
(51) Int. Cl.: C08F 283/00, B32B 5/28, B32B 15/08, C08J 5/24, C08K 3/013, C08K 5/49, C08L 65/00, C08L 79/04, H05K 1/03

(54) **RESIN COMPOSITION, CURED PRODUCT, PREPREG, METAL FOIL-CLAD LAMINATE, RESIN COMPOSITE SHEET, AND PRINTED WIRING BOARD**

(30) Priority: 27.10.2022 JP 2022172530; 23.12.2022 JP 2022207667; 16.01.2023 JP 2023004512
(71) Applicant: MITSUBISHI GAS CHEMICAL COMPANY, INC., Chiyoda-ku Tokyo 100-8324 (JP)
(72) Inventor: KOBAYASHI Takashi, Tokyo 125-8601 (JP); KAMATA Yuji, Tokyo 125-8601 (JP); HASEBE Keiichi, Tokyo 125-8601 (JP)
(74) Representative: Hoffmann Eitle
(86) International application number: PCT/JP2023/038271
(87) International publication number: WO 2024/090409

(57) **Abstract**

Provided are a resin composition that can provide a novel cured product, and the cured product, a prepreg, a metal foil-clad laminate, a resin composite sheet, and a printed wiring board. A resin composition contains a polymaleimide compound (A) and a cyanate ester compound (B) having, in a molecule, two or more aromatic moieties substituted with at least one cyanato group. The polymaleimide compound (A) contains, as reaction raw materials (1), an aromatic amine compound (a1) having one or more and three or less alkyl groups on an aromatic ring, an aromatic divinyl compound (a2) having two ethenyl groups, and maleic anhydride.

## Description

### Technical Field

The present invention relates to a resin composition, a cured product, a prepreg, a metal foil-clad laminate, a resin composite sheet, and a printed wiring board.

### Background Art

In recent years, semiconductors used in communication equipment, communication devices, personal computers, and the like have been highly integrated and miniaturized. Along with this trend, various properties required for laminates (e.g., metal foil-clad laminates) for semiconductor packages used in printed wiring boards used in these mentioned above are becoming increasingly severe. Examples of the required main properties include peel strength, low water absorption, chemical resistance, desmear resistance, flame resistance, low dielectric properties (low dielectric constant and/or low dielectric loss tangent), low thermal expansion, and heat resistance.

To obtain a printed wiring board improved in each of these properties, resin compositions used as materials for the printed wiring boards have been investigated. For example, Patent Document 1 discloses a resin composition for a printed wiring board containing a bifunctional vinylbenzyl compound (a) containing a predetermined polyphenylene ether skeleton, a predetermined maleimide compound (b), a predetermined cyanate ester resin (c), and a predetermined epoxy resin (d).

Patent Document 2 discloses a resin composition containing, as components, a predetermined cyanate ester compound (A), a predetermined polymaleimide compound (B), and a filler (C).

Further, Patent Document 3 discloses a resin composition containing a predetermined polymaleimide compound (A), a modified polyphenylene ether (B) terminal-modified with a substituent containing a carbon-carbon unsaturated double bond, and a filler (C).

### Citation List

### Patent Document

Patent Document 1: JP 2010-138364 A
Patent Document 2: WO 2016/072404 A
Patent Document 3: WO 2019/138992 A

### Summary of Invention

### Technical Problem

As described above, various resin compositions for printed wiring boards have been investigated. Along with recent technological innovations and increasing demand, a novel resin composition that can provide a cured product having low dielectric properties is required. In particular, a printed wiring board or the like containing the cured product may be treated with an alkali solution, and the cured product is required to have alkali resistance.

The present invention is intended to solve such problems, and an object thereof is to provide a novel resin composition that can provide a cured product having low dielectric properties and alkali resistance, and the cured product, a prepreg, a metal foil-clad laminate, a resin composite sheet, and a printed wiring board.

### Solution to Problem

As a result of the investigation by the present inventors under the above-mentioned problems, it has been found that a novel resin composition that can provide a cured product having low dielectric properties and alkali resistance can be obtained by using a predetermined maleimide compound and a predetermined cyanate ester compound in combination.

Specifically, the aforementioned issues have been solved by the following means:
<1> A resin composition containing a polymaleimide compound (A) and a cyanate ester compound (B), the cyanate ester compound (B) having, in a molecule, two or more aromatic moieties substituted with at least one cyanato group,
   wherein the polymaleimide compound (A) contains, as reaction raw materials (1), an aromatic amine compound (a1) having one or more and three or less alkyl groups on an aromatic ring, an aromatic divinyl compound (a2) having two ethenyl groups, and maleic anhydride.
<2> The resin composition according to <1>, wherein, a content of the polymaleimide compound (A) is from 1 to 90 parts by mass, relative to 100 parts by mass of a resin solid content in the resin composition.
<3> The resin composition according to <1> or <2>, a content of the cyanate ester compound (B) comprising, in a molecule, two or more aromatic moieties substituted with at least one cyanato group is from 1 to 90 parts by mass, relative to 100 parts by mass of a resin solid content in the resin composition.
<4> The resin composition according to any one of <1> to <3>, wherein the cyanate ester compound (B) having, in a molecule, two or more aromatic moieties substituted with at least one cyanato group contains at least one selected from the group consisting of a phenol novolac-type cyanate ester compound, a naphthol aralkyl-type cyanate ester compound, a naphthylene ether-type cyanate ester compound, a biphenyl aralkyl-type cyanate ester compound, a xylene resin-type cyanate ester compound, a trisphenol methane-type cyanate ester compound, an adamantane skeleton-type cyanate ester compound, a bisphenol M-type cyanate ester compound, and a bisphenol A-type cyanate ester compound.
<5> The resin composition according to any one of <1> to <4>, further containing one or more additional resin components (C) selected from the group consisting of a maleimide compound other than the polymaleimide compound (A), a compound containing a polymerizable carbon-carbon unsaturated double bond, an epoxy compound, a phenolic compound, an oxetane resin, a benzoxazine compound, and a thermoplastic elastomer.
<6> The resin composition according to any one of <1> to <5>, further containing a filler (D).
<7> The resin composition according to <6>, wherein the filler (D) contains one or more selected from the group consisting of silica, aluminum hydroxide, aluminum nitride, boron nitride, forsterite, titanium oxide, barium titanate, strontium titanate, and calcium titanate.
<8> The resin composition according to <6> or <7>, wherein a content of the filler (D) in the resin composition is from 1 to 1600 parts by mass, based on 100 parts by mass of a resin solid content in the resin composition.
<9> The resin composition according to any one of <1> to <8>, further containing from 0.5 to 30 parts by mass of a monomer or oligomer having an ethylenically unsaturated group, based on 100 parts by mass of the resin solid content in the resin composition.
<10> The resin composition according to any one of <1> to <9>, further containing a flame retardant, wherein the flame retardant contains a phosphorus-based flame retardant.
<11> The resin composition according to any one of <1> to <10>, wherein, a content of the polymaleimide compound (A) is from 1 to 90 parts by mass, relative to 100 parts by mass of a resin solid content in the resin composition;
   a content of the cyanate ester compound (B) comprising, in a molecule, two or more aromatic moieties substituted with at least one cyanato group is from 1 to 90 parts by mass, relative to 100 parts by mass of a resin solid content in the resin composition;
   the cyanate ester compound (B) comprising, in a molecule, two or more aromatic moieties substituted with at least one cyanato group comprises at least one selected from the group consisting of a phenol novolac-type cyanate ester compound, a naphthol aralkyl-type cyanate ester compound, a naphthylene ether-type cyanate ester compound, a biphenyl aralkyl-type cyanate ester compound, a xylene resin-type cyanate ester compound, a trisphenol methane-type cyanate ester compound, an adamantane skeleton-type cyanate ester compound, a bisphenol M-type cyanate ester compound, and a bisphenol A-type cyanate ester compound,
   the resin composition further comprises a filler (D),
   the filler (D) contains one or more selected from the group consisting of silica, aluminum hydroxide, aluminum nitride, boron nitride, forsterite, titanium oxide, barium titanate, strontium titanate, and calcium titanate, and
   a content of the filler (D) in the resin composition is from 1 to 1600 parts by mass, based on 100 parts by mass of the resin solid content in the resin composition.
<12> The resin composition according to any one of <1> to <11>, wherein the polymaleimide compound (A) contains, as reaction raw materials (3), an intermediate amine compound (C) in which the aromatic amine compounds (a1) are linked to each other via the aromatic divinyl compound (a2), and the maleic anhydride.
<13> The resin composition according to <12>, wherein an amine equivalent of the intermediate amine compound (C) is in a range from 172 to 400 g/equivalent.
<14> The resin composition according to any one of <1> to <13>, wherein the reaction raw materials (1) further include an aromatic monovinyl compound (a3) having one ethenyl group.
<15> The resin composition according to any one of <1> to <14>, wherein the polymaleimide compound (A) has a structural unit represented by the following Formula (1): 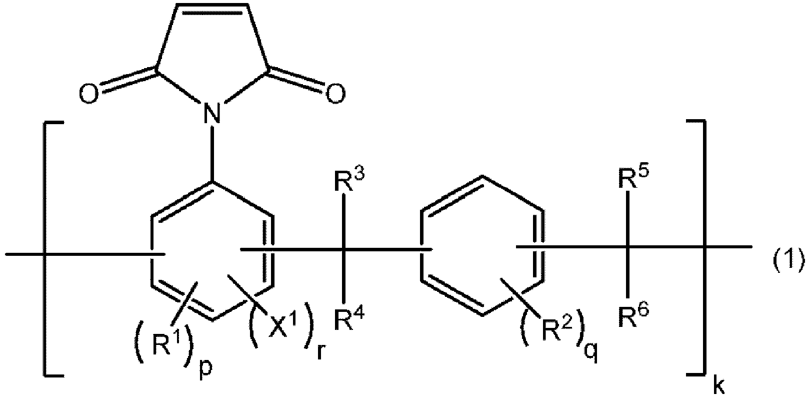 where in Formula (1), R¹ each independently represents an alkyl group having from 1 to 10 carbon atoms, R² each independently represents an alkyl group, an alkoxy group, or an alkylthio group each having from 1 to 10 carbon atoms; an aryl group, an aryloxy group, or an arylthio group each having from 6 to 10 carbon atoms; a cycloalkyl group having from 3 to 10 carbon atoms; a halogen atom; a hydroxyl group; or a mercapto group;
   R³, R⁴, R⁵, and R⁶ each independently represent a hydrogen atom or a methyl group, for R³ and R⁴, one is a hydrogen atom and the other is a methyl group, for R⁵ and R⁶, one is a hydrogen atom and the other is a methyl group;
   X¹ represents a substituent represented by the following Formula (x): 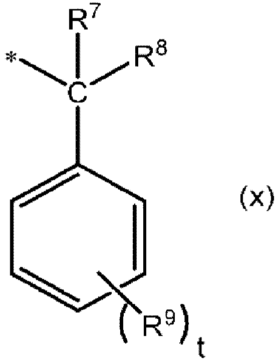 where in Formula (x), R⁷ and R⁸ each independently represent a hydrogen atom or a methyl group, for R⁷ and R⁸, one is a hydrogen atom and the other is a methyl group, R⁹ each independently represents an alkyl group, an alkoxy group, or an alkylthio group each having from 1 to 10 carbon atoms; an aryl group, an aryloxy group, or an arylthio group each having from 6 to 10 carbon atoms; a cycloalkyl group having from 3 to 10 carbon atoms; a halogen atom; a hydroxyl group; or a mercapto group, and t represents an integer from 0 to 4; and
   r is an average value of the numbers of X¹ substituents per benzene ring to which X¹ is bonded and represents a number from 0 to 4, p represents an integer from 1 to 3, q represents an integer from 0 to 4, and k represents an integer from 1 to 100.
<16> The resin composition according to any one of <1> to <15> for a printed wiring board.
<17> A cured product of the resin composition according to any one of <1> to <16>.
<18> A prepreg formed from a substrate and a resin composition according to any one of <1> to < 16>.
<19> A metal foil-clad laminate including at least one prepreg described in <18> and a metal foil disposed on one side or both sides of the prepreg.
<20> A resin composite sheet including: a support; and a layer disposed on a surface of the support, the layer formed from ae resin composition according to any one of <1> to <16>.
<21> A printed wiring board including an insulating layer and a conductor layer disposed on a surface of the insulating layer, wherein
   the insulating layer includes a layer formed from a resin composition according to any one of <1> to <16>.

### Advantageous Effects of Invention

The present invention has made it possible to provide a novel resin composition that can provide a cured product having low dielectric properties and alkali resistance, and the cured product, a prepreg, a metal foil-clad laminate, a resin composite sheet, and a printed wiring board.

### Description of Embodiments

The following sections describe the embodiments for implementing the present invention (hereinafter, may be referred to as "the present embodiment") in detail. It should be noted that the present embodiments described below are illustrative examples and do not limit the scope of the present invention.

A numerical range "from A to B" as used herein includes both endpoints, "A" and "B," as the lower and upper limits, respectively.

As used herein, various physical property values and characteristic values are those at 23°C unless otherwise stated.

In a notation of a group (atomic group) in the present specification, a notation that does not indicate substitution or no substitution is meant to include a group (atomic group) with a substituent as well as a group (atomic group) with no substituent. For example, an "alkyl group" includes not only an alkyl group with no substituent (unsubstituted alkyl group) but also an alkyl group with a substituent (substituted alkyl group). The "vinyl group" similarly includes not only a vinyl group with no substituent (unsubstituted vinyl group) but also a vinyl group with a substituent (substituted vinyl group). In the present specification, a notation that does not indicate substitution or no substitution preferably means unsubstituted one.

In the present specification, (meth)allyl represents both or any of allyl and methallyl, and is preferably allyl. (Meth)acryloyl represents both or any of acryloyl and methacryloyl, and is preferably methacryloyl.

In the present specification, a relative permittivity indicates a ratio of a dielectric constant to the vacuum dielectric constant of a substance. In the present specification, the relative permittivity may be simply referred to as a "dielectric constant".

"Parts by mass" in the present specification indicates a relative amount of a component, and "mass%" indicates an absolute amount of a component.

In the present specification, a resin solid content refers to components excluding a filler and a solvent, and is intended to include: a polymaleimide compound (A); a cyanate ester compound (B) having, in a molecule, two or more aromatic moieties substituted with at least one cyanato group; and an additional resin component (C), a monomer or oligomer having an ethylenically unsaturated group, a silane coupling agent, and other components (additives such as a dispersant and a flame retardant), which are blended as necessary.

When the measurement methods or the like described in the standards shown in the present specification differ depending on the year, the measurement methods or the like are based on the standards as of January 1, 2022, unless otherwise stated.

The polymaleimide compound (A) includes a compound having one or more maleimide groups, and is preferably a compound having two or more maleimide groups.

The resin composition of the present embodiment contains the polymaleimide compound (A) and the cyanate ester compound (B) having, in a molecule, two or more aromatic moieties substituted with at least one cyanato group, and is characterized by the polymaleimide compound (A) containing, as reaction raw materials (1), an aromatic amine compound (a1) having one or more and three or less alkyl groups on an aromatic ring, an aromatic divinyl compound (a2) having two ethenyl groups, and maleic anhydride. With such a configuration, a novel resin composition that can provide a cured product having low dielectric properties and alkali resistance can be obtained.

### <Polymaleimide compound (A)>

The resin composition of the present embodiment contains the polymaleimide compound (A). By containing the polymaleimide compound (A), a cured product excellent in low dielectric properties and alkali resistance can be provided. In particular, compared to the case where, for example, a compound represented by Formula (M1-3) to be described later is used as the maleimide compound, a resin composition more excellent in alkali resistance can be obtained.

The polymaleimide compound (A) according to the present embodiment is a compound containing, as the reaction raw material (1), an aromatic amine compound (a1) having one or more and three or less alkyl groups (hereinafter, also referred to as an "aromatic amine compound (a1)"), an aromatic divinyl compound (a2) having two ethenyl groups (hereinafter, also referred to as an aromatic divinyl compound (a2)), and maleic anhydride. As a result, a small dimensional change rate (or excellent dimensional stability) can be achieved when cured, and a low moisture absorption rate and a low dielectric loss tangent of the cured product can both be achieved at a high level.

In the present embodiment, the reaction raw materials (1) may further include an aromatic monovinyl compound (a3) having one ethenyl group (hereinafter, also referred to as an aromatic monovinyl compound (a3)). In addition, the polymaleimide compound (A) of the present embodiment is preferably a polymaleimide compound containing, as reaction raw materials (3): an intermediate amine compound (C) in which aromatic amine compounds (a1) each having one or more and three or less alkyl groups are crosslinked via an aromatic divinyl compound (a2) having two ethenyl groups; and maleic anhydride. Furthermore, the intermediate amine compound (C) is preferably a compound containing, as reaction raw materials (2), the aromatic amine compound (a1) having one or more and three or less alkyl groups, the aromatic divinyl compound (a2) having two ethenyl groups, and an aromatic monovinyl compound (a3) having one ethenyl group that is added as necessary.

In other words, the intermediate amine compound (C) in the present embodiment preferably has a structure in which a structural unit of the aromatic amine compound (a1), having an aromatic ring to which amino groups (including substituted amino groups in which hydrogen atoms of amino groups are further substituted with alkyl groups having from 1 to 6 carbon atoms) are bonded and one or more and three or less alkyl groups on the aromatic ring, and a structural unit of the aromatic divinyl compound (a2) having two ethenyl groups are linked by a chemical bond, and if necessary, a structural unit of the aromatic monovinyl compound (a3) is chemically bonded to the aromatic ring in the structural unit of the aromatic amine compound (a1). The polymaleimide compound (A) in the present embodiment has a structure in which an amino group (including -NH₂ and a substituted amino group) bonded to the aromatic ring of the intermediate amine compound (C) is substituted with an N-substituted maleimide ring.

Therefore, the "polymaleimide compound (A)" in the present embodiment and the "intermediate amine compound (C)", which is a precursor of the "polymaleimide compound (A)", are different polymer compounds in that the amino group (including -NH₂ and a substituted amino group) bonded to the aromatic ring is replaced with an N-substituted maleimide ring.

The structural unit of the aromatic amine compound (a1) refers to a group obtained by removing two hydrogen atoms from the aromatic ring of the aromatic amine compound (a1). For example, when the aromatic amine compound (a1) is represented by the later-described Formula (a), a group obtained by removing two hydrogen atoms from the benzene ring of Formula (a) is referred to as the structural unit of the aromatic amine compound (a1). **In** addition, the structural unit of the aromatic divinyl compound (a2) refers to a group formed by cleavage of the unsaturated bond between the two ethenyl groups of the aromatic divinyl compound (a2).

**In** the present embodiment, the aromatic amine compound (a1) having a specific aromatic ring structure is used as a reaction raw material, and hence the reaction site with the later-described aromatic divinyl compound (a2) can be easily controlled. Therefore, the polymaleimide compound (A) having a uniform chemical structure or chain length can be easily obtained, and as a result, the polymaleimide compound (A), exhibiting a low moisture absorption rate and a low dielectric loss tangent after being cured, can be provided.

### <<Preferred form of polymaleimide compound (A)>>

It is preferable that the polymaleimide compound (A) in the present embodiment have a structural unit represented by the following Formula (1): 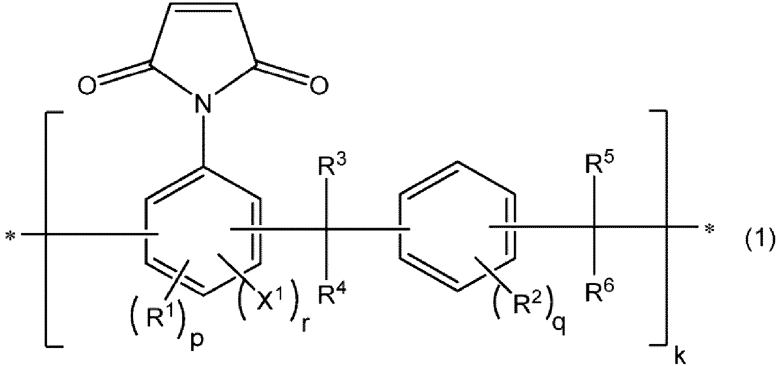 (In Formula (1), R¹ each independently represents an alkyl group, R² each independently represents an alkyl group, an alkoxy group, or an alkylthio group each having from 1 to 10 carbon atoms; an aryl group, an aryloxy group, or an arylthio group each having from 6 to 10 carbon atoms; a cycloalkyl group having from 3 to 10 carbon atoms; a halogen atom; a hydroxyl group; or a mercapto group,
R³, R⁴, R⁵, and R⁶ each independently represent a hydrogen atom or a methyl group, for R³ and R⁴, one is a hydrogen atom and the other is a methyl group, for R⁵ and R⁶, one is a hydrogen atom and the other is a methyl group,
X¹ represents a substituent represented by the following Formula (x): 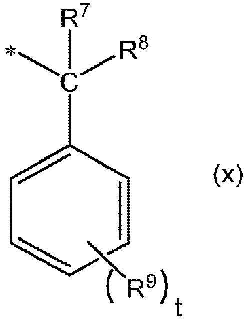 (In Formula (x), R⁷ and R⁸ each independently represent a hydrogen atom or a methyl group, for R⁷ and R⁸, one is a hydrogen atom and the other is a methyl group, R⁹ each independently represents an alkyl group, an alkoxy group, or an alkylthio group each having from 1 to 10 carbon atoms; an aryl group, an aryloxy group, or an arylthio group each having from 6 to 10 carbon atoms; a cycloalkyl group having from 3 to 10 carbon atoms; a halogen atom; a hydroxyl group; or a mercapto group, and t represents an integer from 0 to 4. * in Formula (1) represents a bond to another atom.)
r is an average value of the numbers of X¹ substitutions per benzene ring to which X¹ is bonded and represents a number from 0 to 4, p represents an integer from 1 to 3, q represents an integer from 0 to 4, and k represents an integer from 1 to 100.)

In the above-mentioned Formula (1), when p is an integer of 2 or more, a plurality of R¹s may be the same as or different from each other. When q is an integer of 2 or more, a plurality of R²s may be the same as or different from each other. When t is an integer of 2 or more, a plurality of R⁹s may be the same as or different from each other.

In Formula (1), R¹ each independently represents preferably an alkyl group having from 1 to 10 carbon atoms, more preferably an alkyl group having from 1 to 6 carbon atoms, even more preferably an alkyl group having from 1 to 3 carbon atoms, and may be an alkyl group having from 1 to 2 carbon atoms. When p is an integer of 2 or more, a plurality of R¹s may be the same as or different from each other. Preferred R¹ in Formula (1) is a methyl group, an ethyl group, or an n-propyl group, and may be a methyl group and/or an ethyl group. The benzene ring, to which R¹ is bonded in Formula (1), can be the benzene ring of the aromatic amine compound (a1).

In Formula (1), p represents preferably 1 or 2, and more preferably 1. When p is 1, the molecular weight distribution of the resulting polymaleimide compound (A) can be increased, and the moisture absorption rate and the dimensional change rate of the resulting cured product tend to be reduced.

In the benzene ring in Formula (1), to which R¹ is bonded, R¹ is bonded at preferably at least one of the 2-, 3-, 4-, 5-, or 6-positions relative to the maleimide group, R¹ is bonded at more preferably at least one of the 2-, 3-, 4-, or 6-positions, and R¹ is bonded even more preferably at the 2- and/or 6-positions. When R¹ is bonded at the 2- and/or 6-positions relative to the maleimide group, there is a tendency that low dielectric properties (low dielectric constant and/or low dielectric loss tangent) are excellent. When p is 2, R¹s are bonded preferably at the 2- and 6-positions or at the 2- and 3-positions relative to the maleimide group. Specific examples thereof include an aspect in which methyl groups are bonded at the 2- and 6-positions relative to the maleimide group. When p is 1, R¹ is preferably bonded at the 2-position relative to the maleimide group. Specific examples thereof include an aspect in which an ethyl group is bonded to the 2-position of the maleimide group.

In Formula (1), R² each independently represents preferably an alkyl group having from 1 to 10 carbon atoms, an alkoxy group having from 1 to 10 carbon atoms; an aryl group having from 6 to 10 carbon atoms; a cycloalkyl group having from 3 to 10 carbon atoms; a halogen atom; or a hydroxyl group, more preferably an alkyl group having from 1 to 10 carbon atoms, and even more preferably an alkyl group having from 1 to 6 carbon atoms. When q is an integer of 2 or more, a plurality of R²s may be the same as or different from each other. Preferred R² in Formula (1) is a methyl group, an ethyl group, or an n-propyl group. The benzene ring in Formula (1), to which R² is bonded, can be the benzene ring of the aromatic divinyl compound (a2). In Formula (1), q preferably represents 0, 1 or 2.

In Formula (1), for R³ and R⁴, one is a hydrogen atom and the other is a methyl group, and for R⁵ and R⁶, one is a hydrogen atom and the other is a methyl group. As a result, the reactivity of the unsaturated bond the polymaleimide compound (A) itself has can be maintained at a high level. In R³, R⁴, R⁵, and R⁶ in Formula (1), when the proportion of the alkyl group is increased, there is a tendency for the reactivity of the unsaturated bond the polymaleimide compound (A) itself has to be possibly decreased due to its steric hindrance. Therefore, when all of R³, R⁴, R⁵, and R⁶ are alkyl groups, the reactivity of the unsaturated bond the polymaleimide compound (A) itself has decreases, and a cured product cannot be efficiently formed. As a result, this can be a cause of deteriorated dimensional change rate.

In Formula (1), X¹ is represented by the above-mentioned Formula (x). In Formula (x), it is preferable that R⁷ represents a hydrogen atom and R⁸ represents a methyl group. In Formula (x), R⁹ each independently represents preferably an alkyl group having from 1 to 10 carbon atoms, an alkoxy group having from 1 to 10 carbon atoms; an aryl group having from 6 to 10 carbon atoms; a cycloalkyl group having from 3 to 10 carbon atoms; a halogen atom; or a hydroxyl group, more preferably an alkyl group having from 1 to 10 carbon atoms or an aryl group having from 6 to 10 carbon atoms, even more preferably an alkyl group having from 1 to 6 carbon atoms or an aryl group having from 6 to 10 carbon atoms, still preferably an alkyl group having from 1 to 10 carbon atoms, and yet more preferably an alkyl group having from 1 to 6 carbon atoms. **In** Formula (x), t represents an integer from 0 to 4, preferably an integer from 0 to 3, and more preferably an integer from 0 to 2. When t is an integer of 2 or more, a plurality of R⁹s may be the same as or different from each other.

**In** Formula (1), r means an average value of the numbers of X¹ substitutions per benzene ring to which X¹ is bonded, and is a number in the range from 0 to 4, more preferably a number in the range from 0 to 3, and may be a number in the range from 0 to 2.

**In** Formula (1), k represents the number of repeating units, and is an integer from 1 to 100, preferably an integer from 1 to 90, more preferably an integer from 1 to 80, and may be an integer from 1 to 50 or an integer from 1 to 20.

The polymaleimide compound (A) in the present embodiment contains an indane skeleton (or a structural unit having an indane skeleton) in an amount of preferably 10 mass% or less, more preferably 5 mass% or less, even more preferably 3 mass% or less, still more preferably 2 mass% or less, and particularly preferably 0.9 mass% or less, based on the total amount (100 mass%) of the polymaleimide compound (A).

The structural unit having an indane skeleton is preferably represented by the following Formula (3): 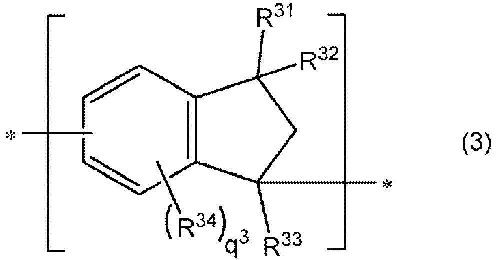 (In the above-mentioned Formula (3), R³¹, R³², and R³³ each independently represent a hydrogen atom or an alkyl group having from 1 to 3 carbon atoms; R³⁴ each independently represents an alkyl group, an alkoxy group, or an alkylthio group each having from 1 to 10 carbon atoms; an aryl group, an aryloxy group, or an arylthio group each having from 6 to 10 carbon atoms; a cycloalkyl group having from 3 to 10 carbon atoms; a halogen atom; a hydroxyl group; or a mercapto group, q³ represents an integer from 0 to 3, and when q³ is an integer of 2 or more, a plurality of R³⁴s may be the same as or different from each other. * represents a bond to another atom.)

In Formula (3), R³⁴ each independently represents preferably an alkyl group having from 1 to 6 carbon atoms, and more preferably an alkyl group having from 1 to 3 carbon atoms. In Formula (3), R³¹, R³², and R³³ are preferably hydrogen atoms or methyl groups.

Hereinafter, the aromatic amine compound (a1) having one or more and three or less alkyl groups, the aromatic divinyl compound (a2) having two ethenyl groups, the aromatic monovinyl compound (a3) having one ethenyl group that can be an optional component, and maleic anhydride, which are constituent components of the reaction raw materials (1) of the polymaleimide compound (A), will be described, and then another preferred embodiment for the polymaleimide compound (A) and a method for manufacturing the polymaleimide compound (A) will be described.

### <<Aromatic amine compound (a1)>>

The aromatic amine compound (a1) in the present embodiment has an aromatic ring to which an amino group (-NH₂ or substituted amino group) is bonded, and one or more and three or less alkyl groups are bonded to the aromatic ring. Therefore, the aromatic amine compound (a1) can be an amine-based compound. In addition, the aromatic ring forming the central structure of the aromatic amine compound (a1) is preferably monocyclic, and examples thereof include an aromatic hydrocarbon ring and an aromatic heterocyclic ring. The aromatic hydrocarbon ring is preferably a benzene ring. Examples of the aromatic heterocyclic ring include a hetero six-membered ring such as a pyran ring or a pyridine ring. In addition, it is more preferable that the aromatic amine compound (a1) in the present embodiment has an aromatic ring to which -NH₂ not containing a substituted amino group is bonded, and one or more and three or less alkyl groups are bonded to the aromatic ring.

In the aromatic amine compound (a1) of the present embodiment, examples of the alkyl group that substitutes one or more and three or less hydrogen atoms on the aromatic ring of the aromatic amine compound (a1) include an alkyl group having from 1 to 10 carbon atoms, preferably an alkyl group having from 1 to 6 carbon atoms, more preferably an alkyl group having from 1 to 3 carbon atoms, and may be an alkyl group having from 1 to 2 carbon atoms. The alkyl group may be linear, branched, or cyclic. Examples thereof include a methyl group, an ethyl group, a propyl group, an isopropyl group, a butyl group, an isobutyl group, an s-butyl group, and a t-butyl group. As the molecular weight of the alkyl group decreases, an effect (low dimensional change rate) exhibited by the present invention becomes more remarkable. In addition, as the molecular weight of the alkyl group increases, an effect (low moisture absorption) exhibited by the present invention becomes more remarkable.

The upper limit of the number of the alkyl groups bonded to the aromatic ring having an amino group (including -NH₂ and a substituted amino group) in the aromatic amine compound (a1) may be a number obtained by subtracting 3 from the number of substitutable ring-constituting atoms on the aromatic ring in an unsubstituted state, from the viewpoint that the aromatic ring has an amino group (including -NH₂ and a substituted amino group) and two bonds are used for polymerization. When the aromatic ring is, for example, a benzene ring, the number of the alkyl groups is 3 or less.

In addition, by setting the number of the alkyl groups substituted on the aromatic ring of the aromatic amine compound (a1) to 2 or more, the reaction site with the later-described aromatic divinyl compound (a2) is easily controlled, and hence the polymaleimide compound (A) having a uniform chemical structure or chain length can be easily obtained. As a result, the cured product of the polymaleimide compound (A) easily exhibits low moisture absorbency and excellent high-frequency electrical properties.

A preferred embodiment of the aromatic amine compound (a1) will be described by taking, as an example, a case where the aromatic ring of the aromatic amine compound (a1) in the present embodiment is a benzene ring.

In the present embodiment, it is preferable that, among the carbon atoms in the benzene ring constituting the aromatic amine compound (a1), one or more carbon atoms, each having the highest HOMO electron density (Hückel coefficient), are unsubstituted (or substituted with hydrogen atoms).

This makes it easy to control an ArS_{E} reaction and molecular design by the later-described cathionoid reagent formed from the aromatic divinyl compound (a2). In more detail, when, among the carbon atoms in the benzene ring constituting the aromatic amine compound (a1), the carbon atom having the highest HOMO electron density (Hückel coefficient) is unsubstituted, a carbocation of the aromatic divinyl compound (a2), which is a cathionoid reagent, is likely to react with the carbon atom having the highest HOMO electron density. Therefore, by controlling the number, positions, and the like of the alkyl groups to be bonded to the carbon atoms of the benzene ring, the binding sites to the aromatic divinyl compound (a2), the number of bonds, and the like can be adjusted. Therefore, it is presumed that the chemical structure or molecular chain length of the resulting polymaleimide compound (A) can be easily designed.

For example, when the aromatic amine compound (a1) has an aniline skeleton having a single benzene ring and a single amino group, it is preferable that at least one carbon atom at the 2-, 4-, or 6-position of the aniline nucleus is substituted with a hydrogen atom. As a result, the later-described carthionoid reagent formed from the aromatic divinyl compound (a2) easily attacks at least one carbon atom at the 2-, 4-, or 6-position, which are the ortho and para positions of the aniline nucleus each having a high electron density. In particular, when the aromatic amine compound (a1) having an aniline nucleus, in which an alkyl group is substituted at a specific position, is used, the binding site to the aromatic divinyl compound (a2) can be generally controlled, so that the polymaleimide compound (A) having a uniform chemical structure or chain length can be easily obtained. For example, when 2,6-dialkylamine is used as the aromatic amine compound (a1), it is considered that a large amount of the polymaleimide compound (A) bonded at its 4-position to the aromatic divinyl compound (a2) is obtained.

Specific examples of the aromatic amine compound (a1) of the present embodiment include dimethylaniline (2,3-Xylidine, 2,4-Xylidine, 2,6-Xylidine, 3,4-Xylidine, or 3,5-Xylidine), diethylaniline (2,3-diethylaniline, 2,4-diethylaniline, 2,6-diethylaniline, 3,4-diethylaniline, or 3,5-diethylaniline), diisopropylaniline (2,3-diisopropylaniline, 2,4-diisopropylaniline, 2,6-diisopropylaniline, 3,4-diisopropylaniline, or 3,5-diisopropylaniline), ethylmethylaniline (e.g., ethylmethylaniline in which one of each of the 2- and 3-positions, the 2- and 4-positions, the 2- and 6-positions, the 3- and 4-positions, or the 3- and 5-positions is a methyl group and the other is an ethyl group), cyclobutylaniline, cyclopentylaniline, cyclohexylaniline, o-, m-, or p-toluidine, o-, m-, or p-ethylaniline, o-, m-, or p-isopropylaniline, o-, m-, or p-propylaniline, o-, m-, or p-butylaniline, methylisopropylaniline (e.g., methylisopropylaniline in which one of each of the 2- and 3-positions, the 2- and 4-positions, the 2- and 6-positions, the 3- and 4-positions, or the 3- and 5-positions is a methyl group and the other is an isopropyl group), or ethylbutylaniline (e.g., ethylbutylaniline in which one of each of the 2- and 3-positions, the 2- and 4-positions, the 2- and 6-positions, the 3- and 4-positions, or the 3- and 5-positions is an ethyl group and the other is a butyl group). Examples of the butyl include n-butyl, tert-butyl, and sec-butyl. Note that the aromatic amine compound (a1) in the present embodiment may be used alone or in combination of two or more kinds thereof.

For example, in the case of a chemical structure in which a maleimide group is directly bonded to an unsubstituted benzene ring, like N-phenylmaleimide, the state in which the benzene ring and the 5-membered maleimide ring are aligned on the same plane is stable, and hence stacking is likely to occur and high crystallinity is exhibited. This can cause poor solubility in solvent. On the other hand, in the case of the present embodiment, when an alkyl group (e.g., a methyl group) is contained as a substituent for a benzene ring, like, for example, 2,6-dimethylaniline, the benzene ring and the 5-membered maleimide ring adopt a twisted conformation due to the steric hindrance of the methyl group, and stacking is less likely to occur. Therefore, crystallinity is reduced and solubility in solvent is improved, leading to a preferred aspect. However, if the steric hindrance is too large, there is also concern that the reactivity when maleimide is synthesized may be inhibited. Therefore, it is preferable to use the aromatic amine compound (a1) having, for example, an alkyl group having from 1 to 6 carbon atoms.

The aromatic amine compound (a1), which is essential for the reaction raw materials (1) in the present embodiment, can be represented by, for example, the following Formula (a): 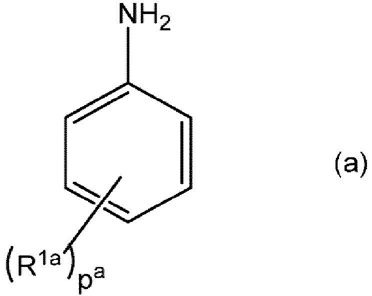 (In the above-mentioned Formula (a), R^{1a} each independently represents an alkyl group, and p^{a} represents an integer from 1 to 3. A plurality of R^{1a}s may be the same or different from each other.)

In Formula (a), the alkyl group represents preferably an alkyl group having from 1 to 10 carbon atoms, more preferably an alkyl group having from 1 to 6 carbon atoms, even more preferably an alkyl group having from 1 to 3 carbon atoms, and may be an alkyl group having from 1 to 2 carbon atoms. Examples of each of the alkyl group having from 1 to 6 carbon atoms, the alkyl group having from 1 to 3 carbon atoms, and the alkyl group having from 1 to 2 carbon atoms are the same as those described above.

In Formula (a), p^{a} is preferably 1 or 2. When there are a plurality of R^{1a}s, they may be the same or different alkyl groups.

Note that, in the present embodiment, the aromatic amine compound (a1) represented by Formula (a) may be used alone or in combination of two or more kinds thereof.

### <<Aromatic divinyl compound (a2)>>

The aromatic divinyl compound (a2) in the present embodiment can be used without particular limitation as long as it has two ethenyl groups (CH₂=CH-) (also referred to as a vinyl group) as substituents on the aromatic ring and can react with the aromatic amine compound (a1). In the present embodiment, the reaction raw materials (1) preferably include a mixture of the aromatic divinyl compound (a2) and the aromatic monovinyl compound (a3).

Examples of the aromatic divinyl compound (a2) include divinylbenzene, divinylbiphenyl, divinylnaphthalene, and various compounds in which one or more substituents, such as: alkyl groups, alkoxy groups, or alkylthio groups each having from 1 to 10 carbon atoms; aryl groups, aryloxy groups, or arylthio groups each having from 6 to 10 carbon atoms; cycloalkyl groups having from 3 to 10 carbon atoms; halogen atoms; hydroxyl groups; or mercapto groups, are substituted on the aromatic rings thereof. A preferred embodiment of the substituent is the same as that of R² in Formula (1). In addition, the alkyl group may be linear or branched. Among them, the number of carbon atoms of the alkyl group or alkoxy group is preferably from 1 to 4 from the viewpoint of exhibiting the effect of high heat resistance. Specific examples of the alkyl group include a methyl group, an ethyl group, a propyl group, an isopropyl group, a butyl group, a t-butyl group, and an isobutyl group. Examples of the alkoxy group include a methoxy group, an ethoxy group, a propyloxy group, and a butoxy group. Examples of the halogen atom include a fluorine atom, a chlorine atom, and a bromine atom.

The aromatic divinyl compound (a2), which is the reaction raw material (1) of the polymaleimide compound (A) in the present embodiment, is preferably represented by the following Formula (b1): 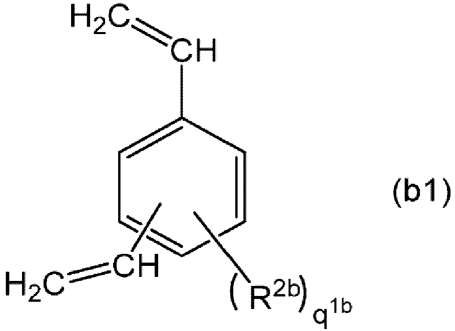 (In Formula (b1), R^{2b} each independently represents an alkyl group, an alkoxy group, or an alkylthio group each having from 1 to 10 carbon atoms; an aryl group, an aryloxy group, or an arylthio group each having from 6 to 10 carbon atoms; a cycloalkyl group having from 3 to 10 carbon atoms; a halogen atom; a hydroxyl group; or a mercapto group, and q^{1b} represents an integer from 0 to 4. Note that when q^{1b} is an integer of 2 or more, a plurality of R^{2b}s may be the same as or different from each other.)

R^{2b} in Formula (b1) can correspond to R² in Formula (1). Therefore, similarly to R² in Formula (1), R^{2b} in Formula (b1) each independently represents preferably an alkyl group having from 1 to 10 carbon atoms, an alkoxy group having from 1 to 10 carbon atoms; an aryl group having from 6 to 10 carbon atoms; a cycloalkyl group having from 3 to 10 carbon atoms; a halogen atom; or a hydroxyl group, more preferably an alkyl group having from 1 to 10 carbon atoms, and even more preferably an alkyl group having from 1 to 6 carbon atoms.

In Formula (b1), q^{1b} is preferably an integer from 0 to 2. Note that when q^{1b} is an integer of 2 or more, a plurality of R^{b1}s may be the same group or different groups.

Specific examples of the aromatic divinyl compound (a2) of the present embodiment include, but are not limited to, divinylbenzenes such as 1,2-divinylbenzene, 1,3-divinylbenzene, 1,4-divinylbenzene, 2,5-dimethyl-1,4-divinylbenzene, 2,5-diethyl-1,4-divinylbenzene, cis, cis, β, β'-diethoxy-m-m-divinylbenzene, 1,4-divinyl-2,5-dibutylbenzene, 1,4-divinyl-2,5-dihexylbenzene, 1,4-divinyl-2,5-dimethoxybenzene, and compounds composed of derivatives thereof; and divinylnaphthalenes such as 1,3-divinylnaphthalene, 1,4-divinylnaphthalene, 1,5-divinylnaphthalene, 1,6-divinylnaphthalene, 1,7-divinylnaphthalene, 2,3-divinylnaphthalene, 2,6-divinylnaphthalene, 2,7-divinylnaphthalene, 3,4-divinylnaphthalene, 1,8-divinylnaphthalene, 1,5-dimethoxy-4,8-divinylnaphthalene, and compounds composed of derivatives thereof.

Note that the aromatic divinyl compound (a2) in the present embodiment may be used alone or in combination of two or more kinds thereof.

In particular, from the viewpoint of fluidity, divinylbenzene and a compound having a substituent on the aromatic ring thereof are preferred as the aromatic divinyl compound (a2), and divinylbenzene is more preferred. In the present embodiment, the substitution position of the vinyl group of the divinylbenzene is not particularly limited, but it is preferable for a meta form to be the main component. The content of the meta form in the divinylbenzene is preferably 40 mass% or more, and more preferably 50 mass% or more, based on the total amount of the divinylbenzene.

In the present embodiment, the content of the structural unit of the aromatic divinyl compound (a2) is preferably from 10 to 90 mass%, and more preferably from 20 to 90 mass%, based on the total amount (100 mass%) of the polymaleimide compound (A). The structural unit of the aromatic divinyl compound (a2) refers to a group obtained by removing two hydrogen atoms (four hydrogen atoms in total) from each of the two ethenyl groups of the aromatic divinyl compound (a2).

### <<Aromatic monovinyl compound (a3)>>

In the present embodiment, the polymaleimide compound (A) may use, as reaction raw materials, the aromatic amine compound (a1), the aromatic divinyl compound (a2), and maleic anhydride, as well as other compounds. Examples of the other compounds include an aromatic monovinyl compound (a3) having a single ethenyl group. That is, it is preferable in the present embodiment to use, as the reaction raw materials (1), the aromatic amine compound (a1), the aromatic divinyl compound (a2), the aromatic monovinyl compound (a3), and maleic anhydride. The use of the aromatic monovinyl compound (a3) in addition to the aromatic amine compound (a1), the aromatic divinyl compound (a2), and maleic anhydride, as the reaction raw materials of the polymaleimide compound (A) in the present embodiment, is preferable because a cured product of the finally resulting polymaleimide compound (A) is excellent in low dielectric loss tangent.

In addition, the aromatic monovinyl compound (a3) also produces carbocations similarly to the aromatic divinyl compound (a2), and hence the aromatic monovinyl compound (a3) easily reacts with the carbon atom having the highest HOMO electron density (Hückel coefficient) among the carbon atoms in the aromatic hydrocarbon ring constituting the aromatic amine compound (a1).

Examples of the aromatic monovinyl compound (a3) in the present embodiment include vinylbenzene (styrene), vinylbiphenyl, vinylnaphthalene, and various compounds in which one or more substituents, such as: alkyl groups, alkoxy groups, or alkylthio groups each having from 1 to 10 carbon atoms; aryl groups, aryloxy groups, or arylthio groups each having from 6 to 10 carbon atoms; cycloalkyl groups having from 3 to 10 carbon atoms; halogen atoms; hydroxyl groups; or mercapto groups, are substituted on the aromatic rings thereof. The alkyl group may be linear or branched, and may have an unsaturated bond in its structure. **In** particular, when low moisture absorbency is emphasized, the alkyl group or the alkoxy group preferably has from 1 to 4 carbon atoms. Specific examples of the alkyl group include a methyl group, an ethyl group, a propyl group, an isopropyl group, a butyl group, a t-butyl group, and an isobutyl group. Examples of the alkoxy group include a methoxy group, an ethoxy group, a propyloxy group, and a butoxy group. Examples of the halogen atom include a fluorine atom, a chlorine atom, and a bromine atom.

**In** the present embodiment, the aromatic monovinyl compound (a3), which can be the reaction raw material (1) of the polymaleimide compound (A), can be represented by the following Formula (b2): 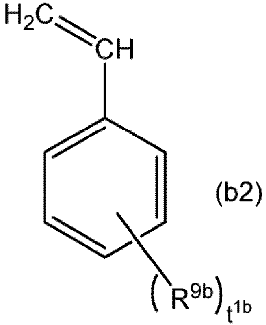 (In the above-mentioned Formula (b2), R^{9b} each independently represents an alkyl group, an alkoxy group, or an alkylthio group each having from 1 to 10 carbon atoms; an aryl group, an aryloxy group, or an arylthio group each having from 6 to 10 carbon atoms; a cycloalkyl group having from 3 to 10 carbon atoms; a halogen atom; a hydroxyl group; or a mercapto group, and t^{1b} represents an integer from 0 to 5. Note that when t^{1b} is an integer of 2 or more, a plurality of R^{b2}s may be the same as or different from each other.)

R^{9b} in Formula (b2) can correspond to R⁹ in Formula (x). Therefore, similarly to R⁹ in Formula (x), R^{9b} in Formula (b1) each independently represents preferably an alkyl group having from 1 to 10 carbon atoms, an alkoxy group having from 1 to 10 carbon atoms; an aryl group having from 6 to 10 carbon atoms; a cycloalkyl group having from 3 to 10 carbon atoms; a halogen atom; or a hydroxyl group, more preferable an alkyl group having from 1 to 10 carbon atoms or an aryl group having from 6 to 10 carbon atoms, even more preferably an alkyl group having from 1 to 6 carbon atoms or an aryl group having from 6 to 10 carbon atoms, still more preferably an alkyl group having from 1 to 10 carbon atoms, and yet more preferably an alkyl group having from 1 to 6 carbon atoms.

In Formula (b1), t^{1b} is preferably from 1 to 4. Note that when t^{1b} is 2 or more, a plurality of R^{9b}s may be the same group or different groups.

Specific examples of the aromatic monovinyl compound (a3) of the present embodiment include, but are not limited to, vinylbenzenes such as styrene, fluorostyrene, vinylbenzyl chloride, alkylvinylbenzene (o-, m-, p-methylstyrene, o-, m-, p-ethylvinylbenzene), o-, m-, p-(chloromethyl)styrene, and compounds composed of derivatives thereof; biphenyl compounds such as 4-vinylbiphenyl, 4-vinyl-p-terphenyl, and compounds composed of derivatives thereof; and vinylnaphthalenes such as 1-vinylnaphthalene, 2-vinylnaphthalene, and compounds composed of derivatives thereof.

In particular, from the viewpoint of availability of raw materials, alkylvinylbenzene and a compound having a substituent on the aromatic ring thereof are preferred, and ethylvinylbenzene is more preferred.

The substitution positions of the vinyl group and ethyl group of ethylvinylbenzene are not particularly limited, but it is preferable for the meta form to be the main component. The content of the meta form in the ethylvinylbenzene is more preferably 40 mass% or more, and even more preferably 50 mass% or more, based on the total amount of the ethylvinylbenzene.

When the aromatic monovinyl compound (a3) is used as the reaction raw material (1) of the polymaleimide compound (A) in the present embodiment, the molar ratio ((a2)/(a3)) of the aromatic monovinyl compound (a3) to the aromatic divinyl compound (a2) in the reaction raw materials (1) is preferably from 99/1 to 50/50, and more preferably 98/2 to 70/30.

In the present embodiment, the content of the structural unit of the aromatic monovinyl compound (a3) is preferably from 0 to 40 mass%, and more preferably from 0 to 30 mass%, based on the total amount (100 mass%) of the polymaleimide compound (A). The structural unit of the aromatic monovinyl compound (a3) refers to a group obtained by removing two hydrogen atoms from one of the vinyl groups of the aromatic monovinyl compound (a3).

As the reaction raw materials containing the aromatic divinyl compound (a2) and the aromatic monovinyl compound (a3), commercially available products can be used. Examples thereof include "DVB-810 (a mixture of divinylbenzene/ethylstyrene (divinylbenzene/ethylstyrene = 81/19 (mol%))" and "DVB-570 (a mixture of divinylbenzene/ethylstyrene (divinylbenzene/ethylstyrene = 57/43 (mol%))" available from Nittetsu Chemical & Materials Co., Ltd.

### <<Maleic anhydride>>

In the present embodiment, maleic anhydride is an essential component of the reaction raw materials (1) of the polymaleimide compound (A), and is used in a reaction to maleimidate an amino group (including -NH₂ and a substituted amino group) derived from the aromatic amine compound (a1) as will be described later in the section of a method for manufacturing the polymaleimide compound (A).

### <<Preferred form of polymaleimide compound (A)>>

In the present embodiment, the polymaleimide compound (A) is preferably represented by the following Formula (2): 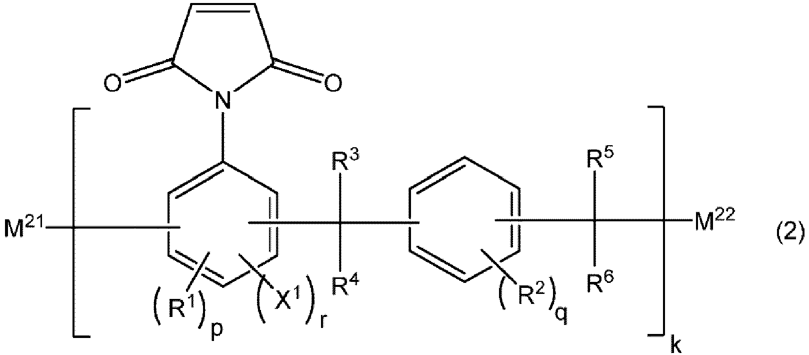 (In the above-mentioned Formula (2), R¹ each independently represents an alkyl group, R² each independently represents an alkyl group having from 1 to 10 carbon atoms, an alkoxy group having from 1 to 10 carbon atoms; an aryl group having from 6 to 10 carbon atoms; a cycloalkyl group having from 3 to 10 carbon atoms; a halogen atom; or a hydroxyl group, R³, R⁴, R⁵, and R⁶ each independently represent a hydrogen atom or a methyl group, for R³ and R⁴, one is a hydrogen atom and the other is a methyl group, for R⁵ and R⁶, one is a hydrogen atom and the other is a methyl group,
X¹ represents a substituent represented by the following Formula (x): 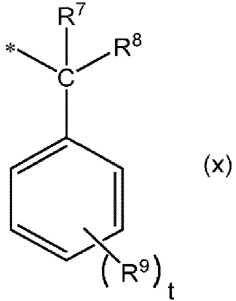 (In the above-mentioned Formula (x), R⁷ and R⁸ each independently represent a hydrogen atom or a methyl group, for R⁷ and R⁸, one is a hydrogen atom and the other is a methyl group, R⁹ each independently represents an alkyl group having from 1 to 10 carbon atoms, an alkoxy group having from 1 to 10 carbon atoms; an aryl group having from 6 to 10 carbon atoms; a cycloalkyl group having from 3 to 10 carbon atoms; a halogen atom; or a hydroxyl group, and t represents an integer from 0 to 4.),
M²¹ represents a hydrogen atom or a group represented by the following Formula (i), M²² represents a hydrogen atom, a group represented by the following Formula (ii), or a group represented by the following Formula (iii): 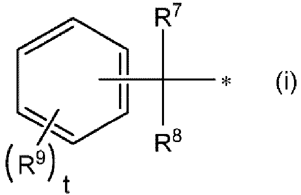 (In the above-mentioned Formula (i), R⁹ each independently represents an alkyl group having from 1 to 10 carbon atoms, an alkoxy group having from 1 to 10 carbon atoms; an aryl group having from 6 to 10 carbon atoms; a cycloalkyl group having from 3 to 10 carbon atoms; a halogen atom; or a hydroxyl group, R⁷ and R⁸ each independently represent a hydrogen atom or a methyl group, for R⁷ and R⁸, one is a hydrogen atom and the other is a methyl group, t represents an integer from 0 to 4, and * represents a bond to another atom. Note that when t is an integer of 2 or more, a plurality of R⁹s may be the same as or different from each other.), 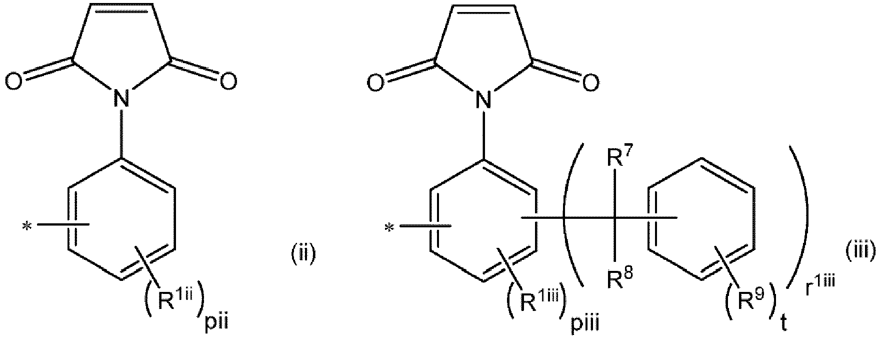 (In the above-mentioned Formula (ii), R¹ⁱⁱ represents an alkyl group, and pⁱⁱ represents an integer from 0 to 4. Note that when pⁱⁱ is an integer of 2 or more, a plurality of R¹ⁱⁱs may be the same as or different from each other. In the aforementioned Formula (iii), R¹ⁱⁱⁱ represents an alkyl group, R⁹ each independently represents an alkyl group having from 1 to 10 carbon atoms, an alkoxy group having from 1 to 10 carbon atoms; an aryl group having from 6 to 10 carbon atoms; a cycloalkyl group having from 3 to 10 carbon atoms; a halogen atom; or a hydroxyl group, R⁷ and R⁸ each independently represent a hydrogen atom or a methyl group, and for R⁷ and R⁸, one is a hydrogen atom and the other is a methyl group, pⁱⁱⁱ represents an integer from 0 to 3, t represents an integer from 0 to 4, r¹ⁱⁱⁱ is an average value of the numbers of substitutions per benzene ring to which X¹ is bonded and represents a number from 1 to 4, and * represents a bond to another atom. Note that when pⁱⁱⁱ is an integer of 2 or more, a plurality of R¹ⁱⁱⁱs may be the same as or different from each other, and when t is an integer of 2 or more, a plurality of R⁹s may be the same as or different from each other. r is an average value of the numbers of X¹ substituents per benzene ring to which X¹ is bonded and represents a number from 0 to 4, p represents an integer from 1 to 3, q represents an integer from 0 to 4, and k represents an integer from 1 to 100.),

In Formula (2), when p is an integer of 2 or more, a plurality of R¹s may be the same as or different from each other; when q is an integer of 2 or more, a plurality of R²s may be the same as or different from each other; and when t is an integer of 2 or more, a plurality of R⁹s may be the same as or different from each other.)

Note that preferred embodiments for R¹ to R⁹, X¹, p, q, r, t, and k in Formula (2) are the same as those in Formula (1). Furthermore, Formula (i) corresponds to Formula (x), R¹ⁱⁱ in Formula (ii) corresponds to R¹ in Formula (1), and R¹ⁱⁱⁱ in Formula (iii) corresponds to R¹ in Formula (1).

Hereinafter, a preferred aspect of the polymaleimide compound (A) of the present embodiment will be described. Examples of the polymaleimide compound (A) of the present embodiment include the following first to third embodiments. In the following embodiments, a preferred example of the aromatic divinyl compound (a2) is divinylbenzene, and a preferred example of the aromatic vinyl compound (a3) is ethylvinylbenzene. Preferred examples of an arylmaleimide structural unit are 2-ethylaniline and a structural unit derived from maleic anhydride.

The first embodiment of the polymaleimide compound (A) includes a compound in which the molar ratio of the total of the structural units derived from the aromatic divinyl compound (a2) and/or the aromatic vinyl compound (a3) to the arylmaleimide structural unit is n: n + 1.

Examples of the compound, in which the molar ratio of the total of the structural units derived from the aromatic divinyl compound (a2) and/or the aromatic vinyl compound (a3) to the arylmaleimide structural unit is n:n + 1, include
compounds of:
arylmaleimide structural unit-structural unit derived from aromatic divinyl compound (a2)-arylmaleimide structural unit; and
arylmaleimide structural unit-structural unit derived from aromatic divinyl compound (a2)-arylmaleimide structural unit-structural unit derived from aromatic divinyl compound (a2)-arylmaleimide structural unit.

The second embodiment of the polymaleimide compound (A) includes a compound in which the molar ratio of the total of the structural units derived from the aromatic divinyl compound (a2) and/or the aromatic monovinyl compound (a3) to the arylmaleimide structural unit is n:n.

Examples of the compound, in which the molar ratio of the total of the structural units derived from the aromatic divinyl compound (a2) and/or the aromatic monovinyl compound (a3) to the arylmaleimide structural unit is n:n, include
compounds of:
arylmaleimide structural unit-structural unit derived from aromatic monovinyl compound (a3); and
arylmaleimide structural unit-structural unit derived from aromatic divinyl compound (a2)-arylmaleimide structural unit-structural unit derived from aromatic monovinyl compound (a3).

The third embodiment of the polymaleimide compound (A) includes a compound in which the molar ratio of the total of the structural unit derived from the aromatic divinyl compound (a2) and the structural unit derived from the aromatic monovinyl compound (a3) to the arylmaleimide structural unit is n + 1:n.

Examples of the compound, in which the molar ratio of the total of the structural unit derived from the aromatic divinyl compound (a2) and the structural unit derived from the aromatic monovinyl compound (a3) to the arylmaleimide structural unit is n + 1:n, include
compounds of:
structural unit derived from aromatic monovinyl compound (a3)-arylmaleimide structural unit-structural unit derived from aromatic monovinyl compound (a3); and
aromatic monovinyl compound (a3)-arylmaleimide structural unit-structural unit derived from aromatic divinyl compound (a2)-arylmaleimide structural unit-aromatic monovinyl compound (a3) unit.

The polymaleimide compound (A) of the present embodiment contains preferably compounds corresponding to at least two embodiments of the compounds of the first to third embodiments, more preferably at least a compound corresponding to the first embodiment and a compound corresponding to the second embodiment and/or a compound corresponding to the third embodiment, and even more preferably contains all of the compounds corresponding to the first to third embodiments.

Note that it is presumed that the structural unit derived from the aromatic monovinyl compound (a3) easily plays a role as an end-capping agent for the arylmaleimide structural unit.

The following embodiments will be described by taking a case where each aromatic ring is a benzene ring as an example. The following chemical structural formulas are intended to illustratively explain the present disclosure, and the scope of the present disclosure is not limited to the following chemical structural formulas.

In the present embodiment, the number average molecular weight (Mn) of the polymaleimide compound (A) is preferably 350 or more, more preferably 400 or more, and is preferably 2000 or less, more preferably 1500 or less. The weight average molecular weight (Mw) of the polymaleimide compound (A) is preferably 400 or more, more preferably 450 or more, and may be 1000 or more, and is preferably 500000 or less, more preferably 400000 or less, and may be 100000 or less or 10000 or less. By setting the Mn and Mw to the aforementioned lower limits or more, the moisture absorption rate and the dimensional change rate of the resulting cured product can be reduced. Furthermore, heat resistance and chemical resistance also tend to be improved.

From the viewpoint of being excellent in low dielectric constant and low dielectric loss tangent, the polymaleimide compound (A) in the present embodiment has a molecular weight distribution (weight average molecular weight (Mw)/number average molecular weight (Mn)), calculated from gel permeation chromatography (GPC) measurement, of preferably 1.001 or more, more preferably 1.01 or more, and preferably 500 or less, more preferably 400 or less. When the GPC chart obtained from the GPC measurement shows that the molecular weight distribution is broad and the amount of a high-molecular-weight component is large, the proportion of the high-molecular-weight component contributing to flexibility is large. Therefore, compared to a cured product using known maleimide, a cured product, having suppressed brittleness and excellent flexibility and softness, can be obtained, leading to a preferred aspect. Furthermore, by setting the molecular weight distribution to the lower limit or more, the moisture absorption rate and the dimensional change rate of the resulting cured product can be reduced. Furthermore, heat resistance and chemical resistance also tend to be improved.

The number average molecular weight (Mn), the weight average molecular weight (Mw), and the molecular weight distribution (weight average molecular weight (Mw)/number average molecular weight (Mn)) of the polymaleimide compound (A) in the present embodiment are measured according to the description in paragraph 0072 of Japanese Patent No. 7160151, the description of which is incorporated herein.

### <<Method for manufacturing polymaleimide compound (A)>>

Hereinafter, a method for manufacturing the polymaleimide compound (A) of the present disclosure will be described.

The method for manufacturing the polymaleimide compound (A) in the present embodiment is not particularly limited, and any methods can be used as long as: the aromatic amine compound (a1), the aromatic divinyl compound (a2), and maleic anhydride are used as the reaction raw materials (1); or the structural unit represented by Formula (1) is included. An example of the method for manufacturing the polymaleimide compound (A) of the present disclosure include a manufacturing method including the following steps (1) and (2).
Step (1): step of reacting, as the reaction raw materials (2), the aromatic amine compound (a1) with the aromatic divinyl compound (a2) to obtain the intermediate amine compound (C) in the present embodiment;
Step (2): step of reacting, as the reaction raw materials (3), the intermediate amine compound (C) obtained in the step (1) with maleic anhydride to obtain the polymaleimide compound (A) of the present disclosure.

Specifically, the method for manufacturing the polymaleimide compound (A) in the present embodiment preferably includes the step (1) (also referred to as a crosslinking step) of reacting the aromatic amine compound (a1) with the aromatic divinyl compound (a2) in the presence of a solid acid catalyst, and the step (2) (also referred to as a condensation step) of condensing the intermediate amine compound (C) produced in the step (1) with maleic anhydride.

Hereinafter, each step of the method for manufacturing the polymaleimide compound (A) of the present disclosure will be described in order.

### <<<Step (1): manufacturing step of intermediate amine compound (C)>>>

Hereinafter, the manufacturing step of the intermediate amine compound (C) in the present embodiment will be described.

The step (1) in the present embodiment is not particularly limited, and is a step of reacting, for example, the aforementioned aromatic amine compound (a1), the aforementioned aromatic divinyl compound (a2) (e.g., divinylbenzene), and, if necessary, other compounds, such as the aromatic monovinyl compound (a3) (e.g., ethylvinylbenzene), in the presence of an acid catalyst. This can produce the intermediate amine compound (C).

**In** consideration of the balance between the physical properties, such as moldability and curability, during the manufacturing of the resulting cured product, the blending ratio of the aromatic amine compound (a1) to the aromatic divinyl compound (a2) is preferably from 0.1 to 10 mol, and more preferably from 0.2 to 3 mol, as the molar ratio of the aromatic divinyl compound (a2) to 1 mol of the aromatic amine compound (a1). When the aromatic monovinyl compound (a3) is used in combination, the molar ratio of the total of the aromatic divinyl compound (a2) and the aromatic monovinyl compound (a3) to 1 mol of the aromatic amine compound (a1) is preferably from 0.1 to 10 mol, and more preferably from 0.2 to 3 mol.

As a specific method for carrying out the aforementioned reaction, a method is common in which: all raw materials are charged at once and reacted as they are at a predetermined temperature; or the aromatic amine compound (a1) and an acid catalyst are charged and reacted while they are maintained at a predetermined temperature and while the aromatic divinyl compound (a2), other compounds (e.g., aromatic monovinyl compound (a3)), and the like are added dropwise. **In** this case, the dropwise addition time is usually from 0.1 to 12 hours, and preferably 6 hours or less. When a solvent is used, the solvent and unreacted materials are distilled off after the reaction, if necessary, thereby obtaining the intermediate amine compound (C). When a solvent is not used, unreacted materials are distilled off, thereby obtaining the intermediate amine compound (C) as a target product.

Examples of the acid catalyst used in the step (1) in the present embodiment include: inorganic acids such as phosphoric acid, hydrochloric acid, and sulfuric acid; organic acids such as oxalic acid, benzenesulfonic acid, toluenesulfonic acid, methanesulfonic acid, and fluoromethanesulfonic acid; solid acids such as activated clay, acid clay, silica-alumina, zeolite, and strongly acidic ion exchange resin; and heteropolyhydrochloric acid. Solid acids, from which the catalyst can be easily removed by filtration after the reaction, are preferred also from the viewpoint of handleability, and when another acid is used, neutralization with a base and washing with water are preferably performed after the reaction.

The acid catalyst is blended in an amount of from 1 to 100 parts by mass based on 100 parts by mass of the total amount of raw materials to be charged (the aromatic divinyl compound (a2) or a mixture of the aromatic divinyl compound (a2) and the aromatic monovinyl compound (a3), and the aromatic amine compound (a1)), and the amount is preferably from 1 to 60 parts by mass from the viewpoint of handleability and cost-effectiveness. The reaction temperature may usually be in a range from 100 to 270°C, but is preferably from 100 to 220°C in order to suppress the formation of an isomeric structure and avoid side reactions such as thermal decomposition.

In the step (1) in the present embodiment, the mixture reaction time of the aromatic divinyl compound (a2) or the mixture of the aromatic divinyl compound (a2) and the aromatic monovinyl compound (a3) with the aromatic amine compound (a1), that is, the time of crosslinking reaction, is usually in the range from 1 to 48 hours in total under the reaction temperature condition, but preferably in the range from 1 to 30 hours in total, because the reaction does not completely proceed in a short time and a side reaction, such as a thermal decomposition reaction of a product, occurs in a long time.

In the method for manufacturing the intermediate amine compound (C) in the present embodiment, aniline or a derivative thereof also serves as a solvent, and hence another solvent may not necessarily be used, but a solvent can also be used. For example, when divinylbenzene is used as a raw material for the reaction, a method may be adopted in which: water contained in the catalyst or the like is azeotropically dehydrated, if necessary, using a solvent capable of azeotropic dehydration, such as toluene, xylene, or chlorobenzene, and after the solvent is distilled off, the reaction is carried out within the aforementioned reaction temperature range.

The intermediate amine compound (C) obtained in the step (1) is preferably represented by, for example, the following Formula (4): 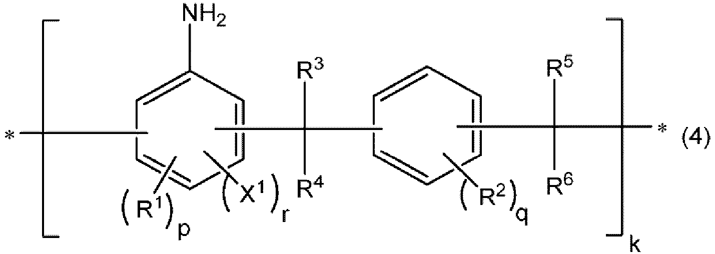 (In the above-mentioned Formula (4), R¹ each independently represents an alkyl group, R² each independently represents an alkyl group, an alkoxy group, or an alkylthio group each having from 1 to 10 carbon atoms; an aryl group, an aryloxy group, or an arylthio group each having from 6 to 10 carbon atoms; a cycloalkyl group having from 3 to 10 carbon atoms; a halogen atom; a hydroxyl group; or a mercapto group,
R³, R⁴, R⁵, and R⁶ each independently represent a hydrogen atom or a methyl group, for R³ and R⁴, one is a hydrogen atom and the other is a methyl group, for R⁵ and R⁶, one is a hydrogen atom and the other is a methyl group,
X¹ represents a substituent represented by the following Formula (x): 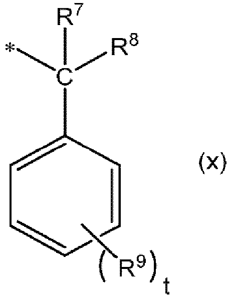 (In the above-mentioned Formula (x), R⁷ and R⁸ each independently represent a hydrogen atom or a methyl group, for R⁷ and R⁸, one is a hydrogen atom and the other is a methyl group, R⁹ each independently represents an alkyl group, an alkoxy group, or an alkylthio group each having from 1 to 10 carbon atoms; an aryl group, an aryloxy group, or an arylthio group each having from 6 to 10 carbon atoms; a cycloalkyl group having from 3 to 10 carbon atoms; a halogen atom; a hydroxyl group; or a mercapto group, and t represents an integer from 0 to 4),
r is an average value of the numbers of X¹ substituents per benzene ring to which X¹ is bonded and represents a number from 0 to 4, p represents an integer from 1 to 3, q represents an integer from 0 to 4, and k represents an integer from 1 to 100.)

In Formula (4), when p is an integer of 2 or more, a plurality of R¹s may be the same as or different from each other. When q is an integer of 2 or more, a plurality of R²s may be the same as or different from each other. When t is an integer of 2 or more, a plurality of R⁹s may be the same as or different from each other.

Note that preferred embodiments for R¹ to R⁹, X¹, p, q, r, t, and k in Formula (4) are the same as those in Formula (1). Another preferred embodiment of the intermediate amine compound (C) obtained in the step (1) includes a structure in which the N-substituted maleimide group in Formula (2), which is also a preferred embodiment of the polymaleimide compound (A), is substituted with an amino group (including -NH₂ and a substituted amino group).

In the present embodiment, the amine equivalent of the intermediate amine compound (C) is preferably from 172 to 400 g/equivalent, and more preferably from 172 to 350 g/equivalent.

Note that the amine equivalent of the intermediate amine compound (C) in the present specification is a value measured by a method in accordance with the neutralization titration method defined in JIS K 0070 (1992).

### <<<Step (2): Maleimidation>>>

The step (2) in the present embodiment is a step of reacting the intermediate amine compound (C) obtained in the step (1) with maleic anhydride. Since the amino group (including - NH₂ and a substituted amino group) of the intermediate amine compound (C) can form a chemical structure in which the amino group is substituted with an N-substituted maleimide ring by a maleimidation reaction, the polymaleimide compound (A) of the present disclosure is obtained.

In the present embodiment, the intermediate amine compound (C) represented by Formula (4), obtained in the step (1), is charged into a reactor, dissolved in an appropriate solvent, and then reacted with maleic anhydride in the presence of a catalyst. After the reaction, unreacted maleic anhydride or other impurities are removed by washing with water or the like, and the solvent is removed under reduced pressure, thereby obtaining the polymaleimide compound (A) as a target product. If necessary, a dehydrating agent may be used during the reaction.

Examples of the organic solvent used in the step (2) in the present embodiment include: ketones such as acetone, methyl ethyl ketone (MEK), methyl isobutyl ketone, cyclohexanone, and acetophenone; aprotic solvents such as N,N-dimethylformamide, N,N-dimethylacetamide, dimethylsulfoxide, N-methyl-2-pyrrolidone, acetonitrile, and sulfolane; cyclic ethers such as dioxane and tetrahydrofuran; esters such as ethyl acetate and butyl acetate; and aromatic solvents such as benzene, toluene, and xylene. These may be used alone or in combination.

In the step (2) in the present embodiment, it is a preferred aspect in which: the intermediate amine compound (C) and the maleic anhydride are preferably blended such that, as the mixing ratio between them, the ratio of the equivalent of the maleic anhydride to the amino equivalent of the intermediate amine compound (C) is in the range from 1 to 5, and more preferably charged in the range from 1 to 3; and they are reacted in an organic solvent at a mass ratio from 0.1 to 10, and preferably at a mass ratio from 0.2 to 5, based on the total amount of the intermediate amine compound (C) and the maleic anhydride.

Examples of the catalyst that can be used in the step (2) in the present embodiment include: acetates such as nickel, cobalt, sodium, calcium, iron, lithium, and manganese; inorganic salts such as chlorides, bromides, sulfates, and nitrates; inorganic acids such as phosphoric acid, hydrochloric acid, and sulfuric acid; organic acids such as oxalic acid, benzenesulfonic acid, toluenesulfonic acid, methanesulfonic acid, and fluoromethanesulfonic acid; solid acids such as activated clay, acid clay, silica-alumina, zeolite, and strongly acidic ion exchange resin; and heteropolyhydrochloric acid, and toluenesulfonic acid is particularly preferably used.

Examples of the dehydrating agent used in the step (2) in the present embodiment include: lower aliphatic carboxylic acid anhydrides such as acetic anhydride, propionic anhydride, and butyric anhydride; oxides such as phosphorus pentoxide, calcium oxide, and barium oxide; inorganic acids such as sulfuric acid; and porous ceramics such as molecular sieve, and acetic anhydride can be preferably used.

The usage amounts of the catalyst and the dehydrating agent used in the step (2) in the present embodiment are not particularly limited, but usually, the catalyst can be used in an amount from 0.0001 to 1.0 mol, preferably from 0.01 to 0.3 mol, and the dehydrating agent can be used in an amount from 1 to 3 mol, preferably from 1 to 1.5 mol, based on 1 equivalent of the amino group (-NH₂) of the intermediate amine compound (C).

In the step (2) in the present embodiment, the maleimidation reaction conditions are as follows: the intermediate amine compound (C) and the maleic anhydride are charged, reacted in a temperature range from 10 to 100°C, preferably from 30 to 60°C, for from 0.5 to 12 hours, preferably from 1 to 4 hours; and then the catalyst is added, and they are reacted in a temperature range from 90 to 130°C, preferably from 105 to 120°C, for from 1 to 24 hours, preferably from 1 to 10 hours.

As the polymaleimide compound (A) of the present embodiment (preferably a maleimide compound having a structural unit represented by Formula (1), more preferably a maleimide compound having a structural unit represented by Formula (2)), a commercially available product can also be used, and examples thereof include "NE-X-9500" available from DIC Corporation.

In addition, the cured product of the polymaleimide compound (A) used in the present embodiment is preferably excellent in low dielectric properties.

For example, the cured product of the polymaleimide compound (A) used in the present embodiment has a dielectric constant (Dk), at a frequency of 10 GHz as measured in accordance with a cavity resonator perturbation method, of preferably 3.0 or less, more preferably 2.8 or less, and even more preferably 2.6 or less. The lower limit of the dielectric constant is practically, for example, 2.0 or more. The cured product of the polymaleimide compound (A) used in the present embodiment has a dielectric loss tangent (Df), at a frequency of 10 GHz as measured in accordance with a cavity resonator perturbation method, of preferably 0.010 or less, and more preferably 0.007 or less. The lower limit of the dielectric loss tangent is practically, for example, 0.0001 or more. The relative permittivity and the dielectric loss tangent can be measured according to, for example, the methods (curing conditions, measurement conditions) described in Examples.

In addition, the cured product of the polymaleimide compound (A) used in the present embodiment preferably has high heat resistance. The cured product of the polymaleimide compound (A) used in the present embodiment has a glass transition temperature, measured in accordance with JIS C6481 dynamic viscoelasticity measurement, of preferably 180°C or higher, more preferably 200°C or higher, and even more preferably 230°C or higher. By setting the glass transition temperature to the aforementioned lower limit or more, a cured product with better heat resistance is obtained. In addition, the upper limit of the glass transition temperature is practically 400°C or lower.

In addition, for the details of the polymaleimide compound (A) used in the present embodiment, reference can be made to the description in Japanese Patent No. 7160151, the contents of which are incorporated herein.

In the description of the polymaleimide compound (A), the "alkyl group" may be linear, branched, or cyclic, and examples thereof include a methyl group, an ethyl group, a propyl group, an isopropyl group, an n-butyl group, an isobutyl group, a sec-butyl group, a tert-butyl group, an n-pentyl group, an isopentyl group, a tert-pentyl group, a neopentyl group, a 1,2-dimethylpropyl group, an n-hexyl group, an isohexyl group, an (n-)heptyl group, an (n-)octyl group, an (n-)nonyl group, an (n-)decyl group, an (n-)undecyl group, an (n-)dodecyl group, a cyclopropyl group, a cyclobutyl group, a cyclopentyl group, a cyclohexyl group, a cycloheptyl group, a cyclooctyl group, or a cyclononyl group.

Furthermore, in the description of the polymaleimide compound (A), examples of the "cycloalkyl group" include a cyclopropyl group, a cyclobutyl group, a cyclopentyl group, a cyclohexyl group, a cycloheptyl group, a cyclooctyl group, a cyclononyl group, a cyclodecyl group, a norbornyl group, or an adamantyl group.

In the description of the polymaleimide compound (A), examples of the "alkylthio group" include a methylthio group, an ethylthio group, a propylthio group, a butylthio group, an octylthio group, or a 2-ethylhexylthio group.

In the description of the polymaleimide compound (A), examples of the "alkenyl group" include an ethynyl group, a 1-propynyl group, a 2-propynyl group, a 2-butynyl group, a pentynyl group, a hexynyl group, a vinyl group, an allyl group, or an isopropenyl group.

In the description of the polymaleimide compound (A), examples of the "alkoxy group" include a methoxy group, an ethoxy group, a propoxy group, an isopropoxy group, a butoxy group, a pentyloxy group, a hexyloxy group, a 2-ethylhexyloxy group, an octyloxy group, or a nonyloxy group.

In the description of the polymaleimide compound (A), examples of the "aryl group" include a phenyl group, a 1-naphthyl group, or a 2-naphthyl group.

In addition, in the resin composition of the present embodiment, the content of the polymaleimide compound (A) is preferably 1 part by mass or more, more preferably 5 parts by mass or more, even more preferably 10 parts by mass or more, still more preferably 15 parts by mass or more, yet more preferably 20 parts by mass or more, far more preferably 25 parts by mass or more, and further, much more preferably 30 parts by mass or more, 35 parts by mass or more, 40 parts by mass or more, or 45 parts by mass or more, based on 100 parts by mass of the resin solid content in the resin composition. By setting the content of the polymaleimide compound (A) to the aforementioned lower limit or more, low dielectric properties (low dielectric constant and/or low dielectric loss tangent) and chemical resistance (in particular, alkali resistance) tend to be further improved. The upper limit of the content of the polymaleimide compound (A) is preferably 90 parts by mass or less, more preferably 88 parts by mass or less, even more preferably 86 parts by mass or less, still more preferably 84 parts by mass or less, yet more preferably 82 parts by mass or less, and depending on application and the like, may be 80 parts by mass or less, 70 parts by mass or less, 65 parts by mass or less, 50 parts by mass or less, or 40 parts by mass or less, based on 100 parts by mass of the resin solid content in the resin composition. By setting the content of the polymaleimide compound (A) to the aforementioned upper limit or less, low water absorption, chemical resistance, and desmear resistance tend to be further improved.

The resin composition of the present embodiment may contain only one kind or two or more kinds of the polymaleimide compound (A). When two or more kinds thereof are contained, the total amount thereof is preferably within the aforementioned ranges.

### <Cyanate ester compound (B) having, in a molecule, two or more aromatic moieties substituted with at least one cyanato group>

The resin composition of the present embodiment contains a cyanate ester compound (B) having, in its molecule, two or more aromatic moieties substituted with at least one cyanato group (hereinafter, may be simply referred to as "cyanate ester compound (B)").

The type of the cyanate ester compound (B) is not particularly limited as long as it is a cyanate ester compound having, in its molecule, two or more aromatic moieties substituted with at least one cyanato group.

The lower limit of the number of cyanato groups of the cyanate ester compound (B) is 2 or more, and more preferably 3 or more. By setting the number thereof to the aforementioned lower limit or more, heat resistance tends to be further improved. The upper limit of the number of the cyanato groups is preferably 100 or less, and more preferably 50 or less.

The cured product of the cyanate ester compound (B) is preferably excellent in low dielectric properties. For example, the cured product of the cyanate ester compound (B) has a dielectric constant (Dk), at a frequency of 10 GHz measured in accordance with a cavity resonator perturbation method, of preferably 4.0 or less, and more preferably 3.5 or less. The lower limit of the dielectric constant is practically, for example, 2.0 or more. The cured product of the cyanate ester compound (B) has a dielectric loss tangent (Df), at a frequency of 10 GHz measured in accordance with a cavity resonator perturbation method, of preferably 0.02 or less, and more preferably 0.015 or less. The lower limit of the dielectric loss tangent is practically, for example, 0.0001 or more. The dielectric constant and the dielectric loss tangent can be measured according to, for example, the methods (curing conditions, measurement conditions) described in Examples.

The cured product of the cyanate ester compound (B) preferably has high heat resistance. The cured product of the cyanate ester compound (B) has a glass transition temperature, measured in accordance with the JIS C 6481 dynamic viscoelasticity measurement, of preferably 150°C or higher, more preferably 180°C or higher, and even more preferably 200°C or higher. By setting the glass transition temperature to the aforementioned lower limit or higher, a cured product excellent in heat resistance can be obtained.

The cyanate ester compound (B) has a weight average molecular weight, by a GPC method calibrated with polystyrene, of preferably 200 or more, more preferably 300 or more, and even more preferably 400 or more. By setting the weight average molecular weight to the aforementioned lower limit or more, heat resistance tends to be further improved. The weight average molecular weight of the cyanate ester compound (B) is preferably 1000 or less, more preferably 900 or less, and even more preferably 800 or less. By setting the weight average molecular weight to the aforementioned upper limit or less, the moldability and handleability tend to be further improved.

Examples of the cyanate ester compound (B) include a compound represented by Formula (B1): 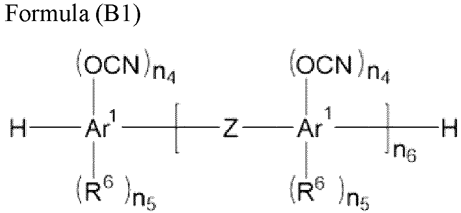 (In Formula (B1), Ar¹ each independently represents a phenylene group that may have a substituent, a naphthylene group that may have a substituent, or a biphenylene group that may have a substituent. R⁶ is each independently selected from any one of a hydrogen atom, an alkyl group having from 1 to 6 carbon atoms that may have a substituent, an aryl group having from 6 to 12 carbon atoms that may have a substituent, an alkoxy group having from 1 to 4 carbon atoms that may have a substituent, an aralkyl group that may have a substituent in which an alkyl group having from 1 to 6 carbon atoms and an aryl group having from 6 to 12 carbon atoms are bonded, and an alkylaryl group that may have a substituent in which an alkyl group having from 1 to 6 carbon atoms and an aryl group having from 6 to 12 carbon atoms are bonded. n₄ represents the number of cyanato groups bonded to Ar¹ and is an integer from 1 to 3. n₅ represents the number of R⁶ bonded to Ar¹, and is 4-n₄ when Ar¹ is a phenylene group, 6-n₄ when Ar¹ is a naphthylene group, or 8-n₄ when Ar¹ is a biphenylene group. n₆ represents the average number of repetitions and is from 1 to 50. The compound represented by Formula (B1) may be a mixture of compounds having different n₅ and/or n₆. Z is each independently selected from any one of a single bond, a divalent organic group having from 1 to 50 carbon atoms (a hydrogen atom may be substituted with a heteroatom), and a divalent organic group having from 1 to 10 nitrogen atoms (-N-R-N- or the like)).

The substituent in the aforementioned Formula (B1) is preferably a non-polar group.

The alkyl group in R⁶ in Formula (B1) may have at least one of a linear structure, a branched structure, or a cyclic structure (such as a cycloalkyl group). The hydrogen atoms in the alkyl group in Formula (B1) and the aryl group in R⁶ may be substituted with: a halogen atom such as a fluorine atom or a chlorine atom; an alkoxy group such as a methoxy group or a phenoxy group; a cyano group; or the like.

Specific examples of the alkyl group include a methyl group, an ethyl group, a propyl group, an isopropyl group, an n-butyl group, an isobutyl group, a tert-butyl group, an n-pentyl group, a 1-ethylpropyl group, a 2,2-dimethylpropyl group, a cyclopentyl group, a hexyl group, a cyclohexyl group, and a trifluoromethyl group.

Specific examples of the aryl group include a phenyl group, a xylyl group, a mesityl group, a naphthyl group, a phenoxyphenyl group, an ethylphenyl group, an o-, m-, or p-fluorophenyl group, a dichlorophenyl group, a dicyanophenyl group, a trifluorophenyl group, a methoxyphenyl group, and an o-, m-, or p-tolyl group.

Specific examples of the alkoxy group include a methoxy group, an ethoxy group, a propoxy group, an isopropoxy group, an n-butoxy group, an isobutoxy group, and a tert-butoxy group.

### n₅ is preferably 1.

Specific examples of the divalent organic group of Z in Formula (B1) include a methylene group, an ethylene group, a trimethylene group, a cyclopentylene group, a cyclohexylene group, a trimethylcyclohexylene group, a biphenylylmethylene group, a dimethylmethylene-phenylene-dimethylmethylene group, a fluorenediyl group, and a phthalidodiyl group. The hydrogen atom in the divalent organic group may be substituted with: a halogen atom such as a fluorine atom or a chlorine atom; an alkoxy group such as a methoxy group or a phenoxy group; a cyano group; or the like. Examples of the divalent organic group containing from 1 to 10 nitrogen atoms of Z in Formula (B1) include an imino group and a polyimide group.

In addition, examples of Z in Formula (B1) include a structure represented by the following Formula (B2) or Formula (B3): 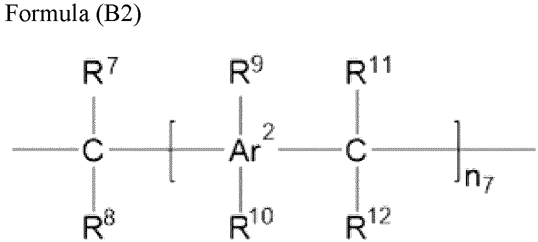 (In Formula (B2), Ar² is selected from any one of a phenylene group, a naphthylene group, and a biphenylene group. R⁷, R⁸, R¹¹, and R¹² are each independently selected from any one of a hydrogen atom, an alkyl group having from 1 to 6 carbon atoms, an aryl group having from 6 to 12 carbon atoms, and an aryl group replaced by at least one of a trifluoromethyl group or a phenolic hydroxyl group. R⁹ and R¹⁰ are each independently selected from a hydrogen atom, an alkyl group having from 1 to 6 carbon atoms, an aryl group having from 6 to 12 carbon atoms, an alkoxy group having from 1 to 4 carbon atoms, and a hydroxy group. n₇ represents an integer from 1 to 5, but the compound represented by Formula (B1) may be a mixture of compounds having different n7s.) 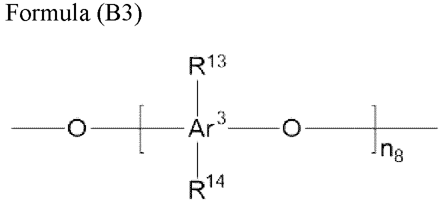 (In Formula (B3), Ar³ is selected from any one of a phenylene group, a naphthylene group, and a biphenylene group. R¹³ and R¹⁴ are each independently selected from any one of a hydrogen atom, an alkyl group having from 1 to 6 carbon atoms, an aryl group having from 6 to 12 carbon atoms, a benzyl group, an alkoxy group having from 1 to 4 carbon atoms, and an aryl group substituted with at least one of a hydroxy group, a trifluoromethyl group, or a cyanato group. n₈ represents an integer from 1 to 5, but the compound represented by Formula (B 1) may be a mixture of compounds having different n₈s.)

Furthermore, examples of Z in Formula (B 1) include a divalent group represented by the following Formulas: 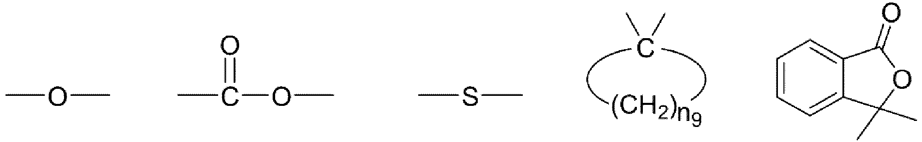 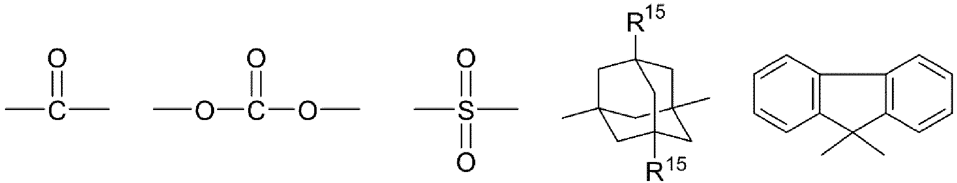 (In the formulas, n₉ represents an integer from 4 to 7. R¹⁵ each independently represents a hydrogen atom or an alkyl group having from 1 to 6 carbon atoms.)

Specific examples of Ar² in Formula (B2) and Ar³ in Formula (B3) include a 1,4-phenylene group, a 1,3-phenylene group, a 4,4'-biphenylene group, a 2,4'-biphenylene group, a 2,2'-biphenylene group, a 2,3'-biphenylene group, a 3,3'-biphenylene group, a 3,4'-biphenylene group, a 2,6-naphthylene group, a 1,5-naphthylene group, a 1,6-naphthylene group, a 1,8-naphthylene group, a 1,3-naphthylene group, and a 1,4-naphthylene group. The alkyl groups and aryl groups for R⁷ to R¹² in Formula (B2) and for R¹³ and R¹⁴ in Formula (B3) are the same as those described for Formula (B1).

n₆ represents the average number of repetitions, and is preferably from 1 to 20, more preferably from 1 to 15, and even more preferably from 1 to 10.

Specific examples of the cyanate ester compound (B) include 1,2-dicyanatobenzene, 1,3-dicyanatobenzene, 1,4-dicyanatobenzene, 1,4-dicyanato-2-tert-butylbenzene, 1,4-dicyanato-2,4-dimethylbenzene, 1,4-dicyanato-2,3,4-trimethylbenzene, 1,3-dicyanato-2,4,6-trimethylbenzene, 1,3-dicyanato-5-methylbenzene, 2,2'-dicyanato-1,1'-binaphthyl, 1,3-, 1,4-, 1,5-, 1,6-, 1,7-, 2,3-, 2,6-, or 2,7-dicyanatonaphthalene, 2,2'- or 4,4'-dicyanatobiphenyl, 4,4'-dicyanatooctafluorobiphenyl, 2,4'- or 4,4'-dicyanatodiphenylmethane, bis(4-cyanato-3,5-dimethylphenyl)methane, 1,1-bis(4-cyanatophenyl)ethane, 1,1-bis(4-cyanatophenyl)propane, 2,2-bis(4-cyanatophenyl)propane, 2,2-bis(3-allyl-4-cyanatophenyl)propane, 2,2-bis(4-cyanato-3-methylphenyl)propane, 2,2-bis(2-cyanato-5-biphenylyl)propane, 2,2-bis(4-cyanatophenyl)hexafluoropropane, 2,2-bis(4-cyanato-3,5-dimethylphenyl)propane, 1,1-bis(4-cyanatophenyl)butane, 1,1-bis(4-cyanatophenyl)isobutane, 1,1-bis(4-cyanatophenyl)pentane, 1,1-bis(4-cyanatophenyl)-3-methylbutane, 1,1-bis(4-cyanatophenyl)-2-methylbutane, 1,1-bis(4-cyanatophenyl)-2,2-dimethylpropane, 2,2-bis(4-cyanatophenyl)butane, 2,2-bis(4-cyanatophenyl)pentane, 2,2-bis(4-cyanatophenyl)hexane, 2,2-bis(4-cyanatophenyl)-3-methylbutane, 2,2-bis(4-cyanatophenyl)-4-methylpentane, 2,2-bis(4-cyanatophenyl)-3,3-dimethylbutane, 3,3-bis(4-cyanatophenyl)hexane, 3,3-bis(4-cyanatophenyl)heptane, 3,3-bis(4-cyanatophenyl)octane, 3,3-bis(4-cyanatophenyl)-2-methylpentane, 3,3-bis(4-cyanatophenyl)-2-methylhexane, 3,3-bis(4-cyanatophenyl)-2,2-dimethylpentane, 4,4-bis(4-cyanatophenyl)-3-methylheptane, 3,3-bis(4-cyanatophenyl)-2 methylheptane, 3,3-bis(4-cyanatophenyl)-2,2-dimethylhexane, 3,3-bis(4-cyanatophenyl)-2,4-dimethylhexane, 3,3-bis(4-cyanatophenyl)-2,2,4-trimethylpentane, 2,2-bis(4-cyanatophenyl)-1,1,1,3,3,3-hexafluoropropane, bis(4-cyanatophenyl)phenylmethane, 1,1-bis(4-cyanatophenyl)-1-phenylethane, bis(4-cyanatophenyl)biphenylmethane, 1,1-bis(4-cyanatophenyl)cyclopentane, 1,1-bis(4-cyanatophenyl)cyclohexane, 2,2-bis(4-cyanato-3-isopropylphenyl)propane, 1,1-bis(3-cyclohexyl-4-cyanatophenyl)cyclohexane, bis(4-cyanatophenyl)diphenylmethane, bis(4-cyanatophenyl)-2,2-dichloroethylene, 1,3-bis[2-(4-cyanatophenyl)-2 propyl]benzene, 1,4-bis[2-(4-cyanatophenyl)-2-propyl]benzene, 1,1-bis(4-cyanatophenyl)-3,3,5-trimethylcyclohexane, 4-[bis(4-cyanatophenyl)methyl]biphenyl, 4,4-dicyanatobenzophenone, 1,3-bis(4-cyanatophenyl)-2-propene-1-one, bis(4-cyanatophenyl)ether, bis(4-cyanatophenyl)sulfide, bis(4-cyanatophenyl)sulfone, 4-cyanatobenzoic acid-4-cyanatophenyl ester(4-cyanatophenyl-4-cyanatobenzoate), bis-(4-cyanatophenyl)carbonate, 1,3-bis(4-cyanatophenyl)adamantane, 1,3-bis(4-cyanatophenyl)-5,7-dimethyladamantane, 3,3-bis(4-cyanatophenyl)isobenzofuran-1(3H)-one(cyanate of phenolphthalein), 3,3-bis(4-cyanato-3-methylphenyl)isobenzofuran-1(3H)-one(cyanate of o-cresol phthalein), 9,9-bis(4-cyanatophenyl)fluorene, 9,9-bis(4-cyanato-3-methylphenyl)fluorene, 9,9-bis(2-cyanato-5-biphenylyl)fluorene, tris(4-cyanatophenyl)methane, 1,1,1-tris(4-cyanatophenyl)ethane, 1,1,3-tris(4-cyanatophenyl)propane, α,α,α '-tris(4-cyanatophenyl)-1-ethyl-4-isopropylbenzene, 1,1,2,2-tetrakis(4-cyanatophenyl)ethane, tetrakis(4-cyanatophenyl)methane, 2,4,6-tris(N-methyl-4- cyanatanilino)-1,3,5-triazine, 2,4-bis(N-methyl-4-cyanatanilino)-6-(N-methylanilino)-1,3,5-triazine, bis(N-4-cyanato-2-methylphenyl)-4,4'-oxydiphthalimide, bis(N-3-cyanato-4-methylphenyl)-4,4'-oxydiphthalimide, bis(N-4-cyanatophenyl)-4,4'-oxydiphthalimide, bis(N-4-cyanato-2-methylphenyl)-4,4'-(hexafluoroisopropylidene)diphthalimide, tris(3,5-dimethyl-4-cyanatobenzyl)isocyanurate, 2-phenyl-3,3-bis(4-cyanatophenyl)phthalimidine, 2-(4-methylphenyl)-3,3-bis(4-cyanatophenyl)phthalimidine, 2-phenyl-3,3-bis(4-cyanato-3-methylphenyl)phthalimidine, 1-methyl-3,3-bis(4-cyanatophenyl)indoline-2-one, 2-phenyl-3,3-bis(4-cyanatophenyl)indoline-2-one, phenol novolac resin or cresol novolac resin (a product obtained by reacting phenol, alkylsubstituted phenol, or halogen-substituted phenol with a formaldehyde compound, such as formalin or paraformaldehyde, in an acidic solution by a known method), a trisphenol novolac resin (obtained by reacting hydroxybenzaldehyde with phenol in the presence of an acidic catalyst), a fluorene novolac resin (compound obtained by reacting a fluorenone compound with 9,9-bis(hydroxyaryl) fluorenes in the presence of an acidic catalyst), a phenol aralkyl resin, a cresol aralkyl resin, a naphthol aralkyl resin or a biphenyl aralkyl resin (a product obtained by reacting a bishalogenomethyl compound represented by Ar⁴-(CH₂Z')₂ with a phenolic compound in the presence of an acidic catalyst or without a catalyst, a product obtained by reacting a bis(alkoxymethyl)compound represented by Ar⁴-(CH₂OR)₂ or a bis (hydroxymethyl) compound represented by Ar⁴-(CH₂OH)₂ with a phenolic compound in the presence of an acidic catalyst, or a product obtained by polycondensing an aromatic aldehyde compound, an aralkyl compound, or a phenolic compound by a known method), a phenol-modified xylene formaldehyde resin (a product obtained by reacting a xylene formaldehyde resin with a phenolic compound in the presence of an acidic catalyst by a known method), a modified naphthalene formaldehyde resin (a product obtained by reacting a naphthalene formaldehyde resin with a hydroxy-substituted aromatic compound in the presence of an acidic catalyst by a known method), a phenol-modified dicyclopentadiene resin, a phenolic resin having a polynaphthylene ether structure (a polyvalent hydroxynaphthalene compound having two or more phenolic hydroxy groups in a single molecule is dehydrated and condensed in the presence of a basic catalyst by a known method), and a product obtained by cyanate-esterifying a phenolic resin, but are not particularly limited thereto. These cyanate ester compounds (B) may be used alone or in combination of two or more kinds thereof.

Preferred examples of the cyanate ester compound (B) include at least one selected from the group consisting of a phenol novolac-type cyanate ester compound, a naphthol aralkyl-type cyanate ester compound (naphthol aralkyl-type cyanate), a naphthylene ether-type cyanate ester compound, a biphenyl aralkyl-type cyanate ester compound, a xylene resin-type cyanate ester compound, a trisphenol methane-type cyanate ester compound, an adamantane skeleton-type cyanate ester compound, a bisphenol M-type cyanate ester compound, and a bisphenol A-type cyanate ester compound. Among these, from the viewpoint of further improving low water absorption, the cyanate ester compound is preferably at least one selected from the group consisting of a phenol novolac-type cyanate ester compound, a naphthol aralkyl-type cyanate ester compound, a naphthylene ether-type cyanate ester compound, a xylene resin-type cyanate ester compound, a bisphenol M-type cyanate ester compound, and a bisphenol A-type cyanate ester compound, more preferably at least one selected from the group consisting of a phenol novolac-type cyanate ester compound, a naphthol aralkyl-type cyanate ester compound, a naphthylene ether-type cyanate ester compound, a bisphenol A-type cyanate ester compound, and a bisphenol M-type cyanate ester compound, even more preferably at least one selected from the group consisting of a phenol novolac-type cyanate ester compound, a naphthol aralkyl-type cyanate ester compound, and a bisphenol A-type cyanate ester compound, still more preferably a naphthol aralkyl-type cyanate ester compound and/or a bisphenol A-type cyanate ester compound, and yet more preferably a naphthol aralkyl-type cyanate ester compound.

As the naphthol aralkyl-type cyanate ester compound, a compound represented by Formula (N1) is more preferred. 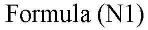 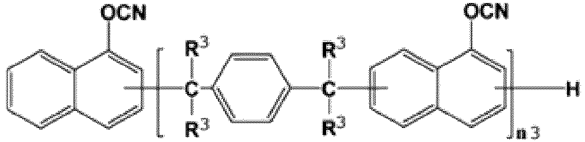 (In Formula (N1), R³ each independently represents a hydrogen atom or a methyl group, and n3 represents an integer of 1 or more.)

In Formula (N1), R³ each independently represents a hydrogen atom or a methyl group, and among them, a hydrogen atom is preferred.

In Formula (N1), n3 is an integer of 1 or more, preferably an integer from 1 to 50, more preferably an integer from 1 to 20, even more preferably an integer from 1 to 10, and still more preferably an integer from 1 to 6.

The phenol novolac-type cyanate ester compound is not particularly limited, but for example, a compound represented by Formula (VII) is preferred. 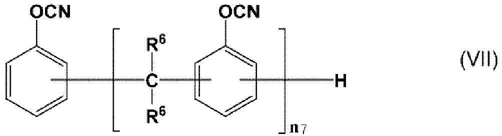 (In Formula (VII), R⁶ each independently represents a hydrogen atom or a methyl group; and n7 represents an integer of 1 or more.)

In Formula (VII), R⁶ each independently represents a hydrogen atom or a methyl group, and among them, a hydrogen atom is preferred.

In Formula (VII), n7 is an integer of 1 or more, preferably an integer from 1 to 20, more preferably an integer from 1 to 10, and even more preferably an integer from 1 to 6.

As the bisphenol A-type cyanate ester compound, one or more selected from the group consisting of 2,2-bis(4-cyanatophenyl)propane and a prepolymer of 2,2-bis(4-cyanatophenyl)propane.

These cyanate ester compounds (B) may be prepared by known methods, or commercially available products may be used. A cyanate ester compound having a naphthol aralkyl skeleton, a naphthylene ether skeleton, a xylene skeleton, a trisphenol methane skeleton, or an adamantane skeleton has a relatively large functional group equivalent number, and the amount of unreacted cyanate ester groups is reduced, so that a resin composition using these compounds tends to be further excellent in low water absorption. In addition, mainly due to having an aromatic skeleton or an adamantane skeleton, plating adhesion tends to be further improved.

The content of the cyanate ester compound (B) in the resin composition of the present embodiment is preferably 1 part by mass or more, more preferably 5 parts by mass or more, even more preferably 10 parts by mass or more, still more preferably 15 parts by mass or more, and depending on application and the like, may be 18 parts by mass or more, 20 parts by mass or more, 25 parts by mass or more, 30 parts by mass or more, or 40 parts by mass or more, based on 100 parts by mass of the resin solid content in the resin composition. When the content of the cyanate ester compound (B) is the aforementioned lower limit or more, heat resistance, flame resistance, chemical resistance, low dielectric constant, low dielectric loss tangent, and insulation tend to be improved. The upper limit of the content of the cyanate ester compound (B) is preferably 90 parts by mass or less, more preferably 85 parts by mass or less, even more preferably 80 parts by mass or less, still more preferably 75 parts by mass or less, yet more preferably 70 parts by mass or less, far more preferably 60 parts by mass or less, and depending on application and the like, may be 50 parts by mass or less, 40 parts by mass or less, or 30 parts by mass or less, based on 100 parts by mass of the resin solid content in the resin composition. By setting the content of the cyanate ester compound (B) to the aforementioned upper limit or less, low dielectric properties, chemical resistance (in particular, alkali resistance), and desmear resistance tend to be more effectively exhibited.

The resin composition of the present embodiment may contain only one kind or two or more kinds of the cyanate ester compound (B). When two or more kinds thereof are contained, the total amount thereof is preferably within the aforementioned ranges.

In the resin composition of the present embodiment, the total amount of the polymaleimide compound (A) and the cyanate ester compound (B) is preferably 20 mass% or more, more preferably 30 mass% or more, even more preferably 40 mass% or more, still more preferably 50 mass% or more, yet more preferably 60 mass% or more, far more preferably 70 mass% or more, and may be 80 mass% or more, 90 mass% or more, or 95 mass% or more of the resin solid content in the resin composition. By setting the total amount of the polymaleimide compound (A) and the cyanate ester compound (B) to the aforementioned lower limit or more, low water absorption, low dielectric properties (low dielectric constant and/or low dielectric loss tangent), chemical resistance, and desmear resistance tend to be further improved. The upper limit of the total amount is preferably 99.9 mass% or less of the resin solid content in the resin composition. By setting the total amount of the polymaleimide compound (A) and the cyanate ester compound (B) to the aforementioned upper limit or less, heat resistance tends to be further improved.

In the resin composition of the present embodiment, the mass ratio of the polymaleimide compound (A) and the cyanate ester compound (B) is preferably 5 parts by mass or more, more preferably 10 parts by mass or more, and even more preferably 15 parts by mass or more, based on 100 parts by mass of the polymaleimide compound (A). The mass ratio is preferably 500 parts by mass or less, more preferably 450 parts by mass or less, and even more preferably 400 parts by mass or less, based on 100 parts by mass of the polymaleimide compound (A). Such a blend ratio makes it possible to improve heat resistance, low thermal expansion, low water absorption, chemical resistance, and desmear resistance while maintaining low dielectric properties.

### <Additional resin component (C)>

The resin composition of the present embodiment preferably contains one or more additional resin components (C) selected from the group consisting of a maleimide compound other than the polymaleimide compound (A), a compound containing a polymerizable carbon-carbon unsaturated double bond, an epoxy compound, a phenolic compound, an oxetane resin, a benzoxazine compound, a thermoplastic elastomer, an aryl cyclobutene resin, a polyamide resin, a polyimide resin, a perfluorovinyl ether resin, and a petroleum resin, and preferably contains one or more additional resin components (C) selected from the group consisting of a maleimide compound other than the polymaleimide compound (A), a compound containing a polymerizable carbon-carbon unsaturated double bond, an epoxy compound, a phenolic compound, an oxetane resin, a benzoxazine compound, and a thermoplastic elastomer. By containing such a component, other desired performance required for a printed wiring board can be more effectively exhibited.

The cured product of each of the additional resin components (C) is preferably excellent in low dielectric properties. For example, the cured product of each of the additional resin components (C) has a dielectric constant (Dk), at a frequency of 10 GHz measured in accordance with a cavity resonator perturbation method, of preferably 4.0 or less, and more preferably 3.5 or less. In addition, the lower limit of the dielectric constant (Dk) is practically, for example, 2.0 or more. The cured product of each of the additional resin components (C) has a dielectric loss tangent (Df), at a frequency of 10 GHz measured in accordance with a cavity resonator perturbation method, of preferably 0.03 or less, and more preferably 0.002 or less. In addition, the lower limit of the dielectric loss tangent (Df) is practically, for example, 0.0001 or more. The dielectric constant and the dielectric loss tangent can be measured according to, for example, the methods (curing conditions, measurement conditions) described in Examples.

In addition, the cured products of the additional resin components (C) preferably have high heat resistance. For example, the cured products of the additional resin components (C) each have a glass transition temperature measured in accordance with the JIS C 6481 dynamic viscoelasticity measurement of preferably 150°C or higher, more preferably 180°C or higher, and even more preferably 200°C or higher. By setting the glass transition temperature to the aforementioned lower limit or higher, a cured product more excellent in heat resistance can be obtained. In addition, the upper limit of the glass transition temperature is practically 400°C or lower.

When the resin composition of the present embodiment contains the additional resin components (C), the total content thereof is preferably 1 part by mass or more, more preferably 5 parts by mass or more, even more preferably 10 parts by mass or more, still more preferably 20 parts by mass or more, and may be 30 parts by mass or more, based on 100 parts by mass of the resin solid content in the resin composition. By setting the content of the additional resin components (C) to the aforementioned lower limit or more, heat resistance tends to be further improved. The upper limit of the total content of the additional resin components (C) is preferably 70 parts by mass or less, more preferably 60 parts by mass or less, even more preferably 50 parts by mass or less, and may be 40 parts by mass or less, based on 100 parts by mass of the resin solid content in the resin composition. By setting the content of the additional resin components (C) to the aforementioned upper limit or less, low dielectric properties tend to be further improved.

### <<Maleimide compound other than polymaleimide compound (A)>>

The resin composition of the present embodiment may contain a maleimide compound other than the polymaleimide compound (A) (hereinafter, it may be simply referred to as "additional maleimide compound").

The additional maleimide compound is not particularly limited as long as it is a compound containing, in a molecule, one or more (preferably from 2 to 12, more preferably from 2 to 6, even more preferably from 2 to 4, still more preferably 2 or 3, and far more preferably 2) maleimide groups. Compounds usually used in the field of printed wiring boards can be widely used.

In the present embodiment, the additional maleimide compound: preferably contains one or more selected from the group consisting of a compound represented by Formula (M0), a compound represented by Formula (M1), a compound represented by Formula (M2), a compound represented by Formula (M3), a compound represented by Formula (M4), a compound represented by Formula (M5), and a maleimide compound (M6); more preferably contains one or more selected from the group consisting of a compound represented by Formula (M0), a compound represented by Formula (M1), a compound represented by Formula (M3), a compound represented by Formula (M4), a compound represented by Formula (M5), and a maleimide compound (M6); even more preferably contains one or more selected from the group consisting of a compound represented by Formula (M1), a compound represented by Formula (M3), a compound represented by Formula (M4), and a compound represented by Formula (M5); still more preferably contains one or more selected from the group consisting of a compound represented by Formula (M1), a compound represented by Formula (M3), and a compound represented by Formula (M5); and yet more preferably contains a compound represented by Formula (M1) and/or a compound represented by Formula (M3). When these additional maleimide compounds are used in a material for a printed wiring board (e.g., a metal foil-clad laminate), excellent heat resistance can be impaired. 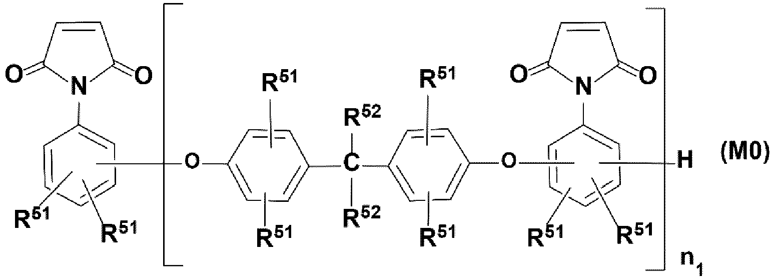 (In Formula (M0), R⁵¹ each independently represents a hydrogen atom, an alkyl group having from 1 to 8 carbon atoms, or a phenyl group; R⁵² each independently represents a hydrogen atom or a methyl group; and n₁ represents an integer of 1 or more.)

R⁵¹ is each independently preferably one selected from the group consisting of a hydrogen atom, a methyl group, an ethyl group, an n-propyl group, an isopropyl group, an n-butyl group, an isobutyl group, a t-butyl group, an n-pentyl group, and a phenyl group; more preferably a hydrogen atom and/or a methyl group; and still more preferably a hydrogen atom.

R⁵² is preferably a methyl group.

n₁ is preferably an integer from 1 to 10, more preferably an integer from 1 to 5, even more preferably an integer from 1 to 3, still more preferably 1 or 2, and yet more preferably 1.

Specifically, the following compound is cited as a preferred example of Formula (M0).

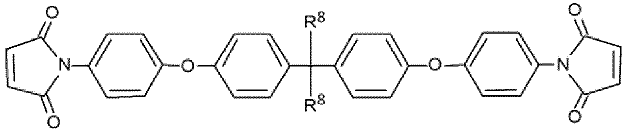

In the aforementioned Formula, R⁸ each independently represents a hydrogen atom, a methyl group, or an ethyl group, and a methyl group is preferred.

The compound represented by Formula (M0) may be of only one kind or a mixture of two or more kinds. Examples of the mixture include: a mixture of compounds having different n₁s; a mixture of compounds having different kinds of substituent of R⁵¹ and/or R⁵²; a mixture of compounds in which maleimide groups and oxygen atoms are bonded to benzene rings at different positions (meta positions, para positions, and ortho positions); and a mixture of compounds in which two or more different points described above are combined. The same shall apply to the compounds represented by Formulas from (M1) to (M6): 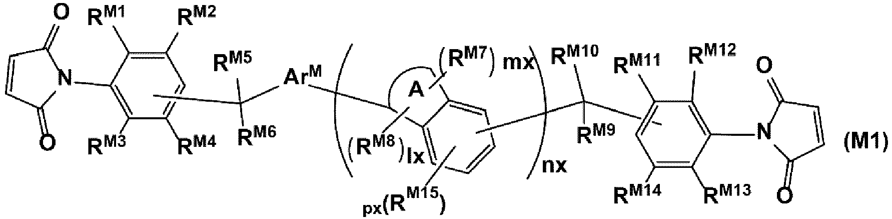 (In Formula (M1), R^{M1}, R^{M2}, R^{M3}, and R^{M4} each independently represent a hydrogen atom or an organic group. R^{M5} and R^{M6} each independently represent a hydrogen atom or an alkyl group. Ar^{M} represents a divalent aromatic group. A is a 4- to 6-membered alicyclic group. R^{M7} and R^{M8} are each independently an alkyl group. mx is 1 or 2, and 1x is 0 or 1. R^{M9} and R^{M10} each independently represent a hydrogen atom or an alkyl group. R^{M11}, R^{M12}, R^{M13}, and R^{M14} each independently represent a hydrogen atom or an organic group. R^{M15} each independently represents an alkyl group having from 1 to 10 carbon atoms, an alkyloxy group having from 1 to 10 carbon atoms, an alkylthio group having from 1 to 10 carbon atoms, a cycloalkyl group having from 3 to 10 carbon atoms, an aryl group having from 6 to 10 carbon atoms, an aryloxy group having from 6 to 10 carbon atoms, an arylthio group having from 6 to 10 carbon atoms, a halogen atom, a hydroxyl group, or a mercapto group. px represents an integer from 0 to 3. nx represents an integer from 1 to 20.)

R^{M1}, R^{M2}, R^{M3}, and R^{M4} in the formula each independently represent a hydrogen atom or an organic group. The organic group here is preferably an alkyl group, more preferably an alkyl group having from 1 to 12 carbon atoms, even more preferably an alkyl group having from 1 to 6 carbon atoms, still more preferably a methyl group, an ethyl group, a propyl group, or a butyl group, and among them, a methyl group is particularly preferred. R^{M1} and R^{M3} are each independently preferably an alkyl group, and R^{M2} and R^{M4} are preferably hydrogen atoms.

R^{M5} and R^{M6} each independently represent a hydrogen atom or an alkyl group, and are preferably alkyl groups. The alkyl group here is preferably an alkyl group having from 1 to 12 carbon atoms, more preferably an alkyl group having from 1 to 6 carbon atoms, even more preferably a methyl group, an ethyl group, a propyl group, or a butyl group, and among them, a methyl group is particularly preferred.

Ar^{M} represents a divalent aromatic group, and is preferably a phenylene group, a naphthalenediyl group, a phenanthrenediyl group, or an anthracenediyl group, more preferably a phenylene group, and even more preferably an m-phenylene group. Ar^{M} may have a substituent, and the substituent is preferably an alkyl group, more preferably an alkyl group having from 1 to 12 carbon atoms, even more preferably an alkyl group having from 1 to 6 carbon atoms, still more preferably a methyl group, an ethyl group, a propyl group, or a butyl group, and particularly preferably a methyl group. However, Ar^{M} is preferably unsubstituted.

A is a 4- to 6-membered alicyclic group, and more preferably a 5-membered alicyclic group (preferably a group to form an indane ring with a benzene ring). R^{M7} and R^{M8} are each independently an alkyl group, preferably an alkyl group having from 1 to 6 carbon atoms, more preferably an alkyl group having from 1 to 3 carbon atoms, and particularly preferably a methyl group.

mx is 1 or 2, and preferably 2.

lx is 0 or 1, and preferably 1.

R^{M9} and R^{M10} each independently represent a hydrogen atom or an alkyl group, and more preferably an alkyl group. The alkyl group here is preferably an alkyl group having from 1 to 12 carbon atoms, more preferably an alkyl group having from 1 to 6 carbon atoms, even more preferably a methyl group, an ethyl group, a propyl group, or a butyl group, and among them, a methyl group is particularly preferred.

R^{M11}, R^{M12}, R^{M13}, and R^{M14} each independently represent a hydrogen atom or an organic group. The organic group here is preferably an alkyl group, more preferably an alkyl group having from 1 to 12 carbon atoms, even more preferably an alkyl group having from 1 to 6 carbon atoms, still more preferably a methyl group, an ethyl group, a propyl group, or a butyl group, and among them, a methyl group is particularly preferred. R^{M12} and R^{M13} are each independently preferably an alkyl group, and R^{M11} and R^{M14} are preferably hydrogen atoms.

R^{M15} each independently represents an alkyl group having from 1 to 10 carbon atoms, an alkyloxy group having from 1 to 10 carbon atoms, an alkylthio group having from 1 to 10 carbon atoms, a cycloalkyl group having from 3 to 10 carbon atoms, an aryl group having from 6 to 10 carbon atoms, an aryloxy group having from 6 to 10 carbon atoms, an arylthio group having from 6 to 10 carbon atoms, a halogen atom, a hydroxyl group, or a mercapto group; and is preferably an alkyl group having from 1 to 4 carbon atoms, a cycloalkyl group having from 3 to 6 carbon atoms, or an aryl group having from 6 to 10 carbon atoms.

px represents an integer from 0 to 3, and is preferably an integer from 0 to 2, more preferably 0 or 1, and even more preferably 0.

nx represents an integer from 1 to 20. nx may be an integer of 10 or less.

Note that the resin composition of the present embodiment may contain only one kind or two or more kinds of compound that are represented by Formula (M1) and have at least different nx values. When two or more kinds are contained, the average value (average number of repeating units) n of nxs in the compounds represented by Formula (M1) in the resin composition is preferably 0.92 or more, more preferably 0.95 or more, even more preferably 1.0 or more, and still more preferably 1.1 or more in order to obtain a compound having a low melting point (low softening point), low melt viscosity, and excellent handleability. n is preferably 10.0 or less, more preferably 8.0 or less, even more preferably 7.0 or less, still more preferably 6.0 or less, and may be 5.0 or less. The same applies to the later-described Formula (M1-1) and the like.

The compound represented by Formula (M1) is preferably a compound represented by the following Formula (M1-1): 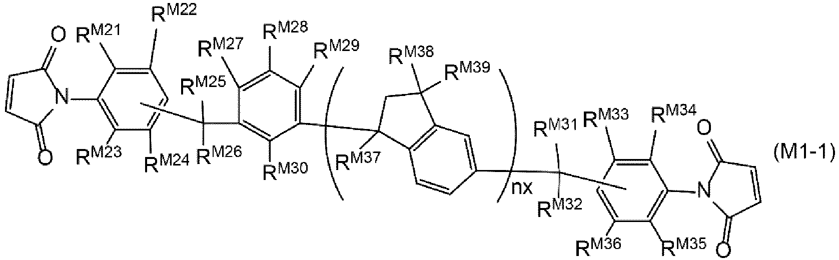 (In Formula (M1-1), R^{M21}, R^{M22}, R^{M23}, and R^{M24} each independently represent a hydrogen atom or an organic group. R^{M25} and R^{M26} each independently represent a hydrogen atom or an alkyl group. R^{M27}, R^{M28}, R^{M29}, and R^{M30} each independently represent a hydrogen atom or an organic group. R^{M31} and R^{M32} each independently represent a hydrogen atom or an alkyl group. R^{M33}, R^{M34}, R^{M35}, and R^{M36} each independently represent a hydrogen atom or an organic group. R^{M37}, R^{M38}, and R^{M39} each independently represent a hydrogen atom or an alkyl group. nx represents an integer of 1 or more and 20 or less.)

R^{M21}, R^{M22}, R^{M23}, and R^{M24} in the formula each independently represent a hydrogen atom or an organic group. The organic group here is preferably an alkyl group, more preferably an alkyl group having from 1 to 12 carbon atoms, even more preferably an alkyl group having from 1 to 6 carbon atoms, still more preferably a methyl group, an ethyl group, a propyl group, or a butyl group, and particularly preferably a methyl group. R^{M21} and R^{M23} are preferably alkyl groups, and R^{M22} and R^{M24} are preferably hydrogen atoms.

R^{M25} and R^{M26} each independently represent a hydrogen atom or an alkyl group, and are preferably alkyl groups. The alkyl group here is preferably an alkyl group having from 1 to 12 carbon atoms, more preferably an alkyl group having from 1 to 6 carbon atoms, even more preferably a methyl group, an ethyl group, a propyl group, or a butyl group, and among them, a methyl group is particularly preferred.

R^{M27} R^{M28}, R^{M29}, and R^{M30} each independently represent a hydrogen atom or an organic group, and are preferably hydrogen atoms. The organic group here is preferably an alkyl group, more preferably an alkyl group having from 1 to 12 carbon atoms, even more preferably an alkyl group having from 1 to 6 carbon atoms, still more preferably a methyl group, an ethyl group, a propyl group, or a butyl group, and particularly preferably a methyl group.

R^{M31} and R^{M32} each independently represent a hydrogen atom or an alkyl group, and are preferably alkyl groups. The alkyl group here is preferably an alkyl group having from 1 to 12 carbon atoms, more preferably an alkyl group having from 1 to 6 carbon atoms, even more preferably a methyl group, an ethyl group, a propyl group, or a butyl group, and among them, a methyl group is particularly preferred.

R^{M33}, R^{M34}, R^{M35}, and R^{M36} each independently represent a hydrogen atom or an organic group. The organic group here is preferably an alkyl group, more preferably an alkyl group having from 1 to 12 carbon atoms, even more preferably an alkyl group having from 1 to 6 carbon atoms, still more preferably a methyl group, an ethyl group, a propyl group, or a butyl group, and particularly preferably a methyl group.

R^{M33} and R^{M36} are preferably hydrogen atoms, and R^{M34} and R^{M35} are preferably alkyl groups.

R^{M37} R^{M38}, and R^{M39} each independently represent a hydrogen atom or an alkyl group, and are preferably alkyl groups. The alkyl group here is preferably an alkyl group having from 1 to 12 carbon atoms, more preferably an alkyl group having from 1 to 6 carbon atoms, even more preferably a methyl group, an ethyl group, a propyl group, or a butyl group, and among them, a methyl group is particularly preferred.

nx represents an integer of 1 or more and 20 or less. nx may be an integer of 10 or less.

The compound represented by Formula (M1-1) is preferably a compound represented by the following Formula (M1-2): 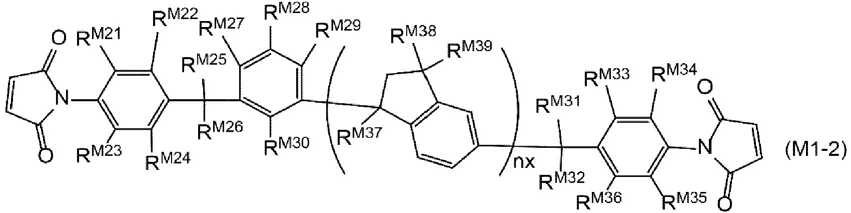 (In Formula (M1-2), R^{M21}, R^{M22}, R^{M23}, and R^{M24} each independently represent a hydrogen atom or an organic group. R^{M25} and R^{M26} each independently represent a hydrogen atom or an alkyl group. R^{M27} R^{M28} R^{M29}, and R^{M30} each independently represent a hydrogen atom or an organic group. R^{M31} and R^{M32} each independently represent a hydrogen atom or an alkyl group. R^{M33}, R^{M34}, R^{M35}, and R^{M36} each independently represent a hydrogen atom or an organic group. R^{M37}, R^{M38}, and R^{M39} each independently represent a hydrogen atom or an alkyl group. nx represents an integer of 1 or more and 20 or less.)

In Formula (M1-2), R^{M21}, R^{M22}, R^{M23}, R^{M24}, R^{M25}, R^{M26}, R^{M27}, R^{M28}, R^{M29}, R^{M30}, R^{M31}, R^{M32}, R^{M33}, R^{M34}, R^{M35}, R^{M36} R^{M37} R^{M38} R^{M39}, and nx have the same meanings as those of R^{M21}, R^{M22}, R^{M23}, R^{M24}, R^{M25}, R^{M26}, R^{M27}, R^{M28}, R^{M29}, R^{M30}, R^{M31}, R^{M32}, R^{M33}, R^{M34}, R^{M35}, R^{M36}, R^{M37}, R^{M38}, R^{M39}, and nx in Formula (M1-1), respectively, and preferred ranges thereof are also the same.

The compound represented by Formula (M1-1) is preferably a compound represented by the following Formula (M1-3), and more preferably a compound represented by the following Formula (M1-4): 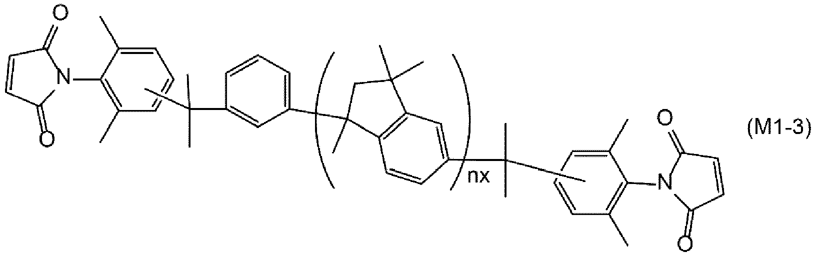 (In Formula (M1-3), nx represents an integer of 1 or more and 20 or less.)

nx may be an integer of 10 or less. 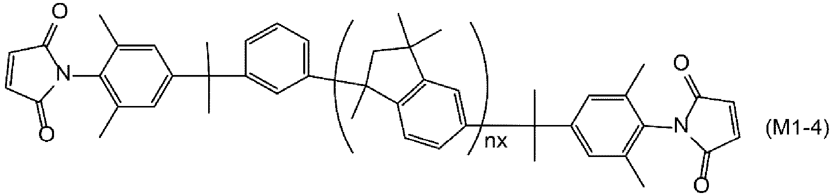 (In Formula (M1-4), nx represents an integer of 1 or more and 20 or less.)

nx may be an integer of 10 or less.

The molecular weight of the compound represented by Formula (M1) is preferably 500 or more, more preferably 600 or more, and even more preferably 700 or more. By setting the molecular weight to the lower limit or more, the low dielectric properties and low water absorption of the resulting cured product tend to be further improved. The molecular weight of the compound represented by Formula (M1) is preferably 10000 or less, more preferably 9000 or less, even more preferably 7000 or less, still more preferably 5000 or less, and yet more preferably 4000 or less. By setting the molecular weight to the upper limit or less, the heat resistance and handleability of the resulting cured product tend to be further improved.

The maleimide group equivalent of the compound represented by Formula (M1) is preferably 50 g/eq. or more, more preferably 100 g/eq. or more, and even more preferably 200 g/eq. or more. The upper limit of the maleimide equivalent is preferably 2000 g/eq. or less, more preferably 1000 g/eq. or less, and even more preferably 800 g/eq. or less. Here, the maleimide group equivalent represents the mass of a maleimide compound per equivalent of a maleimide group. When the maleimide group equivalent of the compound represented by Formula (M1) is within the aforementioned ranges, the low dielectric properties, low water absorption, heat resistance, and handleability of the resulting cured product tend to be further improved.

The compound represented by Formula (M1) has a molecular weight distribution Mw/Mn, calculated from gel permeation chromatography (GPC) measurement, of preferably from 1.0 to 4.0, more preferably from 1.1 to 3.8, even more preferably from 1.2 to 3.6, and still more preferably from 1.3 to 3.4. When the Mw/Mn of the compound represented by Formula (M1) is within the aforementioned ranges, the low dielectric properties, low water absorption, heat resistance, and handleability of the resulting cured product tend to be further improved.

For the details of the compound represented by Formula (M1), reference can be made to the description in WO 2020/217679 A, the contents of which are incorporated herein. 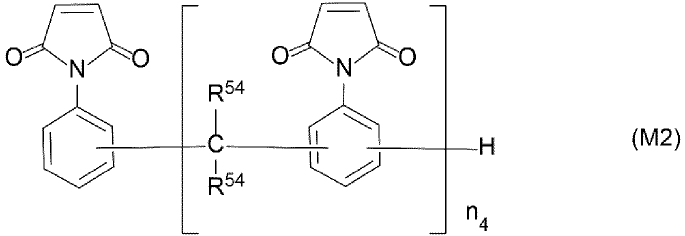 (In Formula (M2), R⁵⁴ each independently represents a hydrogen atom or a methyl group, and n₄ represents an integer of 1 or more.)

n₄ is preferably an integer from 1 to 10, more preferably an integer from 1 to 5, even more preferably an integer from 1 to 3, still more preferably 1 or 2, and may be 1.

The compound represented by Formula (M2) may be a mixture of compounds having different n₄s, and is preferably the mixture. In addition, as described in the section of the compound represented by Formula (M0), a mixture of compounds having different other moieties may be used. 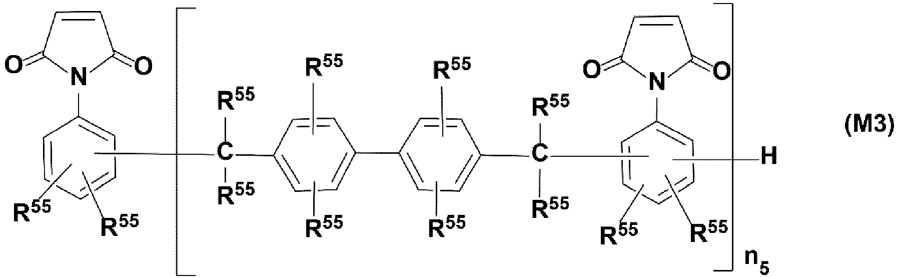 (In Formula (M3), R⁵⁵ each independently represents a hydrogen atom, an alkyl group having from 1 to 8 carbon atoms, or a phenyl group, and n₅ represents an integer of 1 or more and 10 or less.)

R⁵⁵ is each independently preferably one selected from the group consisting of a hydrogen atom, a methyl group, an ethyl group, an n-propyl group, an isopropyl group, an n-butyl group, an isobutyl group, a t-butyl group, an n-pentyl group, and a phenyl group, more preferably a hydrogen atom and/or a methyl group, and even more preferably a hydrogen atom.

n₅ is preferably an integer of 1 or more and 5 or less, more preferably an integer from 1 to 3, and even more preferably 1 or 2.

The compound represented by Formula (M3) may be a mixture of compounds having different n₅s, and is preferably the mixture. In addition, as described in the section of the compound represented by Formula (M0), a mixture of compounds having different other moieties may be used. 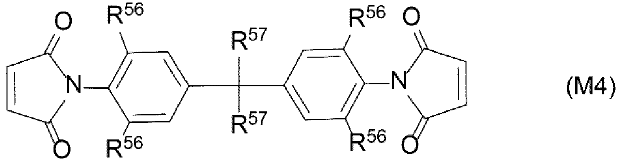 (In Formula (M4), R⁵⁶ each independently represents a hydrogen atom, a methyl group, or an ethyl group, and R⁵⁷ each independently represents a hydrogen atom or a methyl group.)

R⁵⁶ is each independently preferably a methyl group or an ethyl group, more preferably a methyl group or an ethyl group in each of the two benzene rings, and R⁵⁷ is preferably a methyl group. 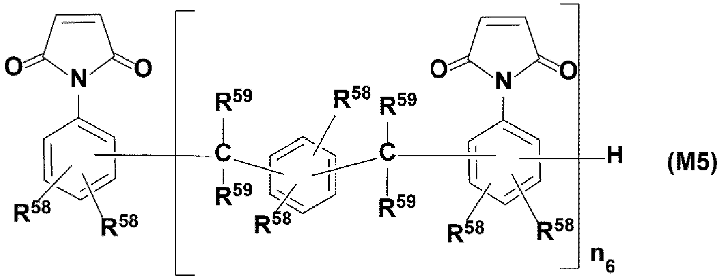 (In Formula (M5), R⁵⁸ each independently represents a hydrogen atom, an alkyl group having from 1 to 8 carbon atoms, or a phenyl group; R⁵⁹ each independently represents a hydrogen atom or a methyl group; and n₆ represents an integer of 1 or more.)

R⁵⁸ is each independently preferably one selected from the group consisting of a hydrogen atom, a methyl group, an ethyl group, an n-propyl group, an isopropyl group, an n-butyl group, an isobutyl group, a t-butyl group, an n-pentyl group, and a phenyl group, more preferably a hydrogen atom and/or a methyl group, and even more preferably a hydrogen atom.

R⁵⁹ is preferably a methyl group.

n₆ is preferably an integer from 1 to 10, more preferably an integer from 1 to 5, even more preferably an integer from 1 to 3, still more preferably 1 or 2, and may be 1.

The compound represented by Formula (M5) may be a mixture of compounds having different n₆s, and is preferably the mixture. In addition, as described in the section of the compound represented by Formula (M0), a mixture of compounds having different other moieties may be used.

The maleimide compound (M6) is a compound having a structural unit represented by Formula (M6) and maleimide groups at both terminals of the molecular chain. 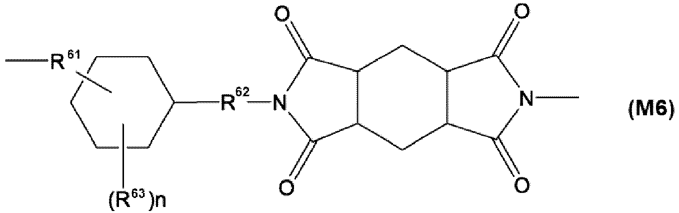 (In Formula (M6), R⁶¹ represents a linear or branched alkylene group having from 1 to 16 carbon atoms, or a linear or branched alkenylene group having from 2 to 16 carbon atoms. R⁶² represents a linear or branched alkylene group having from 1 to 16 carbon atoms, or a linear or branched alkenylene group having from 2 to 16 carbon atoms. R⁶³ each independently represents a linear or branched alkyl group having from 1 to 16 carbon atoms, or a linear or branched alkenyl group having from 2 to 16 carbon atoms. n each independently represents an integer from 0 to 10.)

The weight average molecular weight of the maleimide compound (M6) is preferably 100 or more, more preferably 300 or more, and is preferably 5000 or less, more preferably 4500 or less. By setting the weight average molecular weight of the maleimide compound (M6) to be within the aforementioned ranges, a suitable viscosity can be obtained, and an increase in viscosity of the varnish can be suppressed. The weight average molecular weight of the maleimide compound (M6) means a mass average molecular weight by a gel permeation chromatography (GPC) method calibrated with polystyrene.

Next, the maleimide compound (M6) will be described in detail.

In Formula (M6) of the maleimide compound (M6), R⁶¹ represents a linear or branched alkylene group having from 1 to 16 carbon atoms, or a linear or branched alkenylene group having from 2 to 16 carbon atoms. R⁶¹ is preferably a linear or branched alkylene group, and more preferably a linear alkylene group, from the viewpoint that an optimal viscosity is obtained and an increase in the viscosity of the varnish can be controlled.

The number of carbon atoms of the alkylene group is preferably from 2 to 14, and more preferably from 4 to 12, from the viewpoint that a more optimal viscosity is obtained and an increase in the viscosity of the varnish can be further controlled.

Examples of the linear or branched alkylene group include a methylene group, an ethylene group, a propylene group, a butylene group, a pentylene group, a hexylene group, a heptylene group, an octylene group, a nonylene group, a decylene group, a dodecylene group, an undecylene group, a tridecylene group, a tetradecylene group, a pentadecylene group, and a hexadecylene group, a neopentylene group, a dimethylbutylene group, a methylhexylene group, an ethylhexylene group, a dimethylhexylene group, a trimethylhexylene group, a methylheptylene group, a dimethylheptylene group, a trimethylheptylene group, a tetramethylheptylene group, an ethylheptylene group, a methyloctylene group, a methylnonylene group, a methyldecylene group, a methyldodecylene group, a methylundecylene group, a methyltridecylene group, a methyltetradecylene group, a methylpentadecylene group, and a methylhexadecylene group.

The number of carbon atoms of the alkenylene group is preferably from 2 to 14, and more preferably from 4 to 12, from the viewpoint that a more optimal viscosity is obtained and an increase in the viscosity of the varnish can be further controlled.

Examples of the linear or branched alkenylene group include a vinylene group, a 1-methylvinylene group, an arylene group, a propenylene group, an isopropenylene group, a 1-butenylene group, a 2-butenylene group, a 1-pentenylene group, a 2-pentenylene group, an isopentylene group, a cyclopentenylene group, a cyclohexenylene group, and a dicyclopentadienylene group.

In Formula (M6), R⁶² represents a linear or branched alkylene group having from 1 to 16 carbon atoms, or a linear or branched alkenylene group having from 2 to 16 carbon atoms. R⁶² is preferably a linear or branched alkylene group, and more preferably a linear alkylene group, from the viewpoint that an optimal viscosity is obtained and an increase in the viscosity of the varnish can be controlled.

The number of carbon atoms of the alkylene group is preferably from 2 to 14, and more preferably from 4 to 12, from the viewpoint that a more optimal viscosity is obtained and an increase in the viscosity of the varnish can be further controlled.

Examples of the linear or branched alkylene group include the same groups as those described for the R⁶¹.

The number of carbon atoms of the alkenylene group is preferably from 2 to 14, and more preferably from 4 to 12, from the viewpoint that a more optimal viscosity is obtained and an increase in the viscosity of the varnish can be further controlled.

Examples of the linear or branched alkenylene group include the same groups as those described for the R⁶¹.

In Formula (M6), R⁶¹ and R⁶² may be the same as or different from each other, but are preferably the same as each other because the maleimide compound (M6) can be more easily synthesized.

In Formula (M6), R⁶³ each independently represents a linear or branched alkyl group having from 1 to 16 carbon atoms or a linear or branched alkenyl group having from 2 to 16 carbon atoms. R⁶³ is each independently preferably a linear or branched alkyl group having from 1 to 16 carbon atoms, from the viewpoint that an optimal viscosity is obtained and an increase in the viscosity of the varnish can be controlled; and from one to five of R⁶³s are preferably linear or branched alkyl groups each having from 1 to 16 carbon atoms, and from one to three of them are more preferably linear or branched alkyl groups each having from 1 to 16 carbon atoms. In the present embodiment, the moiety, other than the linear or branched alkyl group having from 1 to 16 carbon atoms, is preferably a hydrogen atom.

The number of carbon atoms of the alkyl group is preferably from 2 to 14, and more preferably from 4 to 12, from the viewpoint that a more optimal viscosity is obtained and an increase in the viscosity of the varnish can be further controlled.

Examples of the linear or branched alkyl group include a methyl group, an ethyl group, an n-propyl group, an isopropyl group, an n-butyl group, a 2-butyl group, an isobutyl group, a tert-butyl group, an n-pentyl group, a 2-pentyl group, a tert-pentyl group, a 2-methylbutyl group, a 3-methylbutyl group, a 2,2-dimethylpropyl group, an n-hexyl group, a 2-hexyl group, a 3-hexyl group, a 2-methylpentyl group, a 3-methylpentyl group, a 4-methylpentyl group, and a 2-methylpentane -3-yl group.

The number of carbon atoms of the alkenyl group is preferably from 2 to 14, and more preferably from 4 to 12, from the viewpoint that a more optimal viscosity is obtained and an increase in the viscosity of the varnish can be further controlled.

Examples of the linear or branched alkenyl group include a vinyl group, an allyl group, a 4-pentenyl group, an isopropenyl group, and an isopentyl group.

In Formula (M6), n each independently represents an integer from 0 to 10, and is preferably 1 or more, more preferably 5 or more, and is preferably 9 or less, more preferably 7 or less.

The maleimide compound (M6) has maleimide groups at both terminals of a molecular chain. In the present embodiment, the both terminals mean both terminals of a molecular chain of the maleimide compound (M6). For example, when the structural units represented by Formula (M6) are at the terminals of a molecular chain of the maleimide compound (M6), the maleimide groups are at the terminals of the molecular chain of R⁶¹; the maleimide groups are at the terminals of the molecular chain at the N atom of the maleimide ring; or the maleimide groups are at the terminals of both of them. The maleimide compound (M6) may have the maleimide group at a position other than both terminals of the molecular chain.

In the present embodiment, the maleimide groups bonded to the maleimide compound (M6) may be the same or different, but the maleimide groups at both terminals of the molecular chain are preferably the same.

Examples of such a maleimide compound (M6) include maleimide compounds represented by the following Formula (M6-1). These can be used singly or in combination of two or more kinds thereof as appropriate. 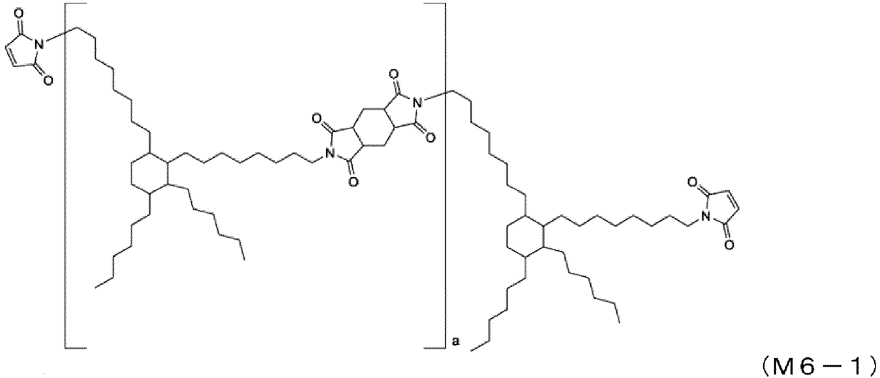 (In Formula (M6-1), a represents an integer from 1 to 10.)
a is preferably an integer from 1 to 6 from the viewpoint that a more optimal viscosity is obtained and an increase in the viscosity of the varnish can be further controlled.

As the maleimide compound (M6), a commercially available product can also be used. Examples of the commercially available product include MIZ-001 available from Nippon Kayaku Co., Ltd.

For the method for manufacturing the maleimide compound (M6), reference can be made to the description in paragraphs from 0061 to 0066 of WO 2020/262577 A, the contents of which are incorporated herein.

The additional maleimide compound may be manufactured by a known method, or a commercially available product may be used. Examples of the commercially available product include "BMI-80" available from K-I CHEMICAL INDUSTRY CO., LTD. as the compound represented by Formula (M0), "NE-X-9470S" and "NE-X-9480S" available from DIC Corporation as the compound represented by Formula (M1), "BMI-2300" available from Daiwa Fine Chemicals Co., Ltd. as the compound represented by Formula (M2), "MIR-3000-70MT" available from Nippon Kayaku Co., Ltd. as the compound represented by Formula (M3), "BMI-70" available from K-I CHEMICAL INDUSTRY CO., LTD. as the compound represented by Formula (M4), and "MIR-5000" available from Nippon Kayaku Co., Ltd. as the compound represented by Formula (M5).

Examples of the additional maleimide compound other than those described above include oligomers of N-phenylmaleimide, N-cyclohexylmaleimide, and phenylmethane maleimide, m-phenylene bismaleimide, 4-methyl-1,3-phenylene bismaleimide, 1,6-bismaleimide-(2,2,4-trimethyl)hexane, 4,4'-diphenylether bismaleimide, 4,4'-diphenylsulfone bismaleimide, 1,3-bis(3-maleimidophenoxy)benzene, 1,3-bis(4-maleimidophenoxy)benzene, and prepolymers of these, and prepolymers of these maleimides and amine.

In particular, a monofunctional maleimide compound, such as N-phenylmaleimide or N-cyclohexylmaleimide, tends to be used in combination with a polymer having the structural unit represented by the aforementioned Formula (V) to yield a resin composition that can provide a cured product more excellent in low dielectric properties.

When the resin composition of the present embodiment contains the additional maleimide compound (preferably, a compound represented by Formula (M1)), the lower limit of the content thereof is preferably 1 part by mass or more, more preferably 5 parts by mass or more, even more preferably 10 parts by mass or more, and may be 20 parts by mass or more, based on 100 parts by mass of the resin solid content in the resin composition. When the content of the additional maleimide compound is the aforementioned lower limit or more, the low dielectric properties and flame resistance of the resulting cured product tend to be improved. The upper limit of the content of the additional maleimide compound is preferably 70 parts by mass or less, more preferably 60 parts by mass or less, even more preferably 50 parts by mass or less, and may be 40 parts by mass or less, based on 100 parts by mass of the resin solid content in the resin composition. When the content of the additional maleimide compound is the aforementioned upper limit or less, metal foil peel strength and low water absorption tend to be improved.

The resin composition in the present embodiments may contain only one additional maleimide compound or may contain two or more additional maleimide compounds. When two or more kinds thereof are contained, the total amount thereof is preferably within the aforementioned ranges.

In addition, the resin composition of the present embodiment can also be formed to be substantially free of the additional maleimide compound. The phrase "substantially free" means that the content of the additional maleimide compound is less than 0.1 parts by mass, based on 100 parts by mass of the resin solid content in the resin composition.

### <<Compound containing polymerizable carbon-carbon unsaturated double bond>>

The resin composition of the present embodiment may contain a compound containing a polymerizable carbon-carbon unsaturated double bond (hereinafter in the present specification, it may be simply referred to as a "compound containing a carbon-carbon unsaturated double bond"). When the resin composition of the present embodiment contains a compound containing a carbon-carbon unsaturated double bond, low dielectric properties, low water absorption, chemical resistance, and desmear resistance tend to be excellent.

As the compound containing a carbon-carbon unsaturated double bond used in the present embodiment, compounds, usually used in printed wiring boards and containing a carbon-carbon unsaturated double bond, can be widely used.

In particular, the lower limit of the number of carbon-carbon unsaturated double bonds of the compound containing a carbon-carbon unsaturated double bond used in the present embodiment is preferably 2 or more, the upper limit thereof is preferably 2000 or less, may be 10 or less, and may further be 5 or less. In the present embodiment, the number of carbon-carbon unsaturated double bonds of the compound containing a carbon-carbon unsaturated double bond may be 2. By setting the number of carbon-carbon unsaturated double bonds to the aforementioned upper limit or less, moisture absorption heat resistance tends to be further improved.

In addition, the cured product of a compound containing a carbon-carbon unsaturated double bond (preferably, a compound containing two or more carbon-carbon unsaturated double bonds, and the same applies hereinafter) used in the present embodiment is preferably excellent in low dielectric properties. For example, the cured product of the compound containing a carbon-carbon unsaturated double bond used in the present embodiment has a dielectric constant (Dk), at a frequency of 10 GHz measured in accordance with a cavity resonator perturbation method, of preferably 3.0 or less, and more preferably 2.6 or less. The lower limit of the dielectric constant is practically, for example, 2.0 or more. In addition, the cured product of the compound containing a carbon-carbon unsaturated double bond used in the present embodiment has a dielectric loss tangent (Df), at a frequency of 10 GHz measured in accordance with a cavity resonator perturbation method, of preferably 0.010 or less, and more preferably 0.007 or less. The lower limit of the dielectric loss tangent is practically, for example, 0.0001 or more. The dielectric constant and the dielectric loss tangent can be measured according to, for example, the methods (curing conditions, measurement conditions) described in Examples.

In addition, the cured product of the compound containing a carbon-carbon unsaturated double bond used in the present embodiment preferably has high heat resistance. The cured product of the compound containing a carbon-carbon unsaturated double bond used in the present embodiment has a glass transition temperature, measured in accordance with JIS C6481 dynamic viscoelasticity measurement, of preferably 100°C or higher, more preferably 130°C or higher, even more preferably 150°C or higher, still more preferably 180°C or higher, and yet more preferably 200°C or higher. By setting the glass transition temperature to the aforementioned lower limit or more, a cured product with excellent heat resistance is obtained. In addition, the upper limit of the glass transition temperature is practically 400°C or lower.

The compound containing a carbon-carbon unsaturated double bond has a number average molecular weight Mn, by a GPC method calibrated with polystyrene, of preferably 195 or more, more preferably 400 or more, even more preferably 500 or more, and still more preferably 800 or more, and it may be 1000 or more, 1200 or more, 1500 or more, or 1800 or more. By setting the number average molecular weight to the aforementioned lower limit or more, low dielectric properties tend to be further improved. The compound containing a carbon-carbon unsaturated double bond has a number average molecular weight Mn of preferably 130000 or less, more preferably 120000 or less, and even more preferably 100000 or less, and it may be 50000 or less, 10000 or less, 7000 or less, 5000 or less, 3500 or less, 3000 or less, or 2500 or less. By setting the number average molecular weight to the aforementioned upper limit or less, a resin composition having good moldability is obtained.

The compound containing a carbon-carbon unsaturated double bond has a weight average molecular weight Mw, by a GPC method calibrated with polystyrene, of preferably 195 or more, more preferably 400 or more, even more preferably 500 or more, and still more preferably 800 or more, and it may be 1,0001200 or more, 1500 or more, or 1800 or more. By setting the weight average molecular weight to the aforementioned lower limit or more, low dielectric properties tend to be further improved. **In** addition, the compound containing a carbon-carbon unsaturated double bond has a weight average molecular weight of preferably 160000 or less, more preferably 150000 or less, even more preferably 140000 or less, still more preferably 130000 or less, and yet more preferably 100000 or less, and it may be 80000 or less, 50000 or less, or 10000 or less. By setting the weight average molecular weight to the aforementioned upper limit or less, heat resistance tends to be further improved.

The carbon-carbon unsaturated double bond contained in the compound containing a carbon-carbon unsaturated double bond used in the present embodiment is a polymerizable unsaturated group, which is usually a carbon-carbon unsaturated double bond that reacts with some reactive functional group.

The compound containing a carbon-carbon unsaturated double bond preferably contains a carbon-carbon unsaturated double bond at its terminal, and more preferably contains two or more carbon-carbon unsaturated double bonds at its terminals.

Specifically, the compound containing a carbon-carbon unsaturated double bond used in the present embodiment preferably contains at least one selected from the group consisting of a compound containing a vinylaryl group, a compound containing a (meth)acryloyl group, and a compound containing a (meth)allyl group, more preferably contains at least one selected from the group consisting of a compound containing a vinylaryl group and a compound containing a (meth)allyl group, and it is even more preferably a compound containing a vinylaryl group, and still more preferably a compound containing a vinylphenyl group. The compound containing a vinylphenyl group may be a compound containing a vinylbenzyl group.

The compound containing a (meth)acryloyl group is preferably a compound containing a methacrylate group, and the compound containing a (meth)allyl group is preferably a compound containing an allyl group.

The resin composition of the present embodiment may also be formed to be substantially free of a compound containing an allyl group. The phrase "substantially free" means that the content of the compound containing an allyl group is less than 1 part by mass, and preferably less than 0.1 parts by mass, based on 100 parts by mass of the total of the additional resin components (C).

**In** particular, the resin composition of the present embodiment may also be formed to be substantially free of diallylbisphenol and a derivative thereof. The phrase "substantially free" means that the content of diallyl bisphenol and a derivative thereof is less than 1 part by mass, and preferably less than 0.1 parts by mass, based on 100 parts by mass of the total of the additional resin components (C).

More specifically, one embodiment of the compound containing a carbon-carbon unsaturated double bond used in the present embodiment is a polyphenylene ether compound containing two or more carbon-carbon unsaturated double bonds, and in particular, a polyphenylene ether compound having a vinylaryl group and/or a polyphenylene ether compound having a (meth)acryloyl group are/is preferred, and a polyphenylene ether compound having a vinylaryl group is more preferred.

**In** addition, another embodiment of the compound containing a carbon-carbon unsaturated double bond used in the present embodiment is a polymer having a structural unit represented by Formula (V). 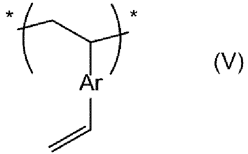 (In Formula (V), Ar represents an aromatic hydrocarbon linking group. * represents a bond position.)

Still another embodiment of the compound containing a carbon-carbon unsaturated double bond used in the present embodiment is a compound containing a (meth)allyl group, and is preferably an allyl group-substituted nadiimide compound.

Details of these preferred embodiments will be described later.

The compound containing a carbon-carbon unsaturated double bond used in the present embodiment may also contain a structural unit derived from maleic anhydride. For the details of such a resin, reference can be made to the description in WO 2017/209108 A, the contents of which are incorporated herein.

Furthermore, the compound containing a carbon-carbon unsaturated double bond may contain a structural unit derived from a compound having an acid group and an acid anhydride group.

The compound containing a carbon-carbon unsaturated double bond used in the present embodiment may also be a compound having an indane skeleton having, at its terminal, a carbon-carbon unsaturated double bond. For such a compound, reference can be made to the description in paragraphs from 0011 to 0025 of WO 2023/176766 A, the description in paragraphs from 0012 to 0033 of WO 2023/176764 A, the description in paragraphs from 0012 to 0033 of WO 2023/176763 A, and paragraphs from 0026 to 0043 of WO 2023/176765 A, the contents of which are incorporated herein.

In the resin composition of the present embodiment, the content (total amount) of the compound containing a carbon-carbon unsaturated double bond is preferably 1 part by mass or more, more preferably 5 parts by mass or more, even more preferably 10 parts by mass or more, still more preferably 20 parts by mass or more, and may be 30 parts by mass or more or 35 parts by mass or more, based on 100 parts by mass of the resin solid content in the resin composition. By setting the content of the compound to the aforementioned lower limit or more, low water absorption, low dielectric properties (low dielectric constant and/or low dielectric loss tangent), chemical resistance, and desmear resistance tend to be further improved. The upper limit of the content (total amount) of the compound containing a carbon-carbon unsaturated double bond is preferably 70 parts by mass or less, more preferably 60 parts by mass or less, even more preferably 50 parts by mass or less, and may be 40 parts by mass or less, based on 100 parts by mass of the resin solid content in the resin composition. By setting the content of the compound to the aforementioned upper limit or less, heat resistance tends to be further improved.

The resin composition of the present embodiment may contain only one kind or two or more kinds of the compound containing a carbon-carbon unsaturated double bond. When two or more kinds thereof are contained, the total amount thereof is preferably within the aforementioned ranges.

In addition, the resin composition of the present embodiment can also be formed to be substantially free of the compound containing a carbon-carbon unsaturated double bond. The phrase "substantially free" means that the content of the compound containing a carbon-carbon unsaturated double bond is less than 0.1 parts by mass, based on 100 parts by mass of the resin solid content in the resin composition.

### <<<Polyphenylene ether compound containing carbon-carbon unsaturated double bond>>>

In the resin composition of the present embodiment, the compound containing a polymerizable carbon-carbon unsaturated double bond preferably contains a polyphenylene ether compound containing a carbon-carbon unsaturated double bond, and more preferably contains a polyphenylene ether compound containing two or more carbon-carbon unsaturated double bonds. The polyphenylene ether compound containing two or more carbon-carbon unsaturated double bonds is preferably a polyphenylene ether compound containing two or more vinylbenzyl groups.

In addition, the polyphenylene ether compound containing two or more carbon-carbon unsaturated double bonds preferably contains a carbon-carbon unsaturated double bond at its terminal, and is preferably a polyphenylene ether compound containing two or more vinylbenzyl groups at its terminals.

Examples of the polyphenylene ether compound containing two or more carbon-carbon unsaturated double bonds include a compound containing a phenylene ether skeleton represented by the following Formula (X1). 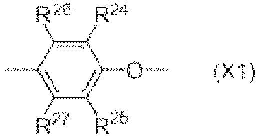 (In Formula (X1), R²⁴, R²⁵, R²⁶, and R²⁷ may be the same or different, and each represent an alkyl group having 6 or less carbon atoms, an aryl group, halogen, or a hydrogen atom.)

The polyphenylene ether compound containing two or more unsaturated carbon-carbon double bonds may further include a repeating unit represented by Formula (X2): 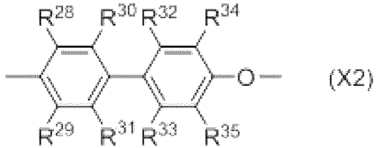 (In Formula (X2), R²⁸, R²⁹, R³⁰, R³⁴, and R³⁵ may be the same or different, and each represent an alkyl group having 6 or less carbon atoms or a phenyl group; R³¹, R³², and R³³ may be identical to or different from one another, and each represent a hydrogen atom, an alkyl group having 6 or less carbon atoms, or a phenyl group)
and/or a repeating unit represented by Formula (X3): 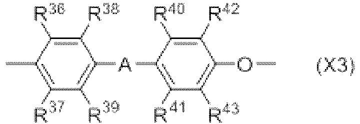 (In Formula (X3), R³⁶, R³⁷, R³⁸, R¹⁹, R⁴⁰, R⁴¹, R⁴², and R⁴³ may be identical to or different from one another, and each represent a hydrogen atom, an alkyl group having 6 or less carbon atoms, or a phenyl group; and -A- is a linear, branched, or cyclic divalent hydrocarbon having 20 or less carbon atoms.)

The polyphenylene ether compound containing two or more carbon-carbon unsaturated double bonds is preferably a modified polyphenylene ether compound in which a part or all of the terminals is functionalized with an ethylenically unsaturated group (hereinafter, the modified polyphenylene ether compound may be referred to as a "modified polyphenylene ether compound (g)"), more preferably a modified polyphenylene ether compound having two or more groups selected from the group consisting of a (meth)acryloyl group, a maleimide group, a (meth)allyl group, and a vinylbenzyl group at its terminals, even more preferably a modified polyphenylene ether compound having two or more groups selected from the group consisting of a (meth)acryloyl group and a vinylbenzyl group, and still more preferably a modified polyphenylene ether compound having two or more vinylbenzyl groups from the viewpoint of being excellent in chemical resistance and desmear resistance. By adopting such a modified polyphenylene ether compound (g), the dielectric loss tangent (Df) of the cured product of the resin composition can be further reduced. These may be used singly or in combination of two or more kinds thereof. By adopting such a modified polyphenylene ether compound (g), Df can be further reduced and peel strength can be increased. These may be used singly or in combination of two or more kinds thereof.

The method for manufacturing the modified polyphenylene ether compound (g) is not particularly limited as long as the effects of the present invention can be obtained. For example, the modified polyphenylene ether compound functionalized with an ethylenically unsaturated group (specifically, a vinylbenzyl group or the like) can be manufactured by dissolving a bifunctional phenylene ether oligomer and vinylbenzyl chloride in a solvent, reacting them by adding a base under heating and stirring, and then solidifying the resin. The modified polyphenylene ether compound functionalized with a carboxy group is manufactured, for example, by melt-kneading an unsaturated carboxylic acid or a functionalized derivative thereof with a polyphenylene ether compound in the presence or absence of a radical initiator and reacting the mixture. Alternatively, the modified polyphenylene ether compound is manufactured by dissolving a polyphenylene ether compound, an unsaturated carboxylic acid, and a functional derivative thereof in an organic solvent in the presence or absence of a radical initiator, and reacting them in a solution.

Examples of the ethylenically unsaturated group contained in the modified polyphenylene ether compound (g) include: alkenyl groups such as an ethenyl group, an allyl group, a methallyl group, an acryloyl group, a methacryloyl group, a propenyl group, a butenyl group, a hexenyl group, and an octenyl group; cyclic alkenyl groups such as a cyclopentenyl group and a cyclohexenyl group; and vinylaryl groups such as a vinylphenyl group and a vinylnaphthyl group. The ethylenically unsaturated group is preferably a methacryloyl group and/or a vinylbenzyl group, and is more preferably a vinylbenzyl group from the viewpoint of being excellent in chemical resistance and desmear resistance. Examples of the vinylbenzyl group include a 4-vinylbenzyl group and a 3-vinylbenzyl group. **In** the modified polyphenylene ether compound (g), two or more ethylenically unsaturated groups may be the same functional group or different functional groups. By adopting such a modified polyphenylene ether compound, Df can be further reduced and peel strength can be increased.

Examples of the modified polyphenylene ether compound (g) include a polyphenylene ether compound represented by Formula (OP). 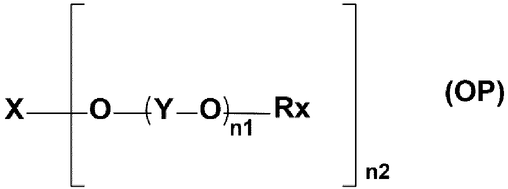 (In Formula (OP), X represents an aromatic group, -(Y-O)ₐ₁- represents a polyphenylene ether structure, n1 represents an integer from 1 to 100, and n2 represents an integer from 1 to 4. Rx is a group represented by Formula (Rx-1) or Formula (Rx-2).) 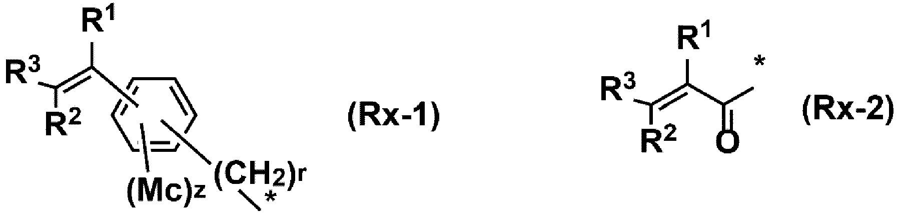 (In Formula (Rx-1) and Formula (Rx-2), R¹, R², and R³ each independently represent a hydrogen atom, an alkyl group, an alkenyl group, or an alkynyl group. * is a binding site to an oxygen atom. Mc each independently represents a hydrocarbon group having from 1 to 12 carbon atoms. z represents an integer from 0 to 4. r represents an integer from 0 to 6).

The aromatic group represented by the X may or may not have a substituent on the benzene ring, but preferably has the substituent. In the case of having a substituent, the above-described substituents Z can be taken as examples, and the substituent is preferably at least one selected from the group consisting of an alkyl group having 6 or less carbon atoms, an aryl group, and a halogen atom, more preferably an alkyl group having 3 or less carbon atoms, and even more preferably a methyl group.

The polyphenylene ether structure represented by the -(Y-O)n₁- may or may not have a substituent on the benzene ring, but preferably has the substituent. In the case of having a substituent, the aforementioned substituents Z can be taken as examples, and the substituent is preferably an alkyl group having 6 or less carbon atoms or a phenyl group, more preferably an alkyl group having 3 or less carbon atoms, and even more preferably a methyl group.

When n₁ and/or n₂ are/is each an integer of 2 or more, n₁ structural units (Y-O) and/or n₂ structural units may be the same as or different from each other. n₂ is preferably 2 or more, and more preferably 2.

In Formula (Rx-1) and Formula (Rx-2), R¹, R², and R³ each independently represent a hydrogen atom, an alkyl group, an alkenyl group, or an alkynyl group.

R¹ is preferably a hydrogen atom or an alkyl group, more preferably a hydrogen atom or a methyl group, and even more preferably a hydrogen atom.

R² and R³ are each independently preferably a hydrogen atom or an alkyl group, more preferably a hydrogen atom or a methyl group, and even more preferably a hydrogen atom.

The number of carbon atoms of each of the alkyl group, the alkenyl group, and the alkynyl group, as R¹, R², and R³, is preferably 5 or less, and more preferably 3 or less.

In Formula (Rx-1), r represents an integer from 0 to 6, and may be an integer of 1 or more, and preferably an integer of 5 or less, more preferably an integer of 4 or less, even more preferably an integer of 3 or less, still more preferably 1 or 2, and yet more preferably 1.

In Formula (Rx-1), Mc each independently represents a hydrocarbon group having from 1 to 12 carbon atoms, and is preferably a hydrocarbon group having from 1 to 10 carbon atoms, more preferably a linear or branched alkyl group having from 1 to 10 carbon atoms, even more preferably a methyl group, an ethyl group, an isopropyl group, an isobutyl group, a t-butyl group, a pentyl group, an octyl group, or a nonyl group, and still more preferably a methyl group, an ethyl group, an isopropyl group, an isobutyl group, or a t-butyl group.

In Formula (Rx-1), z represents an integer from 0 to 4, and is preferably an integer from 0 to 3, more preferably an integer from 0 to 2, even more preferably 0 or 1, and still more preferably 0.

A specific example of the group represented by Formula (Rx-1) is a vinylbenzyl group, and a specific example of the group represented by Formula (Rx-2) is a (meth)acryloyl group.

Rx is preferably a group represented by Formula (Rx-2).

It is also preferable that the resin composition of the present embodiment contain, as the polyphenylene ether compound having a carbon-carbon unsaturated double bond, both a polyphenylene ether compound that is a compound represented by Formula (OP) and has a group represented by Formula (Rx-1) and a compound having a polyphenylene ether group having a group represented by Formula (Rx-2).

Examples of the modified polyphenylene ether compound (g) include a compound represented by Formula (OP-1). 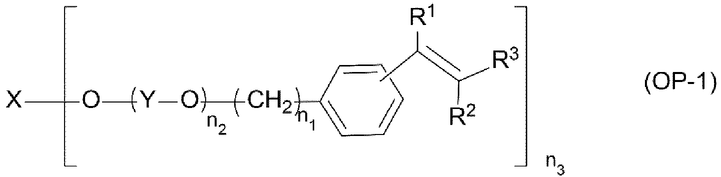 (In Formula (OP-1), X represents an aromatic group; -(Y-O)n₂- represents a polyphenylene ether structure; R¹, R², and R³ each independently represent a hydrogen atom, an alkyl group, an alkenyl group, or an alkynyl group; n₁ represents an integer from 1 to 6; n₂ represents an integer from 1 to 100; and n₃ represents an integer from 2 to 4.)

When n₂ and/or n₃ are/is each an integer of 2 or more, n₂ structural units (Y-O) and/or n₃ structural units may be the same as or different from each other. n₃ is preferably 2 or more, and more preferably 2.

The modified polyphenylene ether compound (g) in the present embodiment is preferably represented by Formula (OP-2).

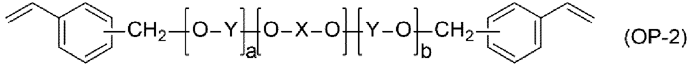

In this case, -(O-X-O)- is preferably represented by Formula (OP-3): 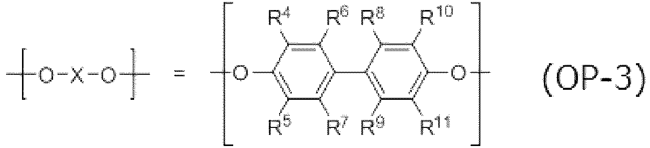 (In Formula (OP-3), R⁴, R⁵, R⁶, R¹⁰, and R¹¹ may be identical to or different from one another, and are each an alkyl group having 6 or less carbon atoms or a phenyl group; and R⁷, R⁸, and R⁹ may be the same or different, and are each a hydrogen atom, an alkyl group having 6 or less carbon atoms, or a phenyl group),
and/or Formula (OP-4): 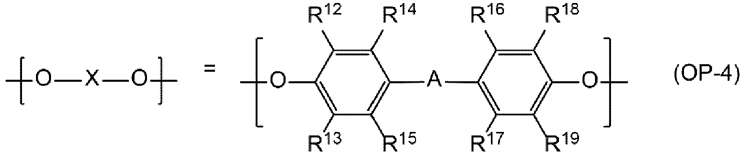 (In Formula (PO-4), R¹², R¹³, R¹⁴, R¹⁵, R¹⁶, R¹⁷, R¹⁸, and R¹⁹ may be the same or different, and are each a hydrogen atom, an alkyl group having 6 or less carbon atoms, or a phenyl group. -A-is a linear, branched, or cyclic divalent hydrocarbon group having 20 or less carbon atoms).

Moreover, -(Y-O)- is preferably represented by Formula (OP-5): 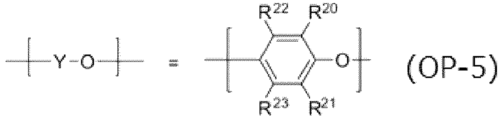 (In Formula (OP-5), R²⁰ and R²¹ may be identical to or different from each other, and are each an alkyl group having 6 or less carbon atoms or a phenyl group; R²² and R²³ may be the same or different, and are each a hydrogen atom, an alkyl group having 6 or less carbon atoms, or a phenyl group).

In Formula (OP-2), a and b each represent an integer from 0 to 100, at least one of which is not 0, and is preferably an integer from 0 to 50, and more preferably an integer from 1 to 30. When a and/or b are/is each an integer of 2 or more, two or more -(Y-O)- may each independently have one structure arranged, or two or more structures arranged in blocks or randomly.

Examples of -A- in Formula (OP-4) include, but are not limited to, divalent organic groups such as a methylene group, an ethylidene group, a 1-methylethylidene group, a 1,1-propylidene group, a 1,4-phenylenebis(1-methylethylidene) group, a 1,3-phenylenebis(1-methylethylidene) group, a cyclohexylidene group, a phenylmethylene group, a naphthylmethylene group, and a 1-phenylethylidene group.

Among the aforementioned modified polyphenylene ether compounds (g), preferred is a polyphenylene ether compound in which R⁴, R⁵, R⁶, R¹⁰, R¹¹, R²⁰, and R²¹ are alkyl groups each having 3 or less carbon atoms, and R⁷, R⁸, R⁹, R¹², R¹³, R¹⁴, R¹⁵, R¹⁶, R¹⁷, R¹⁸, R¹⁹, R²², and R²³ are hydrogen atoms or alkyl groups each having 3 or less carbon atoms. In particular, it is more preferable that: -(O-X-O)- represented by Formula (OP-3) or Formula (OP-4) is Formula (OP-9), Formula (OP-10), and/or Formula (OP-11); and -(Y-O)- represented by Formula (OP-5) is Formula (OP-12) or Formula (OP-13). It is even more preferable that the -(O-X-O)- is Formula (OP-9) and the -(Y-O)- is Formula (OP-12). When a and/or b are/is each an integer of 2 or more, two or more -(Y-O)- may each independently have a structure in which two or more of Formula (OP-12) and/or Formula (OP-13) are arranged, or a structure in which Formula (OP-12) and Formula (OP-13) are arranged in blocks or randomly. 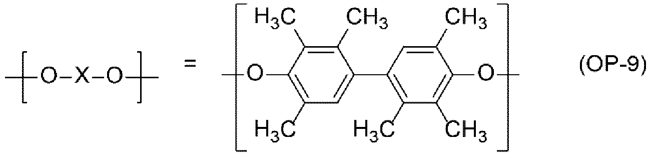 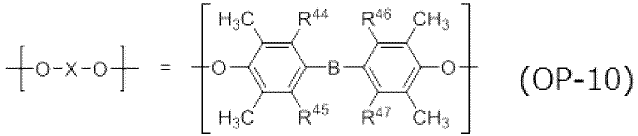 (In Formula (OP-10), R⁴⁴, R⁴¹, R⁴⁶, and R⁴⁷ may be the same or different, and are hydrogen atoms or methyl groups. -B- is a linear, branched, or cyclic divalent hydrocarbon group having 20 or less carbon atoms.)

Specific examples of -B- include the same as the specific examples of -A- in Formula (OP-4). 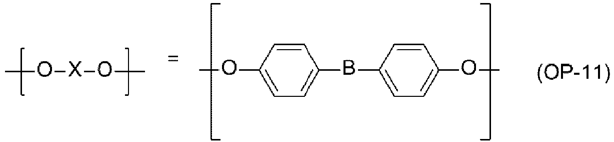 (In Formula (OP-11), -B- is a linear, branched, or cyclic divalent hydrocarbon group having 20 or less carbon atoms.)

Specific examples of -B- include the same as the specific examples of -A- in Formula (OP-4).

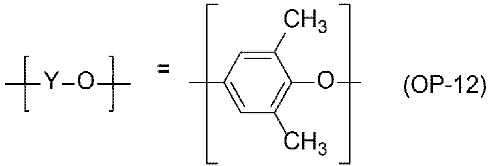

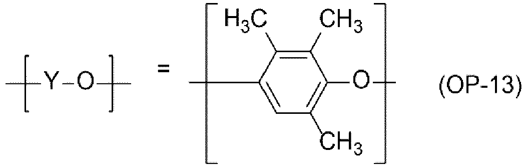

The modified polyphenylene ether compound (g) is more preferably a compound represented by Formula (OP-14) and/or a compound represented by Formula (OP-15), and even more preferably the compound represented by Formula (OP-15). 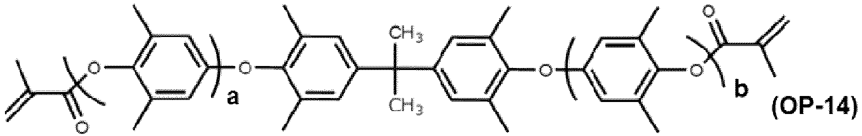 (In Formula (OP-14), a and b each independently represent an integer from 0 to 100, at least one of which is an integer from 1 to 100.)

a and b in Formula (OP-14) each independently have the same meaning as a and b in Formula (OP-2), and preferred ranges thereof are also the same. 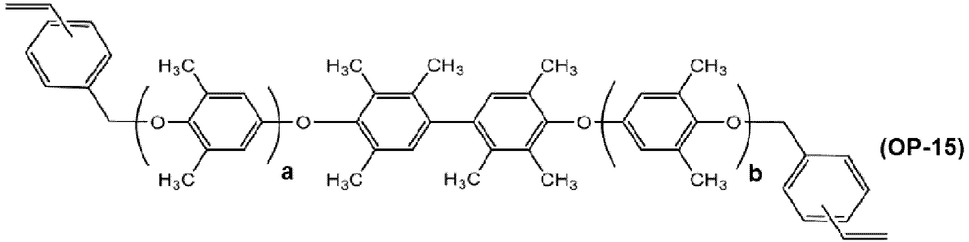 (In Formula (OP-15), a and b each independently represent an integer from 0 to 100, at least one of which is an integer from 1 to 100.)

a and b in Formula (OP-15) each independently have the same meaning as a and b in Formula (OP-2), and preferred ranges thereof are also the same.

The polyphenylene ether compound containing two or more carbon-carbon unsaturated double bonds may be manufactured by a known method, or a commercially available product may be used. Examples of the commercially available product include "SA9000" available from SABIC Innovative Plastics as a modified polyphenylene ether compound whose terminal is a methacryloyl group. Examples of the modified polyphenylene ether compound whose terminal is a vinylbenzyl group include "OPE-2St1200" and "OPE-2st2200" available from MITSUBISHI GAS CHEMICAL COMPANY, INC. As the modified polyphenylene ether compound whose terminal is a vinylbenzyl group, a compound, obtained by modifying the hydroxyl group at the terminal of a polyphenylene ether compound, such as "SA90" available from SABIC Innovative Plastics, to a vinylbenzyl group using vinylbenzyl chloride or the like, can also be used.

In addition, for the details of the polyphenylene ether compound containing two or more carbon-carbon unsaturated double bonds, reference can be made to the descriptions in JP 2006-028111 A, JP 2018-131519 A, WO 2019/138992 A, and WO 2022/054303 A, the contents of which are incorporated herein.

The polyphenylene ether compound having two or more carbon-carbon unsaturated double bonds (preferably, the modified polyphenylene ether compound (g)) has a number average molecular weight, by a gel permeation chromatography (GPC) method (details are according to the methods described in Examples to be described later) calibrated with polystyrene, of preferably 500 or more and 3000 or less. When the number average molecular weight is 500 or more, stickiness tends to be further suppressed when the resin composition of the present embodiment is formed into a coating film. When the number average molecular weight is 3000 or less, solubility in a solvent tends to be further improved.

The polyphenylene ether compound having two or more carbon-carbon unsaturated double bonds (preferably, the modified polyphenylene ether compound (g)) has a weight average molecular weight, by GPC (details are according to the methods described in Examples to be described later) calibrated with polystyrene, of preferably 800 or more and 10000 or less, and more preferably 800 or more and 5000 or less. By setting the weight average molecular weight to the aforementioned lower limit or more, the relative permittivity (Dk) and the dielectric loss tangent (Df) of the cured product of the resin composition tend to be further reduced. By setting the weight average molecular weight to the upper limit or less, the solubility of the resin composition in a solvent, the low viscosity, and the moldability, when a varnish or the like to be described later is produced, tend to be further improved.

Furthermore, in the case of the modified polyphenylene ether compound (g), the carbon-carbon unsaturated double bond equivalent at the terminal is preferably from 400 to 5000 g, and more preferably from 400 to 2500 g, per carbon-carbon unsaturated double bond. By setting the carbon-carbon unsaturated double bond equivalent to the aforementioned lower limit or more, the relative permittivity (Dk) and the dielectric loss tangent (Df) of the cured product of the resin composition tend to be further reduced. By setting the carbon-carbon unsaturated double bond equivalent to the aforementioned upper limit or less, the solubility of the resin composition in a solvent, the low viscosity, and the moldability tend to be further improved.

The functional group equivalent in the polyphenylene ether compound having a carbon-carbon unsaturated double bond (equivalent of the carbon-carbon unsaturated double bond) is calculated from the inverse of the amount of double bonds, which is determined from the measurement results using an infrared spectrometer.

The double bond equivalent [g/eq.] was determined as follows.

Powder of the polyphenylene ether compound is weighed, and the weight is recorded. The powder is put into a measuring flask, and then the volume is increased to a predetermined amount with carbon disulfide to prepare a measurement sample. The sample solution is placed in a measurement cell and set in an infrared spectrophotometer (FT/IR-4600, available from JASCO Corporation). Subsequently, the sample solution is subjected to infrared spectrometry. In the case of the vinyl group in the polyphenylene ether compound, the peak area of the spectrum around 905 cm⁻¹ is recorded. When the carbon-carbon unsaturated double bond has a methacrylic group, the peak area of the spectrum around 1640 cm⁻¹ is recorded. From the area values and the calibration curve, a double bond concentration [mol/L] is obtained as a measured value.

Subsequently, the double bond equivalent is calculated by the following equation. Double bond equivalent [g/eq.] = weight of powder in measurement sample [g]/double bond concentration [mol/L] × volume of measurement sample solution [L]

The functional group equivalents of other compounds can also be determined according to the method described above. However, in a compound (monomer) that can be represented by a single molecular weight, a value obtained by calculating a functional group equivalent by (theoretical molecular weight ÷ number of functional groups) is preferentially adopted.

When the resin composition of the present embodiment contains a polyphenylene ether compound containing two or more carbon-carbon unsaturated double bonds, the lower limit of the content of the polyphenylene ether compound containing two or more carbon-carbon unsaturated double bonds is preferably 1 part by mass or more, more preferably 5 parts by mass or more, even more preferably 10 parts by mass or more, still more preferably 15 parts by mass or more, yet more preferably 20 parts by mass or more, and far more preferably 25 parts by mass or more, based on 100 parts by mass of the resin solid content in the resin composition. By setting the content to the aforementioned lower limit or more, the heat resistance, low water absorption, and low dielectric properties (low dielectric constant and/or low dielectric loss tangent) of the resulting cured product tend to be further improved. The upper limit of the content of the content of the polyphenylene ether compound containing two or more carbon-carbon unsaturated double bonds is preferably 70 parts by mass or less, more preferably 60 parts by mass or less, even more preferably 50 parts by mass or less, still more preferably 40 parts by mass or less, and may be 35 parts by mass or less, based on 100 parts by mass of the resin solid content in the resin composition. By setting the content to the aforementioned upper limit or less, the moisture absorption heat resistance and the metal foil peel strength (preferably, copper foil peel strength) of the resulting cured product tend to be further improved.

The resin composition in the present embodiment may contain only one kind or two or more kinds of the polyphenylene ether compound containing two or more carbon-carbon unsaturated double bonds. When two or more kinds thereof are contained, the total amount thereof is preferably within the aforementioned ranges.

### <<<Polymer having structural unit represented by Formula (V)>>>

The resin composition of the present embodiment preferably contains a polymer having a structural unit represented by Formula (V). The polymer having the structural unit represented by Formula (V) is a polymer containing two or more carbon-carbon unsaturated double bonds, and is preferably a polymer having two or more structural units represented by Formula (V). By containing a polymer having a structural unit represented by Formula (V), a resin composition more excellent in low dielectric properties (low dielectric constant and low dielectric loss tangent) can be obtained. 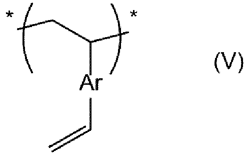 (In Formula (V), Ar represents an aromatic hydrocarbon linking group. * represents a bond position.)

The aromatic hydrocarbon linking group may be a group containing only an aromatic hydrocarbon that may have a substituent, or a group containing a combination of an aromatic hydrocarbon that may have a substituent and any other linking group, and is preferably a group containing only an aromatic hydrocarbon that may have a substituent. Note that, examples of the substituent that the aromatic hydrocarbons may have include a substituent Z (such as an alkyl group having from 1 to 6 carbon atoms, an alkenyl group having from 2 to 6 carbon atoms, an alkynyl group having from 2 to 6 carbon atoms, an alkoxy group having from 1 to 6 carbon atoms, a hydroxy group, an amino group, a carboxy group, and a halogen atom). The aromatic hydrocarbon preferably has no substituent.

The aromatic hydrocarbon linking group is typically a divalent linking group.

Specific examples of the aromatic hydrocarbon linking group include a phenylene group, a naphthalenediyl group, an anthracenediyl group, a phenanthrenediyl group, a biphenyldiyl group, and a fluorenediyl group, every of which may have a substituent, and among them, a phenylene group that may have a substituent is preferred. Examples of the substituent include the substituent Z described above, but the aforementioned groups, such as a phenylene group, preferably have no substituent.

The structural unit represented by Formula (V) more preferably includes at least one of a structural unit represented by the following Formula (V1), a structural unit represented by the following Formula (V2), and a structural unit represented by the following Formula (V3). Note that, * in the following formulas described below represents a bond position. Hereinafter, the structural units represented by Formulas (V1) to (V3) may be collectively referred to as a "structural unit (a)".

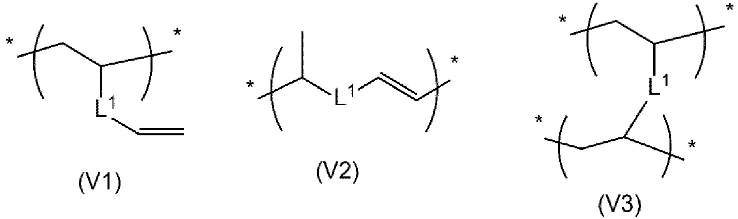

**In** Formulas (V1) to (V3), L¹ is an aromatic hydrocarbon linking group (having preferably from 6 to 22 carbon atoms, more preferably from 6 to 18 carbon atoms, and even more preferably from 6 to 10 carbon atoms). Specific examples thereof include a phenylene group, a naphthalenediyl group, an anthracenediyl group, a phenanthrenediyl group, a biphenyldiyl group, and a fluorenediyl group, every of which may have a substituent, and among them, a phenylene group that may have a substituent is preferred. Examples of the substituent include the substituent Z described above, but the aforementioned groups, such as a phenylene group, preferably have no substituent.

The compound forming the structural unit (a) is preferably a divinyl aromatic compound, and examples thereof include divinylbenzene, bis(1-methylvinyl)benzene, divinylnaphthalene, divinylanthracene, divinylbiphenyl, and divinylphenanthrene. Among them, divinylbenzene is particularly preferred. One kind of these divinyl aromatic compounds may be used, and two or more kinds thereof may be used, as necessary.

As described above, the polymer having the structural unit represented by Formula (V) may be a homopolymer of the structural unit (a), or may be a copolymer with a structural unit derived from any other monomer.

When the polymer having the structural unit represented by Formula (V) is a copolymer, the copolymerization ratio is such that the structural unit (a) is preferably 5 mol% or more, more preferably 10 mol% or more, and even more preferably 15 mol% or more. The upper limit is preferably 90 mol% or less, more preferably 85 mol% or less, even more preferably 80 mol% or less, still more preferably 70 mol% or less, yet more preferably 60 mol% or less, far more preferably 50 mol% or less, much more preferably 40 mol% or less, particularly preferably 30 mol% or less, and may be 25 mol% or less.

Examples of the structural unit (b) derived from the any other monomer include a structural unit derived from an aromatic compound having one vinyl group (monovinyl aromatic compound).

The structural unit (b) derived from the monovinyl aromatic compound is preferably a structural unit represented by the following Formula (V4).

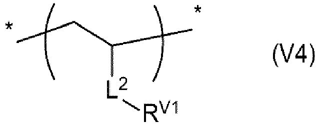

In Formula (V4), L² is an aromatic hydrocarbon linking group, and specific examples of preferred groups include the aforementioned L¹.

R^{V1} is a hydrogen atom or a hydrocarbon group having from 1 to 12 carbon atoms (preferably an alkyl group). When R^{V1} is a hydrocarbon group, the number of its carbon atoms is preferably from 1 to 6, and more preferably from 1 to 3. R^{V1} and L² may have the aforementioned substituent Z.

When the polymer having a structural unit represented by Formula (V) is a copolymer containing the structural unit (b) derived from a monovinyl aromatic compound, examples of the monovinyl aromatic compound include: vinyl aromatic compounds such as styrene, vinylnaphthalene and vinylbiphenyl; nuclear alkyl substituted vinyl aromatic compounds such as o-methylstyrene, m-methylstyrene, p-methylstyrene, o,p-dimethylstyrene, o-ethylvinylbenzene, m-ethylvinylbenzene, p-ethylvinylbenzene, methylvinylbiphenyl, and ethylvinylbiphenyl. The monovinyl aromatic compounds taken here as examples may have the aforementioned substituent Z as appropriate. These monovinyl aromatic compounds may be used singly or in combination of two or more kinds thereof.

When the polymer having the structural unit represented by Formula (V) is a copolymer containing the structural unit (b), the copolymerization ratio of the structural unit (b) is preferably 10 mol% or more, more preferably 15 mol% or more, and may be further 20 mol% or more, 30 mol% or more, 40 mol% or more, 50 mol% or more, 60 mol% or more, 70 mol% or more, or 75 mol% or more. The upper limit thereof is preferably 98 mol% or less, more preferably 90 mol% or less, and even more preferably 85 mol% or less.

The polymer having the structural unit represented by Formula (V) may have any other structural unit other than the structural unit (a) and the structural unit (b). Examples of the any other structural unit include a structural unit (c) derived from a cycloolefin compound. Examples of the cycloolefin compound include hydrocarbons having a double bond in its ring structure. Specific examples thereof include: monocyclic olefins such as cyclobutene, cyclopentene, cyclohexene, and cyclooctene; compounds having a norbornene ring structure such as norbornene and dicyclopentadiene; and cycloolefin compounds having a fused aromatic ring such as indene and acenaphthylene. Examples of the norbornene compound include those described in paragraphs from 0037 to 0043 of JP 2018-39995 A, the contents of which are incorporated herein. Note that, the cycloolefin compounds taken here as examples may further have the aforementioned substituent Z.

When the polymer having the structural unit represented by Formula (V) is a copolymer containing the structural unit (c), the copolymerization ratio of the structural unit (c) is preferably 10 mol% or more, more preferably 20 mol% or more, and even more preferably 30 mol% or more. The upper limit thereof is preferably 90 mol% or less, more preferably 80 mol% or less, even more preferably 70 mol% or less, and may be 50 mol% or less or 30 mol% or less.

The polymer having the structural unit represented by Formula (V) may further incorporate a structural unit (d) derived from a further different polymerizable compound (hereinafter, also referred to as any other polymerizable compound). Examples of the any other polymerizable compound (monomer) include compounds containing three vinyl groups. Specific examples thereof include 1,3,5-trivinylbenzene, 1,3,5-trivinylnaphthalene, and 1,2,4-trivinylcyclohexane. Alternatively, examples thereof include ethylene glycol diacrylate, butadiene (e.g., 1,3-butadiene), and isoprene. The copolymerization ratio of the structural unit (d) derived from the any other polymerizable compound is preferably 30 mol% or less, more preferably 20 mol% or less, and even more preferably 10 mol% or less.

As one embodiment of the polymer having the structural unit represented by Formula (V), a polymer, containing the structural unit (a) as an essential component and at least one of the structural units (b) to (d), can be taken as an example. Furthermore, an aspect in which the total of the structural units (a) to (d) accounts for 95 mol% or more, and further 98 mol% or more of all the structural units is taken as an example.

As another embodiment of the polymer having the structural unit represented by Formula (V), a polymer may be adopted in which the structural unit (a) is essential, and among all structural units excluding those at terminals, the structural unit containing an aromatic ring is preferably 90 mol% or more, more preferably 95 mol% or more, and may be 100 mol%.

In calculating mol% per all structural units, a single structural unit is derived from one molecule of a monomer (e.g., a divinyl aromatic compound, a monovinyl aromatic compound, and the like) used in manufacturing a polymer having the structural unit represented by Formula (V).

The method for manufacturing a polymer having the structural unit represented by Formula (V) is not particularly limited, and may follow the ordinary method, and examples thereof include polymerizing raw materials including a divinyl aromatic compound (If necessary, a monovinyl aromatic compound, a cycloolefin compound, or the like is allowed to coexist) in the presence of a Lewis acid catalyst. As the Lewis acid catalyst, a metal fluoride, such as boron trifluoride, or a complex thereof can be used.

The structure of the chain terminal of the polymer having the structural unit represented by Formula (V) is not particularly limited, but examples of the group derived from the divinyl aromatic compound include a group having a structure of the following Formula (E1). Note that, L¹ in Formula (E1) is the same as that defined in the aforementioned Formula (V1). * represents a bond position.

*-CH=CH-L¹-CH=CH₂ (E1)

When the group derived from a monovinyl aromatic compound becomes a chain terminal, the group may have a structure represented by the following Formula (E2). In Formula (E2), L² and R^{V1} each have the same meaning as that defined in the aforementioned Formula (V4). * represents a bond position.

*-CH=CH-L²-R^{V1} (E2)

The polymer having the structural unit represented by Formula (V) has a molecular weight, in terms of the number average molecular weight Mn, of preferably 300 or more, more preferably 500 or more, even more preferably 1000 or more, and still more preferably 1500 or more. The upper limit of the number average molecular weight Mn is preferably 130000 or less, more preferably 120000 or less, still more preferably 110000 or less, even preferably 100000 or less, and may be 30000 or less, 10000 or less, or 5000 or less.

The polymer having the structural unit represented by Formula (V) has a molecular weight, in terms of the weight average molecular weight Mw, of preferably 1000 or more, more preferably 2000 or more, and even more preferably 3000 or more. By setting the weight average molecular weight to the aforementioned lower limit or more, the cured product of the resin composition can effectively exhibit the excellent low dielectric properties of the polymer having the structural unit represented by Formula (V), particularly Df and the low dielectric properties after moisture absorption. The upper limit of the weight average molecular weight Mw is preferably 160000 or less, more preferably 150000 or less, even more preferably 140000 or less, still more preferably 130000 or less, and may be 80000 or less or 50000 or less. By setting the weight average molecular weight to the aforementioned upper limit or less, an embedding defect tends not to occur when a prepreg or a resin composite sheet is laminated on a circuit-forming substrate.

A monodispersity (Mw/Mn) represented by the ratio of the weight average molecular weight Mw to the number average molecular weight Mn is preferably 100 or less, more preferably 50 or less, even more preferably 20 or less, and may be 15 or less or 12 or less. The lower limit is practically 1.1 or more, preferably 2.0 or more, more preferably 4 or more, even more preferably 5 or more, still more preferably 7 or more, and yet more preferably 8 or more. The Mw and Mn are measured according to the description in Examples to be described later.

When the resin composition of the present embodiment contains two or more kinds of the polymer having the structural unit represented by Formula (V), it is preferable that Mw, Mn and Mw/Mn of the mixture satisfy the aforementioned ranges.

The equivalent of a vinyl group of the polymer having the structural unit represented by Formula (V) is preferably 200 g/eq. or more, more preferably 230 g/eq. or more, even more preferably 250 g/eq. or more, and may be 300 g/eq. or more or 350 g/eq. or more. The equivalent of the vinyl group is preferably 1200 g/eq. or less, more preferably 1000 g/eq. or less, and further may be 800 g/eq. or less, 600 g/eq. or less, 400 g/eq. or less, or 300 g/eq. or less. By setting the equivalent of the vinyl group to the aforementioned lower limit or more, the storage stability of the resin composition tends to be improved, and the fluidity of the resin composition tends to be improved. Therefore, moldability is improved, voids are less likely to occur during the formation of a prepreg or the like, and a printed wiring board having higher reliability tends to be obtained. On the other hand, by setting the equivalent of the vinyl group to the aforementioned upper limit or less, the moisture absorption heat resistance of the resulting cured product tends to be improved.

In addition, the cured product of the polymer having the structural unit represented by Formula (V) used in the present embodiment is preferably excellent in low dielectric properties. For example, the cured product of the polymer having the structural unit represented by Formula (V) used in the present embodiment has a relative permittivity (Dk), at a frequency of 10 GHz as measured in accordance with a cavity resonator perturbation method, of preferably 2.80 or less, more preferably 2.60 or less, even more preferably 2.50 or less, and still more preferably 2.40 or less. The lower limit of the relative permittivity is practically, for example, 1.80 or more. In addition, the cured product of the polymer having the structural unit represented by Formula (V) used in the present embodiment, has a dielectric loss tangent (Df), at a frequency of 10 GHz as measured in accordance with a cavity resonator perturbation method, of preferably 0.0030 or less, more preferably 0.0020 or less, even more preferably 0.0019 or less, still more preferably 0.0018 or less, and yet more preferably 0.0010 or less. The lower limit of the dielectric loss tangent is practically, for example, 0.0001 or more.

The dielectric loss tangent (Df) is measured according to the methods described in Examples to be described later. The relative permittivity (Dk) is also measured according to the method described in Examples Df.

For the polymer having the structural unit represented by Formula (V) in the present specification, reference can be made to: the compound, the synthesis reaction conditions thereof, and the like described in paragraphs from 0029 to 0058 of WO 2017/115813 A; the compound, the synthesis reaction conditions thereof, and the like described in paragraphs from 0013 to 0058 of JP 2018-039995 A; the compound, the synthesis reaction conditions thereof, and the like described in paragraphs from 0008 to 0043 of JP 2018-168347; the compound, the synthesis reaction conditions thereof, and the like described in paragraphs from 0014 to 0042 of JP 2006-070136 A; the compound, the synthesis reaction conditions thereof, and the like described in paragraphs from 0014 to 0061 of JP 2006-089683 A; and the compound, the synthesis reaction conditions thereof, and the like described in paragraphs from 0008 to 0036 of JP 2008-248001 A, contents of which are incorporated herein.

When the resin composition of the present embodiment contains the polymer having the structural unit represented by Formula (V), the lower limit of the content of the polymer having the structural unit represented by Formula (V) is preferably 5 parts by mass or more, more preferably 10 parts by mass or more, even more preferably 15 parts by mass or more, and may be 20 parts by mass or more or 25 parts by mass or more, based on 100 parts by mass of the resin solid content in the resin composition. By setting the content of the polymer having the structural unit represented by Formula (V) to the aforementioned lower limit or more, low dielectric properties, particularly low dielectric loss tangent, can be effectively achieved. On the other hand, the upper limit of the content of the polymer having the structural unit represented by Formula (V) is preferably 70 parts by mass or less, more preferably 60 parts by mass or less, even more preferably 50 parts by mass or less, still more preferably 40 parts by mass or less, and may be 35 parts by mass or less, based on 100 parts by mass of the resin solid content in the resin composition. By setting the content to the aforementioned upper limit or less, the metal foil peel strength of the resulting cured product can be effectively enhanced.

Only one kind or two or more kinds of the polymer having the structural unit represented by Formula (V) may be contained in the resin composition. When two or more kinds of the polymer are contained, the total amount thereof is preferably within the aforementioned ranges.

### «<Compound containing (meth)allyl group>>>

The resin composition of the present embodiment preferably contains a compound containing a (meth)allyl group, and more preferably contains a compound containing an allyl group.

The compound containing a (meth)allyl group is preferably a compound containing two or more (meth)allyl groups, and more preferably a compound containing two or more allyl groups.

The compound containing a (meth)allyl group preferably contains at least one selected from the group consisting of an allyl isocyanurate compound, an allyl group-substituted nadiimide compound, an allyl compound having a glycoluril structure, and diallyl phthalate, more preferably contains at least one selected from the group consisting of an allyl isocyanurate compound, an allyl group-substituted nadiimide compound, and an allyl compound having a glycoluril structure, and even more preferably contains an allyl group-substituted nadiimide compound.

When the resin composition of the present embodiment contains a compound containing a (meth)allyl group, the molecular weight thereof is preferably 195 or more, more preferably 300 or more, even more preferably 400 or more, and still more preferably 500 or more. By setting the molecular weight to the aforementioned lower limit or more, low dielectric properties and heat resistance tend to be further improved. The molecular weight of the compound containing a (meth)allyl group is preferably 3000 or less, more preferably 2000 or less, even more preferably 1000 or less, and still more preferably 800 or less. By setting the molecular weight to the aforementioned upper limit or less, low thermal expansion tends to be further improved.

When the resin composition of the present embodiment contains a compound containing a (meth)allyl group, the content thereof is preferably 1 part by mass or more, more preferably 3 parts by mass or more, even more preferably 5 parts by mass or more, and may be 10 parts by mass or more, based on 100 parts by mass of the resin solid content in the resin composition. By setting the content of the compound containing a (meth)allyl group to the aforementioned lower limit or more, moldability tends to be excellent and heat resistance tends to be further improved. The upper limit of the content of the compound containing a (meth)allyl group is preferably 40 parts by mass or less, more preferably 30 parts by mass or less, and even more preferably 20 parts by mass or less, based on 100 parts by mass of the resin solid content in the resin composition. By setting the content of the compound containing a (meth)allyl group to the aforementioned upper limit or less, low thermal expansion tends to be further improved.

The resin composition of the present embodiment may contain only one kind or two or more kinds of the compound containing a (meth)allyl group. When two or more kinds thereof are contained, the total amount thereof is preferably within the aforementioned ranges.

### <<<<Allyl isocyanurate compound>>>>

The allyl isocyanurate compound is not particularly limited as long as it is a compound having two or more allyl groups and having an isocyanurate ring (nurate skeleton), but a compound represented by Formula (TA) is preferred.

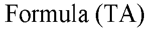

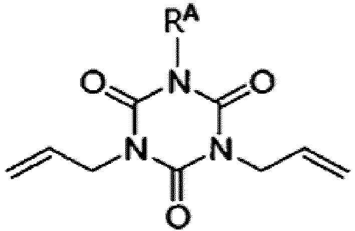

### (In Formula (TA), R^{A} represents a substituent.)

In Formula (TA), R^{A} represents a substituent, and is more preferably a substituent having a formula weight from 15 to 500.

A first example of R^{A} is an alkyl group having from 1 to 22 carbon atoms or an alkenyl group having from 2 to 22 carbon atoms. By using an allyl compound having an alkyl group having from 1 to 22 carbon atoms or an alkenyl group having from 2 to 22 carbon atoms, it is possible to provide a resin composition that can produce a cured product excellent in crosslinking properties and having high toughness. Accordingly, even when a substrate, such as glass cloth, is not included in the resin composition, it is possible to suppress cracking during etching treatment or the like.

The number of carbon atoms of the alkyl group and/or the alkenyl group is preferably 3 or more, more preferably 8 or more, and may be 12 or more, and is preferably 18 or less, from the viewpoint of improving handleability. Accordingly, it is considered that the resin flowability of the resin composition is improved, and the resin composition is more excellent in circuit filling properties and the like when a multilayer circuit board or the like is produced using the resin composition of the present embodiment.

A second example of R^{A} is a group containing an allyl isocyanurate group. When R^{A} contains an allyl isocyanurate group, the compound represented by Formula (TA) is preferably a compound represented by Formula (TA-1).

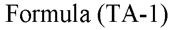

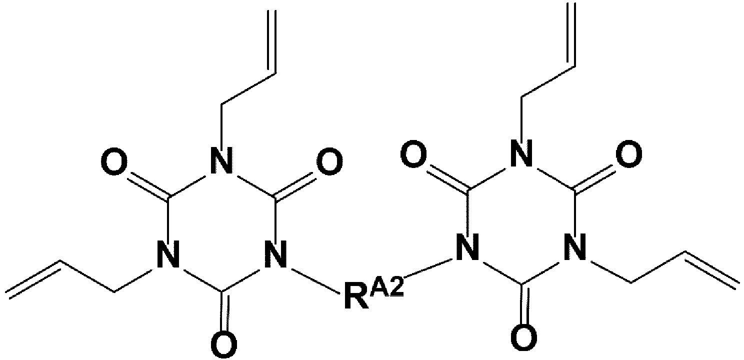

### (In Formula (TA-1), R^{A2} is a divalent linking group.)

In Formula (TA-1), R^{A2} is preferably a divalent linking group having a formula weight from 54 to 250, more preferably a divalent linking group having a formula weight from 54 to 250 and having carbon atoms at both terminals, and even more preferably an aliphatic hydrocarbon group having from 2 to 20 carbon atoms (however, the aliphatic hydrocarbon group may contain an ether group or may have a hydroxyl group). More specifically, R^{A2} is preferably a group represented by any one of the following Formulas (i) to (iii): 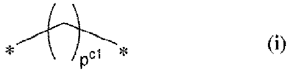 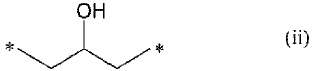 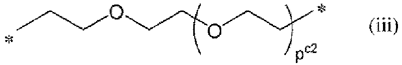 (In Formulas (i) to (iii), p^{c1} represents the number of repeating units of a methylene group, and is an integer from 2 to 18. p^{c2} represents the number of repeating units of an oxyethylene group, and is 0 or 1. * is a binding site.)

The p^{c1} is preferably an integer from 2 to 10, more preferably an integer from 3 to 8, and even more preferably an integer from 3 to 5.

The p^{c2} may be 0 or 1, and is preferably 1.

R^{A2} is preferably the first example.

In the present embodiment, the equivalent of a reactive group (allyl group) of the compound represented by Formula (TA) is desirably 1000 or less. It is considered that, when the equivalent is 1000 or less, a high Tg can be obtained more reliably.

Examples of the alkyl group having from 1 to 22 carbon atoms include linear or branched alkyl groups, and examples thereof include a methyl group, an ethyl group, a propyl group, a butyl group, a pentyl group, a hexyl group, a heptyl group, an octyl group, a nonyl group, a decyl group, a dodecyl group, a tetradecyl group, a hexadecyl group, an octadecyl group, an eicosyl group, and a docosyl group. Examples of the alkenyl group having from 2 to 22 carbon atoms include an allyl group and a decenyl group.

Specific examples of the compound represented by Formula (TA) include triallyl isocyanurate, 5-octyl-1,3-diallyl isocyanurate, 5-dodecyl-1,3-diallyl isocyanurate, 5-tetradecyl-1,3-diallyl isocyanurate, 5-hexadecyl-1,3-diallyl isocyanurate, 5-octadecyl-1,3-diallyl isocyanurate, 5-eicosyl-1,3-diallyl isocyanurate, 5-docosyl-1,3-diallyl isocyanurate, and 5-decenyl-1,3-diallyl isocyanurate. These may be used singly or in combination of two or more kinds thereof, or may be used as a prepolymer.

The method for manufacturing the compound represented by Formula (TA) is not particularly limited, and for example, the compound can be produced by reacting diallyl isocyanurate and an alkyl halide in an aprotic polar solvent, such as N,N'-dimethylformamide, in the presence of a basic substance, such as sodium hydroxide, potassium carbonate, or triethylamine, at a temperature of about from 60°C to 150°C.

As the compound represented by Formula (TA), a commercially available product can also be used. The commercially available compound is not particularly limited, and examples thereof include L-DAIC available from Shikoku Chemicals Corporation and P-DAIC having a phosphorus-based substituent available from Shikoku Chemicals Corporation. Examples of the triallyl isocyanurate include TAIC available from Shinryo Corporation. Examples of the compound represented by Formula (TA-1) include DD-1 available from Shikoku Chemicals Corporation.

The molecular weight of the allyl isocyanurate compound (preferably, the compound represented by Formula (TA)) is preferably 200 or more, more preferably 300 or more, even more preferably 400 or more, and yet more preferably 500 or more. By setting the molecular weight to the aforementioned lower limit or more, low dielectric properties and heat resistance tend to be further improved. The molecular weight of the allyl isocyanurate compound (preferably, the compound represented by Formula (TA)) is preferably 3000 or less, more preferably 2000 or less, even more preferably 1000 or less, and still more preferably 800 or less. By setting the molecular weight to the aforementioned upper limit or less, the low thermal expansion of the resulting cured product tends to be further improved.

When the resin composition of the present embodiment contains an allyl isocyanurate compound, the content thereof is preferably 1 part by mass or more, more preferably 3 parts by mass or more, even more preferably 5 parts by mass or more, and may be 10 parts by mass or more, based on 100 parts by mass of the resin solid content in the resin composition. By setting the content of the allyl isocyanurate compound to the aforementioned lower limit or more, the resin composition tends to be excellent in moldability, and the heat resistance and low water absorption of the resulting cured product tend to be further improved. The upper limit of the content of the allyl isocyanurate compound is preferably 40 parts by mass or less, more preferably 30 parts by mass or less, and even more preferably 20 parts by mass or less, based on 100 parts by mass of the resin solid content in the resin composition. By setting the content of the allyl isocyanurate compound to the aforementioned upper limit or less, the low thermal expansion of the resulting cured product tends to be further improved.

The resin composition of the present embodiment may contain only one kind or two or more kinds of allyl isocyanurate. When two or more kinds thereof are contained, the total amount thereof is preferably within the aforementioned ranges.

### <<<<Allyl group-substituted nadiimide compound>>>>

The allyl group-substituted nadiimide compound is not particularly limited as long as it is a compound having two or more allyl group-substituted nadiimide groups in its molecule. Specific examples thereof include a compound represented by the following Formula (AN). 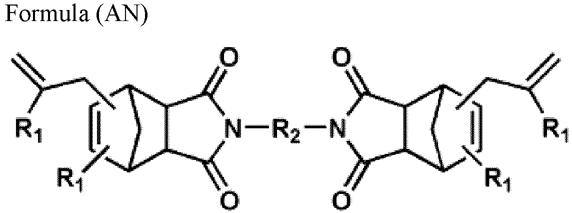 (In Formula (AN), R₁ each independently represents a hydrogen atom or an alkyl group having from 1 to 6 carbon atoms, and R₂ represents an alkylene group having from 1 to 6 carbon atoms, a phenylene group, a biphenylene group, a naphthylene group, or a group represented by Formula (AN-2) or (AN-3). 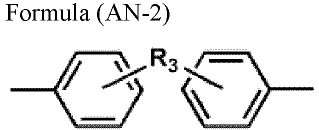 (In Formula (AN-2), R³ represents a methylene group, an isopropylidene group, or a group represented by -C(=O)-, -O-, -S-, or -S(=O)₂-.) 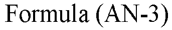 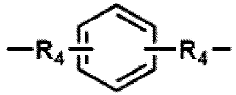 (In Formula (AN-3), R₄ each independently represents an alkylene group having from 1 to 4 carbon atoms or a cycloalkylene group having from 5 to 8 carbon atoms.)

As the compound represented by Formula (AN), a commercially available product can also be used. Examples of the commercially available compound include, but are not particularly limited to, a compound represented by Formula (AN-4) (BANI-M (available from Maruzen Petrochemical Co., Ltd.) and a compound represented by Formula (AN-5) (BANI-X (available from Maruzen Petrochemical Co., Ltd.). These may be used singly or in combination of two or more kinds thereof.

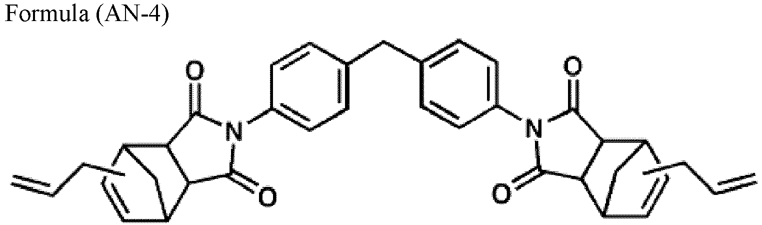

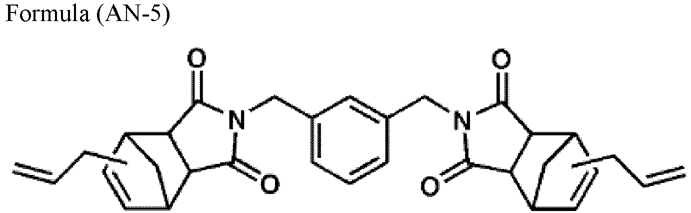

The molecular weight of the allyl group-substituted nadiimide compound (preferably, the compound represented by Formula (AN)) is preferably 400 or more, more preferably 500 or more, and may be 550 or more. By setting the molecular weight of the allyl group-substituted nadiimide compound to the aforementioned lower limit or more, low dielectric properties, low thermal expansion, and heat resistance tend to be further improved. The molecular weight of the allyl group-substituted nadiimide compound (preferably, the compound represented by Formula (AN)) is preferably 1500 or less, more preferably 1000 or less, still more preferably 800 or less, and may be 700 or less, or 600 or less. By setting the molecular weight of the allyl group-substituted nadiimide compound to the aforementioned upper limit or less, moldability and peel strength tend to be further improved.

When the resin composition of the present embodiment contains the allyl group-substituted nadiimide compound (preferably, the compound represented by Formula (AN)), the content thereof is preferably 1 part by mass or more, more preferably 3 parts by mass or more, even more preferably 5 parts by mass or more, and may be 10 parts by mass or more, based on 100 parts by mass of the resin solid content in the resin composition. By setting the content of the allyl group-substituted nadiimide compound to the aforementioned lower limit or more, moldability is excellent, and low dielectric properties, low thermal expansion, and heat resistance tend to be further improved. The upper limit of the content of the allyl group-substituted nadiimide compound (preferably, the compound represented by Formula (AN)) is preferably 40 parts by mass or less, more preferably 30 parts by mass or less, even more preferably 25 parts by mass or less, and may be 20 parts by mass or less, based on 100 parts by mass of the resin solid content in the resin composition. By setting the content of the allyl group-substituted nadiimide compound to the aforementioned upper limit or less, moldability and peel strength tend to be further improved.

The resin composition of the present embodiment may contain only one kind or two or more kinds of the allyl group-substituted nadiimide compound. When two or more kinds thereof are contained, the total amount thereof is preferably within the aforementioned ranges.

### <<<Allyl compound having glycoluril structure>>>

The allyl compound having a glycoluril structure is not particularly limited as long as it is a compound containing two or more glycoluril structures and two or more allyl groups, and a compound represented by Formula (GU-0) is preferred, and a compound represented by Formula (GU) is more preferred. 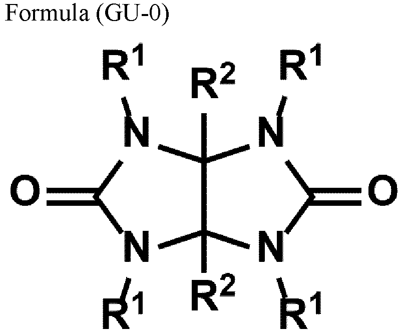 (In Formula (GU-0), R₁ is each independently a hydrogen atom or a substituent, and at least two R¹s are each containing a (meth)allyl group.)

In Formula (GU-0), R₁ is each independently preferably a hydrogen atom, an alkyl group having from 1 to 5 carbon atoms, or an alkenyl group having from 2 to 5 carbon atoms, more preferably an alkenyl group having from 2 to 5 carbon atoms, even more preferably a (meth)allyl group, and still more preferably an allyl group.

In Formula (GU-0), for R¹, three or four of them are preferably groups containing (meth)allyl groups, and more preferably, the four of them are groups containing (meth)allyl groups.

In Formula (GU-0), R² is each independently preferably a hydrogen atom, an alkyl group having from 1 to 4 carbon atoms, or a phenyl group, and more preferably a hydrogen atom or a methyl group. 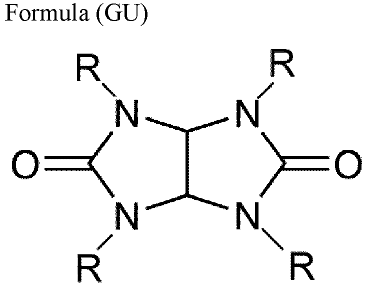 (In Formula (GU), R is each independently a hydrogen atom or a substituent, and at least two R groups are groups each containing an allyl group.)

In Formula (GU), R is each independently preferably a hydrogen atom, an alkyl group having from 1 to 5 carbon atoms, or an alkenyl group having from 2 to 5 carbon atoms, more preferably an alkenyl group having from 2 to 5 carbon atoms, and even more preferably an allyl group.

In Formula (GU), for R, three or four of them are preferably groups containing allyl groups, and more preferably, the four of them are groups containing allyl groups.

Specific examples of the compound represented by Formula (GU) include 1,3,4,6-tetraallylglycoluril (compound wherein R groups are all allyl groups in Formula (GU)).

As the compound represented by Formula (GU), a commercially available product can also be used. The commercially available compound is not particularly limited, and examples thereof include TA-G available from Shikoku Chemicals Corporation.

The molecular weight of the allyl compound having a glycoluril structure (compound represented by Formula (GU-0) is preferred, and compound represented by Formula (GU) is more preferred) is preferably 195 or more, more preferably 220 or more, even more preferably 250 or more, and may be 300 or more or 400 or more. By setting the molecular weight of the allyl compound having a glycoluril structure to the aforementioned lower limit or more, low dielectric properties and heat resistance tend to be further improved. The molecular weight of the allyl compound having a glycoluril structure (compound represented by Formula (GU-0) is preferred, and compound represented by Formula (GU) is more preferred) is also preferably 1500 or less, more preferably 1000 or less, even more preferably 800 or less, and may be 700 or less or 600 or less. By setting the molecular weight of the allyl compound having a glycoluril structure to the aforementioned upper limit or less, low thermal expansion tends to be further improved.

When the resin composition of the present embodiment contains an allyl compound having a glycoluril structure (compound represented by Formula (GU-0) is preferred, and compound represented by Formula (GU) is more preferred), the content thereof is preferably 1 part by mass or more, more preferably 3 parts by mass or more, even more preferably 5 parts by mass or more, and may be 10 parts by mass or more, based on 100 parts by mass of the resin solid content in the resin composition. By setting the content of the allyl compound having a glycoluril structure to the aforementioned lower limit or more, low dielectric properties, heat resistance, and low water absorption tend to be further improved. The upper limit of the content of the allyl compound having a glycoluril structure (compound represented by Formula (GU-0) is preferred, and compound represented by Formula (GU) is more preferred) is preferably 40 parts by mass or less, more preferably 30 parts by mass or less, even more preferably 25 parts by mass or less, and may be 20 parts by mass or less, based on 100 parts by mass of the resin solid content in the resin composition. By setting the content of the allyl compound having a glycoluril structure to the aforementioned upper limit or less, low thermal expansion tends to be further improved.

The resin composition of the present embodiment may contain only one kind or two or more kinds of the allyl compound having a glycoluril structure. When two or more kinds thereof are contained, the total amount thereof is preferably within the aforementioned ranges.

### <<Polyfunctional (meth)acrylate compound>>

The resin composition of the present embodiment may contain a polyfunctional (meth)acrylate compound. However, a compound, which is a polyfunctional (meth)acrylate compound and also corresponds to a polyphenylene ether compound having a carbon-carbon unsaturated double bond, is classified into the polyphenylene ether compound having a carbon-carbon unsaturated double bond.

The polyfunctional (meth)acrylate compound means a compound containing two or more (meth)acryloyloxy groups in a single molecule, and preferably contains three or more (meth)acryloyloxy groups in a single molecule.

The polyfunctional (meth)acrylate compound is preferably a compound having three to five (meth)acryloyloxy groups, more preferably a compound having three or four (meth)acryloyloxy groups, and even more preferably a compound having three (meth)acryloyloxy groups. The polyfunctional (meth)acrylate compound is preferably a compound having a methacryloyloxy group.

Since the polyfunctional (meth)acrylate compound has a large number of (meth)acrylate groups as crosslinking points, the polyfunctional (meth)acrylate compound is firmly cured with the maleimide compound (A) or the like, and a cured product excellent in low dielectric properties (Dk and/or Df) and heat resistance can be obtained. The polyfunctional (meth)acrylate compound is preferably a compound represented by Formula (MA). 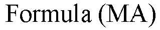 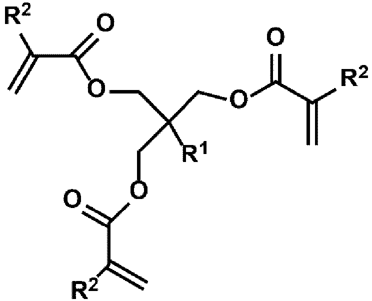 (In Formula (MA), R₁ represents a hydrogen atom or a substituent, and R² each independently represents a hydrogen atom or a methyl group.)

In Formula (MA), R₁ represents a hydrogen atom or a substituent, and is preferably a substituent having a formula weight from 15 to 500, more preferably a substituent having a formula weight from 15 to 300, even more preferably a substituent having a formula weight from 15 to 100, and still more preferably a substituent having a formula weight from 15 to 50.

R₁ is preferably a hydrocarbon group or a (meth)acryloyloxy group, more preferably a hydrocarbon group having 22 or less carbon atoms, and even more preferably an alkyl group having from 1 to 22 carbon atoms or an alkenyl group having from 2 to 22 carbon atoms. By using a compound having an alkyl group having from 1 to 22 carbon atoms or an alkenyl group having from 2 to 22 carbon atoms, it is possible to provide a resin composition that can provide a cured product excellent in crosslinking properties and having high toughness. Accordingly, even when a substrate, such as glass cloth, is not included in the resin composition, it is possible to suppress cracking during etching treatment or the like.

The number of carbon atoms in the alkyl group and/or the alkenyl group is preferably 2 or more, may be 8 or more, may be 12 or more or, and may be 18 or less, from the viewpoint of improving handleability. Accordingly, it is considered that the resin flowability of the resin composition is improved, and the resin composition is more excellent in circuit filling properties and the like when a multilayer circuit board or the like is produced using the resin composition of the present embodiment.

In the present embodiment, the (meth)acrylic group equivalent of the compound represented by Formula (MA) is preferably 1000 g/eq. or less. When the equivalent is 1000 g/eq. or less, a high Tg tends to be obtained more reliably. The lower limit of the (meth)acrylic group equivalent is, for example, 99 g/eq. or more.

The alkyl group having from 1 to 22 carbon atoms is preferably a linear alkyl group having from 1 to 22 carbon atoms, or a branched alkyl group having from 3 to 22 carbon atoms, and examples thereof include a methyl group, an ethyl group, a propyl group, a butyl group, a pentyl group, a hexyl group, a heptyl group, an octyl group, a nonyl group, a decyl group, a dodecyl group, a tetradecyl group, a hexadecyl group, an octadecyl group, an eicosyl group, and a docosyl group. The alkenyl group having from 2 to 22 carbon atoms is preferably an alkenyl group having from 2 to 15 carbon atoms, and examples thereof include an allyl group and a decenyl group.

Specific examples of the compound represented by Formula (MA) include trimethylolpropane trimethacrylate, trimethylolpropane triacrylate, and pentaerythritol tetra(meth)acrylate. These may be used singly or in combination of two or more kinds thereof, or may be used as a prepolymer.

As the compound represented by Formula (MA), a commercially available product can also be used. The commercially available product is not particularly limited, and examples of trimethylolpropane trimethacrylate include "NK Ester TMPT" available from Shin-Nakamura Chemical Co., Ltd.

The molecular weight of the polyfunctional (meth)acrylate compound is preferably 200 or more, more preferably 300 or more, and may be 330 or more, 400 or more, or 500 or more. By setting the molecular weight to the aforementioned lower limit or more, the low dielectric properties (Dk and/or Df) and heat resistance of the resulting cured product tend to be further improved. In addition, the molecular weight of the polyfunctional (meth)acrylate compound (preferably, compound represented by Formula (MA)) is preferably 3000 or less, more preferably 2000 or less, even more preferably 1000 or less, and still more preferably 800 or less. By setting the molecular weight to the aforementioned upper limit or less, the low thermal expansion of the resulting cured product tends to be further improved.

For the polyfunctional (meth)acrylate compound, reference can be made to: in addition to the above, the resin having a (meth)acrylic group described in WO 2022/210095 A (e.g., compounds described in Synthesis Examples 5 and 21 of the same publication); the resin having a (meth)acrylic group described in JP 6962507 B2 (e.g., compounds described Examples from 1 to 9); and the compound described in paragraph 0049 of JP 2019-194312 A, the contents of which are incorporated herein.

When the resin composition of the present embodiment contains the polyfunctional (meth)acrylate compound, the content of the compound is preferably 1 part by mass or more, more preferably 3 parts by mass or more, even more preferably 5 parts by mass or more, and may be 10 parts by mass or more, based on 100 parts by mass of the resin solid content in the resin composition. By setting the content of the polyfunctional (meth)acrylate compound to the aforementioned lower limit or more, the resin composition tends to be excellent in moldability, and the heat resistance and low thermal expansion of the resulting cured product tend to be further improved. The upper limit of the content of the polyfunctional (meth)acrylate compound is preferably 40 parts by mass or less, more preferably 30 parts by mass or less, and may be 20 parts by mass or less, based on 100 parts by mass of the resin solid content in the resin composition. By setting the content of the polyfunctional (meth)acrylate compound to the aforementioned upper limit or less, the heat resistance and low dielectric properties (Dk and/or Df) of the resulting cured product tend to be further improved.

The resin composition of the present embodiment may contain only one kind or two or more kinds of the polyfunctional (meth)acrylate compound. When two or more kinds thereof are contained, the total amount thereof is preferably within the aforementioned ranges.

### <<Epoxy compound>>

The resin composition of the present embodiment may contain an epoxy compound.

The epoxy compound is not particularly limited as long as it is a compound or resin having one or more (preferably from 2 to 12, more preferably from 2 to 6, even more preferably from 2 to 4, still more preferably 2 or 3, and yet more preferably 2) epoxy groups in a single molecule, and compounds usually used in the field of printed wiring boards can be widely used.

Examples of the epoxy compound include: compounds obtained by epoxidizing double bonds of a bisphenol A-type epoxy resin, a bisphenol E-type epoxy resin, a bisphenol F-type epoxy resin, a bisphenol S-type epoxy resin, a phenol novolac-type epoxy resin, a bisphenol A novolac-type epoxy resin, a glycidyl ester-type epoxy resin, an aralkyl novolac-type epoxy resin, a biphenyl aralkyl-type epoxy resin, a naphthylene ether-type epoxy resin, a cresol novolac-type epoxy resin, a polyfunctional phenol-type epoxy resin, a naphthalene-type epoxy resin, an anthracene-type epoxy resin, a naphthalene skeleton modified novolac-type epoxy resin, a phenol aralkyl-type epoxy resin, a naphthol aralkyl-type epoxy resin, a dicyclopentadiene-type epoxy resin, a biphenyl-type epoxy resin, an alicyclic epoxy resin, a polyol-type epoxy resin, a phosphorus-containing epoxy resin, glycidylamine, a glycidyl ester, and butadiene; and compounds obtained by reacting hydroxyl group-containing silicone resins with epichlorohydrin. Among these, from the viewpoint of further improving flame retardancy and heat resistance, a biphenyl aralkyl-type epoxy resin, a naphthylene ether-type epoxy resin, a polyfunctional phenol-type epoxy resin, a naphthalene-type epoxy resin are preferred, and a biphenyl aralkyl-type epoxy resin is more preferred.

The resin composition of the present embodiment preferably contains the epoxy compound in a range that does not impair the effects of the present invention. When the resin composition of the present embodiment contains the epoxy compound, the content of the epoxy compound is preferably 0.1 parts by mass or more, more preferably 1 part by mass or more, and even more preferably 2 parts by mass or more, based on 100 parts by mass of the resin solid content in the resin composition, from the viewpoint of moldability and adhesion. When the content of the epoxy compound is 0.1 parts by mass or more, metal foil peel strength and toughness tend to be improved. When the resin composition of the present embodiment contains the epoxy compound, the upper limit of the content of the epoxy compound is preferably 50 parts by mass or less, more preferably 30 parts by mass or less, even more preferably 20 parts by mass or less, still more preferably 10 parts by mass or less, yet more preferably 8 parts by mass or less, and far more preferably 5 parts by mass or less, based on 100 parts by mass of the resin solid content in the resin composition. When the content of the epoxy resin is 50 parts by mass or less, electrical properties tend to be improved.

The resin composition of the present embodiment may contain only one kind or two or more kinds of the epoxy compound. When two or more kinds thereof are contained, the total amount thereof is preferably within the aforementioned ranges.

The resin composition of the present embodiment may also be formed to be substantially free of the epoxy compound. The phrase "substantially free" means that the content of the epoxy compound is less than 0.1 parts by mass, based on 100 parts by mass of the resin solid content in the resin composition.

### <<Phenolic compound>>

The resin composition of the present embodiment may contain a phenolic compound.

The phenolic compound is not particularly limited as long as it is a phenolic compound having one or more (preferably from 2 to 12, more preferably from 2 to 6, even more preferably from 2 to 4, still more preferably 2 or 3, and yet more preferably 2) phenolic hydroxy groups in a single molecule, and compounds usually used in the field of printed wiring boards can be widely used.

Examples of the phenolic compound include a bisphenol A-type phenolic resin, a bisphenol E-type phenolic resin, a bisphenol F-type phenolic resin, a bisphenol S-type phenolic resin, a phenol novolac resin, a bisphenol A novolac-type phenolic resin, a glycidyl ester-type phenolic resin, an aralkyl novolac phenolic resin, a biphenyl aralkyl-type phenolic resin, a cresol novolac-type phenolic resin, a polyfunctional phenolic resins, a naphthol resin, a naphthol novolac resin, a polyfunctional naphthol resin, an anthracene-type phenolic resin, a naphthalene skeleton-modified novolac-type phenolic resin, a phenol aralkyl-type phenolic resin, a naphthol aralkyl-type phenolic resin, a dicyclopentadiene-type phenolic resin, a biphenyl-type phenolic resin, an alicyclic phenolic resin, a polyol-type phenolic resin, a phosphorus-containing phenolic resin, and a hydroxyl group-containing silicone resin. Among these, from the viewpoint of further improving flame resistance, at least one phenolic resin selected from the group consisting of a biphenylaralkyl-type phenolic resin, a naphtholaralkyl-type phenolic resin, a phosphorus-containing phenolic resin, and a hydroxyl group-containing silicone resin is preferred.

For the phenolic compound, reference can also be made to the description in paragraphs from 0012 to 0025 of WO 2023/176765 A, the contents of which are incorporated herein.

The resin composition of the present embodiment preferably contains the phenolic compound in a range that does not impair the effects of the present invention. When the resin composition of the present embodiment contains the phenolic compound, the content thereof is preferably 0.1 parts by mass or more, and preferably 50 parts by mass or less, based on 100 parts by mass of the resin solid content in the resin composition.

The resin composition of the present embodiment may contain only one kind or two or more kinds of the phenolic compound. When two or more kinds thereof are contained, the total amount thereof is preferably within the aforementioned ranges.

**In** addition, the resin composition of the present embodiment can also be formed to be substantially free of the phenolic compound. The phrase "substantially free" means that the content of the phenolic compound is less than 0.1 parts by mass, based on 100 parts by mass of the resin solid content in the resin composition.

### <<Oxetane resin>>

The resin composition of the present embodiment may contain an oxetane resin.

The oxetane resin is not particularly limited as long as it is a compound having one or more (preferably from 2 to 12, more preferably from 2 to 6, even more preferably from 2 to 4, still more preferably 2 or 3, and yet more preferably 2) oxetanyl groups, and compounds usually used in the field of printed wiring boards can be widely used.

Examples of the oxetane resin include oxetane, alkyl oxetane (e.g., 2-methyloxetane, 2,2-dimethyloxetane, 3-methyloxetane, 3,3-dimethyloxetane), 3-methyl-3-methoxymethyl oxetane, 3,3-di(trifluoromethyl)perfluoxetane, 2-chloromethyl oxetane, 3,3-bis(chloromethyl)oxetane, biphenyl-type oxetane, OXT-101 (available from Toagosei Co., Ltd.), and OXT-121 (available from Toagosei Co., Ltd.).

The resin composition of the present embodiment preferably contains the oxetane resin in a range that does not impair the effects of the present invention. When the resin composition of the present embodiment contains the oxetane resin, the content of the oxetane resin is preferably 0.1 parts by mass or more, more preferably 1 part by mass or more, and even more preferably 2 parts by mass or more, based on 100 parts by mass of the resin solid content in the resin composition. When the content of the oxetane resin is 0.1 parts by mass or more, metal foil peel strength and toughness tend to be improved. When the resin composition of the present embodiment contains the oxetane resin, the upper limit of the content of the oxetane resin is preferably 50 parts by mass or less, more preferably 30 parts by mass or less, even more preferably 20 parts by mass or less, still more preferably 10 parts by mass or less, and yet more preferably 8 parts by mass or less, based on 100 parts by mass of the resin solid content in the resin composition. When the content of the oxetane resin is 50 parts by mass or less, electrical properties tend to be improved.

The resin composition of the present embodiment may contain only one kind or two or more kinds of the oxetane resin. When two or more kinds thereof are contained, the total amount thereof is preferably within the aforementioned ranges.

**In** addition, the resin composition of the present embodiment may also be formed to be substantially free of the oxetane resin. The phrase "substantially free" means that the content of the oxetane resin is less than 0.1 parts by mass, based on 100 parts by mass of the resin solid content in the resin composition.

### <<Benzoxazine compound>>

The resin composition of the present embodiment may contain a benzoxazine compound.

The benzoxazine compound is not particularly limited as long as it is a compound having two or more (preferably from 2 to 12, more preferably from 2 to 6, even more preferably from 2 to 4, still more preferably 2 or 3, and yet more preferably 2) dihydrobenzoxazine rings in a single molecule, and compounds usually used in the field of printed wiring boards can be widely used.

Examples of the benzoxazine compound include a bisphenol A-type benzoxazine BA-BXZ (available from Konishi Chemical Ind. Co., Ltd.), a bisphenol F-type benzoxazine BF-BXZ (available from Konishi Chemical Industry Co., Ltd.), and a bisphenol S-type benzoxazine BS-BXZ (available from Konishi Chemical Industry Co., Ltd.).

The resin composition of the present embodiment preferably contains the benzoxazine compound in a range that does not impair the effects of the present invention. When the resin composition of the present embodiment contains the benzoxazine compound, the content thereof is preferably 0.1 parts by mass or more, and preferably 50 parts by mass or less, based on 100 parts by mass of the resin solid content in the resin composition.

The resin composition of the present embodiment may contain only one kind or two or more kinds of the benzoxazine compound. When two or more kinds thereof are contained, the total amount thereof is preferably within the aforementioned ranges.

The resin composition of the present embodiment may also be formed to be substantially free of the benzoxazine compound. The phrase "substantially free" means that the content of the benzoxazine compound is less than 0.1 parts by mass, based on 100 parts by mass of the resin solid content in the resin composition.

### <<Thermoplastic elastomer>>

The resin composition of the present embodiment may contain a thermoplastic elastomer.

The thermoplastic elastomer in the present embodiment is not particularly limited, and examples thereof include at least one selected from the group consisting of polyisoprene, polybutadiene, styrene butadiene, butyl rubber, ethylene propylene rubber, styrene butadiene ethylene, styrene butadiene styrene, styrene isoprene styrene, styrene ethylene butylene styrene, styrene propylene styrene, styrene ethylene propylene styrene, fluororubber, silicone rubber, hydrogenated compounds thereof, alkyl compounds thereof, and copolymers thereof.

The thermoplastic elastomer used in the present embodiment has a number average molecular weight of preferably 50000 or more. By setting the number average molecular weight to 50000 or more, the dielectric properties (low dielectric loss tangent) of the resulting cured product tends to be more excellent. The number average molecular weight is preferably 60000 or more, more preferably 70000 or more, and even more preferably 80000 or more. The upper limit of the number average molecular weight of the thermoplastic elastomer is preferably 400000 or less, more preferably 350000 or less, and even more preferably 300000 or less. By setting the number average molecular weight to the aforementioned upper limit or less, the solubility of the thermoplastic elastomer component in the resin composition tends to be improved.

When the resin composition of the present embodiment contains two or more kinds of the thermoplastic elastomer, the number average molecular weight of the mixture thereof preferably satisfies the aforementioned ranges.

**In** the present embodiment, the thermoplastic elastomer is preferably a thermoplastic elastomer containing a styrene monomer unit and a conjugated diene monomer unit (hereinafter referred to as "thermoplastic elastomer (E)"). By using such a thermoplastic elastomer (E), the dielectric properties (low dielectric loss tangent) of the resulting cured product are more excellent.

The thermoplastic elastomer (E) contains a styrene monomer unit. When the thermoplastic elastomer (E) contains a styrene monomer unit, the solubility of the thermoplastic elastomer (E) in the resin composition is improved. Examples of the styrene monomer include styrene, α-methylstyrene, p-methylstyrene, divinylbenzene (vinylstyrene), N,N-dimethyl-p-aminoethylstyrene, and N,N-diethyl-p-aminoethylstyrene. Among these, styrene, α-methylstyrene, and p-methylstyrene are preferred from the viewpoint of availability and productivity. Among these, styrene is particularly preferred.

The content of the styrene monomer unit in the thermoplastic elastomer (E) is preferably in a range from 10 to 50 mass%, more preferably in a range from 13 to 45 mass%, and even more preferably in a range from 15 to 40 mass% of all the monomer units. When the content of the styrene monomer unit is 50 mass% or less, adhesion and tackiness to a substrate or the like are further improved. When the content is 10 mass% or more, an increase in adhesiveness can be suppressed, an adhesive residue or a stop mark is less likely to occur, and easy release between adhesive faces tend to be improved, which is preferable.

The thermoplastic elastomer (E) may contain only one of, or two or more of the styrene monomer units. When two or more kinds thereof are contained, the total amount thereof is preferably within the aforementioned ranges.

As for a method for measuring the content of the styrene monomer unit in the thermoplastic elastomer (E) of the present embodiment, reference can be made to WO 2017/126469, the contents of which are incorporated herein by reference. The same shall apply to the conjugated diene monomer unit to be described later.

The thermoplastic elastomer (E) contains a conjugated diene monomer unit. When the thermoplastic elastomer (E) contains a conjugated diene monomer unit, the solubility of the thermoplastic elastomer (E) in the resin composition is improved. The conjugated diene monomer is not particularly limited as long as it is diolefin having a pair of conjugated double bonds. Examples of the conjugated diene monomer include 1,3-butadiene, 2-methyl-1,3-butadiene (isoprene), 2,3-dimethyl-1,3-butadiene, 1,3-pentadiene, 2-methyl-1,3-pentadiene, 1,3-hexadiene, and farnesene. Among these, 1,3-butadiene and isoprene are preferred, and 1,3-butadiene is more preferred.

The thermoplastic elastomer (E) may contain only one of, or two or more of the conjugated diene monomer units.

**In** the thermoplastic elastomer (E), a mass ratio of the styrene monomer unit to the conjugated diene monomer unit (styrene monomer unit/conjugated diene monomer unit) is preferably in a range from 5/95 to 80/20, more preferably in a range from 7/93 to 77/23, and even more preferably in a range from 10/90 to 70/30. When the mass ratio of the styrene polymer unit to the conjugated diene monomer unit is in the range from 5/95 to 80/20, an increase in adhesiveness can be suppressed, adhesive strength can be maintained high, and easy release between adhesive faces is improved.

**In** the thermoplastic elastomer (E), all of the conjugated diene bonds of the thermoplastic elastomer may be hydrogenated, some of the conjugated diene bonds may be hydrogenated, or the conjugated diene bonds may not be hydrogenated.

The thermoplastic elastomer (E) may or may not contain an additional monomer unit in addition to the styrene monomer unit and the conjugated diene monomer unit. Examples of the additional monomer unit include an aromatic vinyl compound unit other than the styrene monomer unit.

**In** the thermoplastic elastomer (E), a total of the styrene monomer unit and the conjugated diene monomer unit is preferably 90 mass% or more, more preferably 95 mass% or more, even more preferably 97 mass% or more, and still more preferably 99 mass% or more of all the monomer units.

As described above, the thermoplastic elastomer (E) may contain only one kind of each of the styrene monomer unit and the conjugated diene monomer unit, or may contain two or more kinds of each of the styrene monomer units and the conjugated diene monomer units. When two or more kinds thereof are contained, the total amount thereof is preferably within the aforementioned ranges.

The thermoplastic elastomer (E) used in the present embodiment may be a block polymer or a random polymer. In addition, the elastomer may be a hydrogenated elastomer in which the conjugated diene monomer unit is hydrogenated, an unhydrogenated elastomer in which the conjugated diene monomer unit is not hydrogenated, or a partially hydrogenated elastomer in which the conjugated diene monomer unit is partially hydrogenated. The thermoplastic elastomer (e) is preferably an unhydrogenated elastomer or a partially hydrogenated elastomer.

In one embodiment of the present embodiments, the thermoplastic elastomer (E) is a hydrogenated elastomer. Here, the hydrogenated elastomer means, for example, one in which the double bonds based on the conjugated diene monomer units in the thermoplastic elastomer are hydrogenated, and it is intended to include those having a hydrogenation percentage (degree of hydrogenation) of 80% or more in addition to those having a hydrogenation percentage of 100%. The hydrogenation percentage in the hydrogenated elastomer is preferably 85% or more, more preferably 90% or more, and even more preferably 95% or more. In the present embodiment, the hydrogenation percentage is calculated from the measurement results of ¹H-NMR spectrum measurement.

In one embodiment of the present embodiment, the thermoplastic elastomer (E) is an unhydrogenated elastomer. Here, the unhydrogenated elastomer refers to an elastomer having a ratio of hydrogenated double bonds to the double bonds based on the conjugated diene monomer units in the elastomer, that is, a hydrogenation percentage (degree of hydrogenation) of 20% or less. The hydrogenation percentage is preferably 15% or less, more preferably 10% or less, and even more preferably 5% or less.

On the other hand, the partially hydrogenated elastomer means one in which some of the double bonds based on the conjugated diene monomer units in thermoplastic elastomer are hydrogenated, and usually refers to a thermoplastic elastomer having a hydrogenation percentage (degree of hydrogenation) of less than 80% and more than 20%.

Examples of commercially available products of the thermoplastic elastomer (E) used in the present embodiment include SEPTON (trade name) 2104, V9461, and S8104 available from Kuraray Co., Ltd., S.O.E. (trade name) S1606, S1613, S1609, and S1605 available from Asahi Kasei Corporation, Tuftec (trade name) H1041, H1043, P2000, and MP10 available from Asahi Kasei Corporation, and DYNARON (trade name) 9901P and TR2250 available from JSR Corporation.

The elastomer used in the present embodiment may also be a liquid diene. The liquid diene means a liquid elastomer containing a conjugated diene monomer unit. Examples of the conjugated diene monomer include 1,3-butadiene, 2-methyl-1,3-butadiene (isoprene), 2,3-dimethyl-1,3-butadiene, 1,3-pentadiene, 2-methyl-1,3-pentadiene, 1,3-hexadiene, and farnesene. Among these, 1,3-butadiene and isoprene are preferred, and 1,3-butadiene is more preferred.

Examples of the liquid diene used in the present embodiment include liquid polybutadiene, liquid polyisoprene, a modified product of liquid polybutadiene, a modified product of liquid polyisoprene, a liquid acrylonitrile-butadiene copolymer, and a liquid styrenebutadiene copolymer.

The number average molecular weight of the liquid diene is not particularly limited as long as it is liquid at 20°C, and is preferably 500 or more or 10000 or less.

When the resin composition of the present embodiment contains a thermoplastic elastomer (preferably, the thermoplastic elastomer (E)), the content thereof is preferably 1 part by mass or more, more preferably 5 parts by mass or more, even more preferably 10 parts by mass or more, and still more preferably 12 parts by mass or more, based on 100 parts by mass of the resin solid content in the resin composition. By setting the content to the aforementioned lower limit or more, dielectric properties (low dielectric loss tangent) tend to be further improved. The upper limit of the content of the thermoplastic elastomer is preferably 45 parts by mass or less, more preferably 40 parts by mass or less, even more preferably 35 parts by mass or less, still more preferably 32 parts by mass or less, and yet more preferably 28 parts by mass or less, based on 100 parts by mass of the resin solid content in the resin composition. By setting the content to the aforementioned upper limit or less, heat resistance tends to be further improved.

The resin composition of the present embodiment may contain only one kind or two or more kinds of the thermoplastic elastomer. When two or more kinds thereof are contained, the total amount thereof is preferably within the aforementioned ranges.

For details of the arylcyclobutene resin, the polyamide resin, the polyimide resin, the perfluorovinyl ether resin, and the petroleum resin, reference can be made to the descriptions in paragraphs from 0198 to 0200 and from 0232 to 0242 of WO 2023/176765 A, the contents of which are incorporated herein.

### <Filler (D)>

The resin composition of the present embodiment preferably contains a filler (D). When the filler (D) is contained, the physical properties, such as low dielectric properties (low dielectric constant, low dielectric loss tangent, etc.), flame resistance, and low thermal expansion, of the resin composition and the cured product thereof can be further improved.

In addition, the filler (D) used in the present embodiment is preferably excellent in low dielectric properties. For example, the filler (D) used in the present embodiment has a relative dielectric constant (Dk) at a frequency of 10 GHz as measured in accordance with a cavity resonator perturbation method of preferably 8.0 or less, more preferably 6.0 or less, and still more preferably 4.0 or less. The lower limit of the relative permittivity is practically, for example, 2.0 or more. The filler (D) used in the present embodiment has a dielectric loss tangent (Df) at a frequency of 10 GHz as measured in accordance with a cavity resonator perturbation method of preferably 0.05 or less, and more preferably 0.01 or less. The lower limit of the dielectric loss tangent is practically, for example, 0.0001 or more.

The kind of the filler (D) used in the present embodiment is not particularly limited, and fillers commonly used in this industry can be suitably used. Specific examples of the filler include inorganic fillers such as silicas (e.g., natural silica, fused silica, synthetic silica, amorphous silica, aerosil, and hollow silica), metal oxides (e.g., alumina, white carbon, titanium white, titanium oxide, zinc oxide, magnesium oxide, and zirconium oxide), composite oxides (e.g., zinc borate, zinc stannate, forsterite, barium titanate, strontium titanate, and calcium titanate). nitrides (e.g., boron nitride, aggregated boron nitride, silicon nitride, and aluminum nitride), metal hydroxides (including hydrates) (e.g., aluminum hydroxide, aluminum hydroxide heat treated product obtained by subjecting aluminum hydroxide to heat treatment to reduce a part of crystal water, boehmite, and magnesium hydroxide), molybdenum compounds (e.g., molybdenum oxide and zinc molybdate), barium sulfate, clay, kaolin, talc, fired clay, fired kaolin, fired talc, mica, E-glass, A-glass, NE-glass, C-glass, L-glass, D-glass, S-glass, M-glass G20, short glass fibers (including fine powders of glass such as E-glass, T-glass, D-glass, S-glass, and Q-glass), hollow glass, and spherical glass, as well as organic fillers such as rubber powder (e.g., styrene-type, butadiene-type, and acrylic-type), core-shell type rubber powder, silicone resin powder, silicone rubber power, and silicone composite powder.

In the present embodiment, the filler (D) preferably contains an inorganic filler, and more preferably contains one or more selected from the group consisting of silica, aluminum hydroxide, aluminum nitride, boron nitride, forsterite, titanium oxide, barium titanate, strontium titanate, and calcium titanate. From the viewpoint of low dielectric properties, the filler (D) even more preferably contains one or more selected from the group consisting of silica and aluminum hydroxide, and still more preferably includes silica. When these fillers are used, the properties, such as heat resistance, low dielectric properties, thermal expansion, dimensional stability, and flame retardancy, of the cured product of the resin composition are further improved.

The content of the filler (D) in the resin composition of the present embodiment can be appropriately determined depending on the desired properties, and is not particularly limited. The content thereof is preferably 1 part by mass or more, more preferably 5 parts by mass or more, even more preferably 10 parts by mass or more, still more preferably 30 parts by mass or more, yet more preferably 50 parts by mass or more, far more preferably 80 parts by mass or more, and further depending on application and the like, may be 100 parts by mass or more or 120 parts by mass or more, based on 100 parts by mass of the resin solid content in the resin composition. By setting the content of the filler to the aforementioned lower limit value or more, low thermal expansion and low dielectric loss tangent properties tend to be further improved. The upper limit of the content of the filler is preferably 1600 parts by mass or less, more preferably 1200 parts by mass or less, even more preferably 800 parts by mass or less, still more preferably 500 parts by mass or less, yet more preferably 300 parts by mass or less, and far more preferably 200 parts by mass or less, based on 100 parts by mass of the resin solid content. By setting the content to the aforementioned upper limit or less, moldability tends to further improve.

In the resin composition of the present embodiment, an aspect, in which the content of the filler (D) is from 1 to 95 mass% of the components excluding the solvent, is taken as an example of a preferred embodiment, and the content is preferably from 30 mass% to 80 mass%.

The resin composition of the present embodiment may contain only one kind or two or more kinds of the filler (D). When two or more kinds thereof are contained, the total amount thereof is preferably within the aforementioned ranges.

When the filler (D), particularly an inorganic filler, is used in the resin composition of the present embodiment, a silane coupling agent may be further contained. When the silane coupling agent is contained, the dispersion of the filler (D) and the adhesive strength between the resin component and the filler (D) and the substrate to be described later tend to be further improved.

The silane coupling agent is not particularly limited, and examples thereof include silane coupling agents generally used in surface treatment of inorganic substances, and examples thereof include an aminosilane-based compound (e.g., γ-aminopropyltriethoxysilane, N-β-(aminoethyl)-γ-aminopropyltrimethoxysilane, or the like), an epoxysilane-based compound (e.g., γ-glycidoxypropyltrimethoxysilane or the like), a vinylsilane-based compound (e.g., vinyltrimethoxysilane or the like), a styrylsilane-based compound (e.g., p-styryltrimethoxysilane or the like), an acrylsilane-based compound (e.g., γ-acryloxypropyltrimethoxysilane or the like), a cathionicsilane-based compound (e.g., N-β-(N-vinylbenzylaminoethyl)-γ-aminopropyltrimethoxysilane hydrochloride or the like), and a phenylsilane-based compound. The silane coupling agent is used singly or in combination of two or more kinds thereof.

In particular, when at least one selected from the group consisting of a vinylsilane-based compound, an acrylsilane-based compound, and a styrylsilane-based compound is used as the silane coupling agent and when a compound containing a vinylaryl group (in particular, a polymer having a structural unit represented by Formula (V)) is used, low dielectric properties tend to be further improved. Furthermore, when at least one selected from the group consisting of a vinyl silane-based compound, an acrylsilane-based compound, and a styrylsilane-based compound is used and when a compound containing a vinylaryl group (in particular, a polymer having a structural unit represented by Formula (V)) and a thermoplastic elastomer are used in combination, low dielectric properties tend to be even further improved.

The content of the silane coupling agent is not particularly limited, but may be from 0.1 to 5.0 parts by mass, based on 100 parts by mass of the resin solid content in the resin composition.

### <Monomer or oligomer having ethylenically unsaturated group>

In the resin composition of the present embodiment, a monomer or oligomer having an ethylenically unsaturated group may be used in combination in order to enhance thermosetting properties and curability with active energy rays (e.g., UV-induced photopolymerization, etc.). The oligomer or monomer having an ethylenically unsaturated group used in the present embodiment is not particularly limited as long as it is an oligomer or monomer having one or more ethylenically unsaturated groups in a single molecule, and examples thereof include a monomer or oligomer having a vinyl group, an allyl group, a (meth)acryloyl group, and the like, and a monomer or oligomer having a vinyl group is preferred.

**In** the present specification, a compound, corresponding to the monomer or oligomer having an ethylenically unsaturated group and also corresponding to a polyphenylene ether compound containing two or more carbon-carbon unsaturated double bonds, is defined as a polyphenylene ether compound containing two or more carbon-carbon unsaturated double bonds.

More specifically, examples of the monomer having an ethylenically unsaturated group include a compound (F1) (compound (F1)) having a molecular weight of less than 1000 and containing one organic group containing an ethylenically unsaturated bond in its molecule.

It is intended that the ethylenically unsaturated bond forming the organic group containing the ethylenically unsaturated bond does not include one that is contained as a part of an aromatic ring. On the other hand, it is intended to include an ethylenically unsaturated bond contained as a part of a non-aromatic ring. Examples of the ethylenically unsaturated bond contained as a part of a non-aromatic ring include a cyclohexenyl group in the molecule. It is also intended to include an ethylenically unsaturated bond contained in a portion other than a terminal of a linear or branched organic group, that is, in a linear or branched chain.

The organic group containing the ethylenically unsaturated bond is preferably one selected from the group consisting of a vinyl group, an isopropenyl group, an allyl group, an acryloyl group, a methacryl group, and a vinylene group, more preferably one selected from the group consisting of a vinyl group, an isopropenyl group, an allyl group, an acryloyl group, and a methacryl group, and even more preferably a vinyl group.

**In** the present specification, a compound, corresponding to a monomer or oligomer having an ethylenically unsaturated group and also corresponding to a silane coupling agent, is defined as a silane coupling agent.

The compound (F1) used in the present embodiment is preferably composed of only atoms selected from a carbon atom, a hydrogen atom, an oxygen atom, and a silicon atom, and more preferably composed of only atoms selected from a carbon atom, a hydrogen atom, and an oxygen atom.

The compound (F1) used in the present embodiment may or may not have a polar group. The compound (F1) used in the present embodiment preferably has no polar group. Examples of the polar group include an amino group, a carboxyl group, a hydroxy group, and a nitro group.

In the present embodiment, the molecular weight of the compound (F1) is preferably 70 or more, more preferably 80 or more, and even more preferably 90 or more. By setting the molecular weight to the aforementioned lower limit or more, volatilization of the compound (F1) from the resin composition of the present embodiment, a cured product thereof, and the like tends to be suppressed. The upper limit of the molecular weight of the compound (F1) is preferably 500 or less, more preferably 400 or less, even more preferably 300 or less, still more preferably 200 or less, and may be 150 or less. By setting the molecular weight to the aforementioned upper limit or less, the effect of enhancing the reactivity with other thermoplastic resin components tends to be further improved.

When the resin composition of the present embodiment contains two or more kinds of the compound (F1), the average molecular weight of the compound (F1) is preferably included within the aforementioned ranges, and the molecular weight of each compound is more preferably included within the aforementioned preferred ranges.

In the present embodiment, the boiling point of the compound (F1) is preferably 110°C or higher, more preferably 115°C or higher, and even more preferably 120°C or higher. By setting the boiling point to the aforementioned lower limit or higher, volatilization of the compound (F1), when the resin composition is thermally cured, is suppressed, and the thermosetting resin (C) and the compound (F1) can react. The boiling point of the compound (F1) is preferably 300°C or lower, more preferably 250°C or lower, and even more preferably 200°C or lower. By setting the boiling point to the aforementioned upper limit or lower, it is possible to make the compound (F1) less likely to remain as a residual solvent in the cured product.

When the resin composition of the present embodiment contains two or more compounds (F1), the average value of the boiling points may be within the aforementioned ranges, but the boiling point of each of the compounds is preferably included within the aforementioned preferred ranges.

Examples of the compound (F1) include a (meth)acrylic acid ester compound, an aromatic vinyl compound (preferably, a styrene-based compound), a saturated fatty acid vinyl compound, a vinyl cyanide compound, an ethylenically unsaturated carboxylic acid, an ethylenically unsaturated carboxylic acid anhydride, an ethylenically unsaturated dicarboxylic acid monoalkyl ester, and an ethylenically unsaturated carboxylic acid amide, and at least one selected from the group consisting of a (meth)acrylic acid ester compound, an aromatic vinyl compound, and a saturated fatty acid vinyl compound is preferred, and an aromatic vinyl compound is more preferred.

When the compound (F1) is an aromatic vinyl compound, specific examples thereof include methylstyrene (e.g., 4-methylstyrene), ethylvinylbenzene, diethyl 4-vinylbenzylphosphonate, 4-vinylbenzylglycidyl ether, α-methylstyrene, 4-tert-butylstyrene, divinylbenzene, 1, 2-bis(4-vinylphenyl)ethane (BVPE), and vinylbenzyl ether, and methylstyrene (e.g., 4-methylstyrene), ethylvinylbenzene, diethyl 4-vinylbenzylphosphonate, 4-vinylbenzylglycidyl ether, and α-methylstyrene are taken as examples.

For specific examples of the compound (F1), reference can be made to the descriptions in paragraphs from 0046 to 0049 of JP 2019-194312 A, the contents of which are incorporated herein.

When the compound (F1) has a vinylene group, acenaphthylene and pyrazylene are preferred as specific examples, and acenaphthylene is more preferred.

In the present specification, a compound that corresponds to a compound having a vinylene group, such as an imidazole compound to be described later, and that is explicitly described as a component (e.g., a curing accelerator) other than the compound having a vinylene group does not correspond to the compound having a vinylene group.

On the other hand, the resin composition according to the present embodiment also preferably contains a styrene oligomer (F2) for improving low dielectric constant and low dielectric loss tangent. The styrene oligomer (F2) according to the present embodiment is preferably formed by polymerizing at least one selected from the group consisting of styrene, the aforementioned styrene derivative, and vinyltoluene. The styrene oligomer (F2) has a number average molecular weight of preferably 178 or more, and preferably 1600 or less. In addition, the styrene oligomer (F2) is preferably a compound having no branched structure in which the average number of aromatic rings is from 2 to 14, the total amount of the styrene oligomer having aromatic rings from 2 to 14 is 50 mass% or more, and the boiling point is 300°C or higher.

Examples of the styrene oligomer (F2) used in the present embodiment include a styrene polymer, a vinyl toluene polymer, an α-methylstyrene polymer, a vinyl toluene-α-methylstyrene polymer, and a styrene-α-styrene polymer. As the styrene polymer, a commercially available product may be used, and examples thereof include picolastic A5 (available from Eastman Chemical Co., Ltd.), picolastic A-75 (available from Eastman Chemical Co., Ltd.), picotex 75 (available from Eastman Chemical Co., Ltd.), FTR-8100 (available from Mitsui Chemicals, Inc.), and FTR-8120 (available from Mitsui Chemicals, Inc.). Examples of the vinyltoluene-α-methylstyrene polymer include Piccotex LC (available from Eastman Chemical Co., Ltd.). Examples of the α-methylstyrene polymer include Crystalex 3070 (abailable from Eastman Chemical Co., Ltd.), Crystalex 3085 (available from Eastman Chemical Co., Ltd.), Crystalex

(3100), Crystalex 5140 (available from Eastman Chemical Co., Ltd.), FMR-0100 (available from Mitsui Chemicals, Inc.), and FMR-0150 (available from Mitsui Chemicals, Inc.). Examples of the styrene-α-styrene polymer include FTR-2120 (available from Mitsui Chemicals, Inc.). These styrene oligomers may be used alone or in combination of two or more kinds thereof.

In the resin composition of the present embodiment, the α-methylstyrene oligomer is preferred because it is thermally cured well, and is excellent in good embeddability of fine wiring, solder heat resistance, low relative permittivity, and low dielectric loss tangent.

In addition, the resin composition according to the present embodiment preferably contains a divinyl compound (F3) as the monomer having an ethylenically unsaturated group in order to improve low dielectric constant and low dielectric loss tangent.

The divinyl compound is a low molecular weight compound having two vinyl groups. Having two vinyl groups results in a favorable crosslinking density that does not become excessively high. As a result, the free volume of the molecule is increased, so that the dielectric loss tangent (Df) of the resulting cured product can be suppressed to a small value. In addition, the divinyl compound (F3) is used as a substitute for the polymaleimide compound (A), the cyanate ester compound (B), and some of the additional resin component (C), and hence it is considered that a reduction in content of a component having a polar group itself also contributes to the reduction of the dielectric loss tangent (Df). It is also considered that since each of the two functional groups of the divinyl compound is a vinyl group, the reactivity with the polymaleimide compound (A) and the cyanate ester compound (B) is improved, and as a result, heat resistance tends to be easily improved.

Here, the divinyl compound (F3) refers to a compound having a molecular weight of less than 195, and the lower limit of the molecular weight is practically 54. Examples of the divinyl compound (F3) include divinylbenzene and 1,3-vinyltetramethylsiloxane.

In addition, for the details of the monomer or oligomer having an ethylenically unsaturated group, reference can be made to the description in paragraphs from 0069 to 0087 of WO 2017/135168 A and in paragraphs from 0065 to 0067 of WO 2019/230945 A, the contents of which are incorporated herein.

In particular, by using the monomer or oligomer having an ethylenically unsaturated group and using an elastomer as the additional resin component (C), a resin composition excellent in moisture absorption heat resistance while maintaining excellent low dielectric properties can be obtained.

In particular, by using the monomer or oligomer having an ethylenically unsaturated group and using a compound containing a vinylaryl group (in particular, a polymer having a structural unit represented by Formula (V)), a resin composition excellent in moisture absorption heat resistance while maintaining excellent low dielectric properties can be obtained.

When the resin composition of the present embodiment contains the monomer or oligomer having an ethylenically unsaturated group, the content thereof is preferably 0.5 parts by mass or more, more preferably 1 part by mass or more, even more preferably 2 parts by mass or more, still more preferably 3 parts by mass or more, and may be 5 parts by mass or more, based on 100 parts by mass of the resin solid content in the resin composition. By setting the content to the aforementioned lower limit or more, low dielectric properties tend to be further improved. The upper limit of the content of the monomer or oligomer having an ethylenically unsaturated group is preferably 30 parts by mass or less, more preferably 25 parts by mass or less, even more preferably 20 parts by mass or less, still more preferably 15 parts by mass or less, and yet more preferably 10 parts by mass or less, based on 100 parts by mass of the resin solid content in the resin composition. By setting the content to the aforementioned upper limit or less, heat resistance tends to be further improved. **In** addition, low dielectric constant, low dielectric loss tangent, and chemical resistance tend to be further improved.

The resin composition of the present embodiment may contain only one kind or two or more of the monomer or oligomer having an ethylenically unsaturated group. When two or more kinds thereof are contained, the total amount thereof is preferably within the aforementioned ranges.

### <Active ester compound>

The resin composition of the present embodiment may contain an active ester compound.

The active ester compound is not particularly limited, and examples thereof include a compound having two or more (preferably from 2 to 12, more preferably from 2 to 6, even more preferably from 2 to 4, still more preferably 2 or 3, and yet more preferably 2) active ester groups in a single molecule.

The active ester compound may be a linear, branched, or cyclic compound. Among these, from the viewpoint of further improving the heat resistance of the resulting cured product, an active ester compound obtained by reacting a carboxylic acid compound and/or a thiocarboxylic acid compound with a hydroxy compound and/or a thiol compound is preferred, an active ester compound obtained by reacting a carboxylic acid compound with one or more compounds selected from the group consisting of a phenolic compound, a naphthol compound, and a thiol compound is more preferred, an aromatic compound obtained by reacting a carboxylic acid compound with an aromatic compound having a phenolic hydroxyl group and having two or more active ester groups in a single molecule is even more preferred, and an aromatic compound obtained by reacting a compound having two or more carboxylic acids in a single molecule with an aromatic compound having a phenolic hydroxyl group and having two or more active ester groups in a single molecule is particularly preferred.

Examples of the carboxylic acid compound include one or more selected from the group consisting of: benzoic acid, acetic acid, succinic acid, maleic acid, itaconic acid, phthalic acid, isophthalic acid, terephthalic acid, and pyromellitic acid. Among these, from the viewpoint of further improving the heat resistance of the resulting cured product, the carboxylic acid compound is preferably one or more selected from the group consisting of succinic acid, maleic acid, itaconic acid, phthalic acid, isophthalic acid, and terephthalic acid, and more preferably one or more selected from the group consisting of isophthalic acid and terephthalic acid.

Examples of the thiocarboxylic acid compound include one or more selected from thioacetic acid and thiobenzoic acid.

Examples of the phenolic compound or naphthol compound include one or more selected from the group consisting of hydroquinone, resorcin, bisphenol A, bisphenol F, bisphenol S, phenolphthalein, methylated bisphenol A, methylated bisphenol F, methylated bisphenol S, phenol, o-cresol, m-cresol, p-cresol, catechol, α-naphthol, β-naphthol, 1,5-dihydroxynaphthalene, 1,6-dihydroxynaphthalene, 2,6-dihydroxynaphthalene, dihydroxybenzophenone, trihydroxybenzophenone, tetrahydroxybenzophenone, phloroglucin, benzenetriol, dicyclopentadienyl diphenol, and phenol novolac. From the viewpoint of further improving the heat resistance and solubility in a solvent of the resulting cured product, the phenolic compound or naphthol compound is preferably bisphenol A, bisphenol F, bisphenol S, methylated bisphenol A, methylated bisphenol F, methylated bisphenol S, catechol, α-naphthol, β-naphthol, 1,5-dihydroxynaphthalene, 1,6-dihydroxynaphthalene, 2,6-dihydroxynaphthalene, dihydroxybenzophenone, trihydroxybenzophenone, tetrahydroxybenzophenone, phloroglucin, benzenetriol, dicyclopentadienyl diphenol, or phenol novolac; more preferably one or more selected from the group consisting of catechol, 1,5-dihydroxynaphthalene, 1,6-dihydroxynaphthalene, 2,6-dihydroxynaphthalene, dihydroxybenzophenone, trihydroxybenzophenone, tetrahydroxybenzophenone, phloroglucin, benzenetriol, dicyclopentadienyl diphenol, and phenol novolac; even more preferably one or more selected from the group consisting of 1,5-dihydroxynaphthalene, 1,6-dihydroxynaphthalene, 2,6-dihydroxynaphthalene, dihydroxybenzophenone, trihydroxybenzophenone, tetrahydroxybenzophenone, dicyclopentadienyl diphenol, and phenol novolac; and particularly preferably one or more selected from the group consisting of dihydroxybenzophenone, trihydroxybenzophenone, tetrahydroxybenzophenone, dicyclopentadienyl diphenol, and phenol novolac (preferably one or more selected from the group consisting of dicyclopentadienyl diphenol and phenol novolac, and more preferably dicyclopentadienyl diphenol).

Examples of the thiol compound include one or more selected from the group consisting of benzenedithiol and triazine dithiol.

From the viewpoint of further improving compatibility with an epoxy compound, the active ester compound is preferably a compound having two or more carboxylic acids in a single molecule and containing an aliphatic chain, and from the viewpoint of further improving heat resistance, the active ester compound is preferably a compound having an aromatic ring. More specific examples of the active ester compound include the active ester compounds described in JP 2004-277460 A.

As the active ester compound, a commercially available product may be used, or the active ester compound may be prepared by a known method. Examples of the commercially available product include compounds containing a dicyclopentadienyl diphenol structure (e.g., such as EXB9451, EXB9460, EXB9460S, and HPC-8000-65T (all available from DIC Corporation)), an acetylated product of phenol novolac (e.g., DC808 (available from Mitsubishi Chemical Corporation)), and a benzoylated product of phenol novolac (e.g., YLH1026, YLH1030, and YLH1048 (all available from Mitsubishi Chemical Corporation)). From the viewpoints of further improving the storage stability of a varnish and the low thermal expansion (of a cured product) when the resin composition is cured, EXB9460S is preferred.

The active ester compound can be prepared by a known method and can be obtained, for example, by a condensation reaction of a carboxylic acid compound with a hydroxy compound. Specific examples thereof include a method in which (a) a carboxylic acid compound or a halide thereof, (b) a hydroxy compound, and (c) an aromatic monohydroxy compound are reacted at a ratio from 0.05 to 0.75 mol of the phenolic hydroxyl group of (b) and from 0.25 to 0.95 mol of (c), based on 1 mol of the carboxy group or the acid halide group of (a).

The active ester compound is preferably contained in a range that does not impair the effects of the present invention. When the resin composition of the present embodiment contains the active ester compound, the content of the active ester compound is preferably 1 part by mass or more and preferably 50 parts by mass or less, based on 100 parts by mass of the resin solid content in the resin composition.

The resin composition of the present embodiment may contain only one kind or two or more kinds of the active ester compound. When two or more kinds thereof are contained, the total amount thereof is preferably within the aforementioned ranges.

The resin composition of the present embodiment may be formed to be substantially free of the active ester compound. The phrase "substantially free" means that the content of the active ester compound is less than 1 part by mass based on 100 parts by mass of the resin solid content in the resin composition, and the content is preferably less than 0.1 parts by mass, and more preferably less than 0.01 parts by mass.

### <Dispersant>

The resin composition of the present embodiment may contain a dispersant. Dispersants commonly used in paints can be suitably used as the dispersant, and the kind of the dispersant is not particularly limited. As the dispersant, a copolymer-based wet dispersant is preferably used, and specific examples thereof include DISPERBYK (trade name) -110, 111, 161, 180, 2009, 2152, and 2155, and BYK (trade name) -W996, W9010, W903, and W940, all of which are available from BYK Japan KK.

When the resin composition of the present embodiment contains a dispersant, the lower limit value of the content thereof is preferably 0.01 parts by mass or more, more preferably 0.1 parts by mass or more, and may be 0.3 parts by mass or more per 100 parts by mass of the resin solid content in the resin composition. The upper limit of the content of the dispersant is preferably 10 parts by mass or less, more preferably 5 parts by mass or less, and even more preferably 3 parts by mass or less relative to 100 parts by mass of the resin solid content in the resin composition.

The dispersant may be used singly or in combination of two or more kinds thereof. When two or more kinds thereof are used, the total amount is within the aforementioned ranges.

### <Curing Accelerator>

The resin composition of the present embodiment may further contain a curing accelerator. The curing accelerator is not particularly limited, and examples thereof include: imidazoles such as 2-ethyl-4-methylimidazole and triphenylimidazole; organic peroxides such as benzoyl peroxide, bis(1-methyl-1-phenylethyl)peroxide), di-t-butyl peroxide, lauroyl peroxide, acetyl peroxide, parachlorobenzoyl peroxide, di-tert-butyl-di-perphthalate, α,α'-di(t-butylperoxy)diisopropylbenzene, 2,5-dimethyl-2,5-di(t-butylperoxy)hexane, and 2,5-dimethyl-2,5-bis(t-butylperoxy)hexine-3; azo compounds such as azobisnitrile (e.g., azobisisobutyronitrile); tertiary amines such as N,N-dimethylbenzylamine, N,N-dimethylaniline, N,N-dimethyltoluidine, 2-N-ethylanilinoethanol, tri-n-butylamine, pyridine, quinoline, N-methylmorpholine, triethanolamine, triethylenediamine, tetramethylbutanediamine, and N-methylpiperidine; phenols such as phenol, xylenol, cresol, resorcin, and catechol; high-temperature decomposition type radical generators such as 2,3-dimethyl-2,3-diphenylbutane; organometallic salts such as lead naphthenate, lead stearate, zinc naphthenate, zinc octylate, manganese octylate, tin oleate, dibutyltin malate, manganese naphthenate, cobalt naphthenate, and iron acetylacetone; products formed by dissolving these organometallic salts in hydroxyl group-containing compounds such as phenol and bisphenol; inorganic metal salts such as tin chloride, zinc chloride, and aluminum chloride; and organotin compounds such as dioctyltin oxide, other alkyltins, and alkyltin oxides.

The curing accelerator is preferably at least one selected from the group consisting of imidazoles, an organic peroxide, and an organometallic salt, more preferably at least one selected from the group consisting of imidazoles and an organometallic salt, and even more preferably an organometallic salt.

In the present embodiment, the resin composition can be formed to be substantially free of a polymerization initiator such as an organic peroxide or an azo compound. The phrase "substantially free" means that the content of the polymerization initiator is less than 0.1 parts by mass based on 100 parts by mass of the resin solid content in the resin composition.

When the resin composition of the present embodiment contains the curing accelerator, the lower limit of the content of the curing accelerator is preferably 0.005 parts by mass or more, more preferably 0.01 parts by mass or more, and even more preferably 0.1 parts by mass or more, based on 100 parts by mass of the resin solid content in the resin composition. The upper limit of the content of the curing accelerator is preferably 10 parts by mass or less, more preferably 5 parts by mass or less, even more preferably 3 parts by mass or less, and still more preferably 2 parts by mass or less, based on 100 parts by mass of the resin solid content in the resin composition. The curing accelerator can be used singly or in combination of two or more kinds thereof. When two or more kinds thereof are used, the total amount is within the aforementioned ranges.

### <Flame Retardant>

The resin composition of the present embodiment may contain a flame retardant. Examples of the flame retardant include a phosphorus-based flame retardant, a halogen-based flame retardant, an inorganic flame retardant, and a silicone-based flame retardant, and a phosphorus-based flame retardant is preferred.

A known flame retardant can be used as the flame retardant, and examples thereof include: halogen-based flame retardants such as a brominated epoxy resin, brominated polycarbonate, brominated polystyrene, brominated styrene, brominated phthalimide, tetrabromobisphenol A, pentabromobenzyl(meth)acrylate, pentabromotoluene, tribromophenol, hexabromobenzene, decabromodiphenyl ether, bis-1,2-pentabromophenylethane, chlorinated polystyrene, and chlorinated paraffin; phosphorus-based flame retardants such as red phosphorus, tricresyl phosphate, triphenyl phosphate, cresyl diphenyl phosphate, trixylenyl phosphate, trialkyl phosphate, dialkyl phosphate, tris(chloroethyl)phosphate, phosphazene, 1,3-phenylene bis(2,6-dixylenyl phosphate), and 10-(2,5-dihydroxyphenyl)-10H-9-oxa-10-phosphaphenanthrene-10-oxide; inorganic flame retardants such as aluminum hydroxide, magnesium hydroxide, partial boehmite, boehmite, zinc borate, and antimony trioxide; and silicone-based flame retardants such as silicone rubber and a silicone resin.

In the present embodiment, among these, 1,3-phenylenebis(2,6-dixylenyl phosphate) is preferred because it does not impair low dielectric properties.

When the resin composition of the present embodiment contains a flame retardant, the content of the flame retardant is preferably 1 part by mass or more, and more preferably 5 parts by mass or more, per 100 parts by mass of the resin solid content in the resin composition. The lower limit of the content of the flame retardant is preferably 25 parts by mass or less, and more preferably 20 parts by mass or less.

The flame retardants can be used singly or in combination of two or more kinds thereof. When two or more kinds thereof are used, the total amount is within the aforementioned ranges.

### <Solvent>

The resin composition of the present embodiment may contain a solvent, and preferably contains an organic solvent. When the resin composition of the present embodiment contains a solvent, the resin composition is in a form (of solution or varnish) in which at least some, or preferably all of the various resin solid contents described aforementioned are dissolved or homogeneously mixed in the solvent. The solvent is not particularly limited as long as it is a polar organic solvent or non-polar organic solvent capable of dissolving or homogeneously mixing at least some, or preferably all of the various resin solid contents described above; examples of the polar organic solvent include ketones (e.g., acetone, methyl ethyl ketone, and methyl isobutyl ketone), cellosolves (e.g., propylene glycol monomethyl ether and propylene glycol monomethyl ether acetate), esters (e.g., ethyl lactate, methyl acetate, ethyl acetate, butyl acetate, isoamyl acetate, ethyl lactate, methyl methoxypropionate, and methyl hydroxyisobutyrate), and amides (e.g., dimethoxy acetamide and dimethylformamide); and examples of the non-polar organic solvent include aromatic hydrocarbons (e.g., toluene and xylene).

The solvent can be used singly or in combination of two or more kinds thereof. When two or more kinds thereof are used, the total amount is within the aforementioned ranges.

### <Other Components>

In addition to the aforementioned components, the resin composition of the present embodiment may contain various polymer compounds such as a thermoplastic resin and an oligomer thereof, and various additives. Examples of the additive include ultraviolet absorbers, antioxidants, photopolymerization initiators, fluorescent whitening agents, photosensitizers, dyes, pigments, thickeners, flow control agents, lubricants, antifoaming agents, leveling agents, brighteners, and polymerization inhibitors. These additives can be used singly or in combination of two or more kinds thereof.

### <Application>

The resin composition of the present embodiment is used as a cured product. Specifically, the resin composition of the present embodiment can be suitably used as a resin composition for electronic materials, such as an insulating layer of printed wiring board or a material of semiconductor package, as it serves as a low relative permittivity material and/or a low dielectric loss tangent material. The resin composition of the present embodiment can be suitably used as a material for a prepreg, a metal foil-clad laminate including the prepreg, a resin composite sheet, and a printed wiring board.

The resin composition of the present embodiment is used in the form of a layered material (intended to include a film-like or sheet-like material), such as a prepreg or a resin composite sheet serving as an insulating layer of a printed wiring board. When the resin composition is formed into such a layered material, the thickness of the layered material is preferably 5 µm or more, and more preferably 10 µm or more. The upper limit of the thickness is preferably 200 µm or less, and more preferably 180 µm or less. The thickness of the layered material means, for example, a thickness including that of a glass cloth when the glass cloth is impregnated with the resin composition of the present embodiment.

The material formed from the resin composition of the present embodiment may be used in an application to form a pattern by exposure and development or may be used in an application not involving exposure and development. In particular, the material is suitable for an application not involving exposure and development.

### <<Prepreg>>

A prepreg of the present embodiment is formed from a substrate (prepreg substrate) and the resin composition of the present embodiment. The prepreg of the present embodiment is produced by, for example, applying the resin composition of the present embodiment to a substrate (e.g., impregnating and/or coating the substrate with the resin composition) and then semi-curing the resin composition by heating (e.g., by a method of drying at from 120 to 220°C for from 2 to 15 minutes). In this case, the amount of the resin composition adhering to the substrate, that is, the amount of the resin composition (including the filler (D)) based on the total amount of the prepreg after semi-curing is preferably in a range from 20 to 99 mass%, and more preferably in a range from 20 to 80 mass%.

The substrate is not particularly limited as long as it is a substrate used in various printed wiring board materials. Examples of the material of the substrate include glass fibers (e.g., E-glass, D-glass, L-glass, S-glass, T-glass, Q-glass, UN-glass, NE-glass, and spherical glass), inorganic fibers other than glass (e.g., quartz), and organic fibers (e.g., polyimide, polyamide, polyester, liquid crystal polyester, and polytetrafluoroethylene). The form of the substrate is not particularly limited, and examples of the form include woven fabric, non-woven fabrics, roving, chopped strand mat, and surfacing mat. These substrates may be used alone or in combination of two or more kinds thereof. Among these substrates, a woven fabric subjected to ultra fiber opening or fine weaving is preferred from the viewpoint of dimensional stability, a glass woven fabric having a thickness of 200 µm or less and a mass of 250 g/m² or less is preferred for the substrate from the viewpoints of strength and low water absorption, and a glass woven fabric having a surface treated with a silane coupling agent such as an epoxysilane or an aminosilane is preferred from the viewpoint of moisture absorption heat resistance. From the viewpoint of electrical properties, a low dielectric glass cloth, made of a glass fiber of L-glass, NE-glass, or Q-glass and exhibiting a low relative permittivity and a low dielectric loss tangent, is more preferred.

Examples of the substrate having a low relative permittivity include a substrate having a relative permittivity of 5.0 or less (preferably from 3.0 to 4.9). Examples of the substrate having a low dielectric loss tangent include a substrate having a dielectric loss tangent of 0.006 or less (preferably from 0.001 to 0.005). The relative permittivity and the dielectric loss tangent are defined as the values obtained by measurement at a frequency of 10 GHz by a perturbation method cavity resonator.

### <<Metal Foil-Clad Laminate>>

The metal foil-clad laminate of the present embodiment includes at least one layer formed from the prepreg of the present embodiment, and a metal foil disposed on one surface or each surface of the layer formed from the prepreg. Examples of the method for producing a metal foil-clad laminate according to the present embodiment include a method for disposing at least one prepreg (preferably, stacking two or more prepregs) of the present embodiment, disposing a metal foil on one or both surfaces of the prepreg, and laminate-molding the metal foil and the prepreg. In more detail, the prepreg can be produced by disposing a metal foil, such as copper or aluminum, on one or both surfaces of the prepreg and laminate-molding the metal foil and the prepreg. The number of the prepregs is preferably from 1 to 10, more preferably from 2 to 10, and even more preferably from 2 to 9.

The metal foil is not particularly limited as long as it is used for a printed wiring board material, and examples thereof include copper foils such as a rolled copper foil and an electrolytic copper foil. The thickness of the metal foil (preferably, a copper foil) is not particularly limited and may be approximately from 1.5 to 70 µm. When a copper foil is used as the metal foil, the surface roughness Rz of the copper foil, measured in accordance with JIS B0601: 2013, is preferably adjusted to from 0.2 to 4.0 µm. By setting the roughness Rz of the surface of the copper foil to 0.2 µm or more, the roughness of the surface of the copper foil becomes an appropriate size, and copper foil peel strength tends to be further improved. On the other hand, by setting the roughness Rz of the surface of the copper foil to 4.0 µm or less, the roughness of the surface of the copper foil becomes an appropriate size, and the dielectric loss tangent of the resulting cured product tends to be further improved. From the viewpoint of copper foil peel strength and dielectric loss tangent, the roughness Rz of the surface of the copper foil is more preferably 0.5 µm or more, even more preferably 0.6 µm or more, particularly preferably 0.7 µm or more, and is more preferably 3.5 µm or less, even more preferably 3.0 µm or less, particularly preferably 2.0 µm or less.

Examples of the laminate-molding method include methods usually used in molding a laminate and a multilayer board for a printed wiring board, and more specific examples include a method of laminate-molding using a multi-stage press, a multi-stage vacuum press, a continuous molding machine, or an autoclave molding machine, under the conditions in which the temperature is approximately from 180 to 350°C, the heating time is approximately from 100 to 300 minutes, and the surface pressure is approximately from 20 to 100 kg/cm² or approximately from 1 to 10 MPa. A multilayer board can also be formed by combining the prepreg of the present embodiment with a separately produced wiring board for an inner layer and laminate-molding them. In a method for manufacturing a multilayer board, a multilayer board can be produced, for example, by disposing copper foils of approximately 35 µm on both sides of a single prepreg of the present embodiment, laminate-molding them by the aforementioned laminate-molding method, then forming an inner layer circuit, subjecting the inner layer circuit to a blackening treatment to form an inner layer circuit board, then alternately disposing the inner layer circuit board and the prepreg of the present embodiment one by one, and further disposing a copper foil on the outermost layer, followed by laminate-molding under the conditions described above preferably under vacuum. The metal foil-clad laminate of the present embodiment can be suitably used as a printed wiring board.

The metal foil-clad laminate of the present embodiment preferably has a low relative permittivity (Dk) measured using a laminate obtained by removing the metal foil by etching. Specifically, the relative permittivity (Dk), at a frequency of 10 GHz measured in accordance with a cavity resonator perturbation method in accordance with JIS C2138: 2007 using a sample obtained by removing the metal foil from the metal foil-clad laminate by etching, is preferably 5.0 or less, and more preferably 4.5 or less. The lower limit of the relative permittivity (Dk) is not particularly specified but is practically, for example, 0.1 or more.

The measurement of the dielectric constant follows the description in Examples to be described later.

The dielectric loss tangent (Df) of the metal foil-clad laminate of the present embodiment, measured using a laminate obtained by removing the metal foil by etching, is preferably low. Specifically, the dielectric loss tangent (Df), at a frequency of 10 GHz measured in accordance with a cavity resonator perturbation method in accordance with JIS C2138: 2007 using a sample obtained by removing the metal foil from the metal foil-clad laminate by etching, is preferably 0.010 or less, more preferably 0.008 or less, and even more preferably 0.007 or less. The lower limit of the dielectric loss tangent (Df) is not particularly specified but is practically, for example, 0.0001 or more.

The measurement of the dielectric loss tangent follows the description in Examples to be described later.

The metal foil peel strength of the metal foil-clad laminate of the present embodiment is preferably high. Specifically, the measured value, obtained in accordance with JIS C6481: 1996, is preferably 0.4 kN/m or more, and more preferably 0.5 kN/m or more. The upper limit of the metal foil peel strength is not particularly limited, but is practically, for example, 1.4 kN/m or less.

As described above, for the resin composition for electronic materials obtained using the resin composition of the present embodiment (resin composition containing a combination of specific components), its cured product can have properties excellent in crack resistance, appearance of the cured product, and low thermal expansion in addition to low dielectric properties (low dielectric loss tangent) and moisture absorption heat resistance.

### <<Printed Wiring Board>>

The printed wiring board of the present embodiment is a printed wiring board including an insulating layer and a conductor layer disposed on a surface of the insulating layer, in which the insulating layer includes at least one of a layer formed from the resin composition of the present embodiment or a layer formed from the prepreg of the present embodiment. Such a printed wiring board can be manufactured by a common method, and the manufacturing method is not particularly limited. Hereinafter, an example of the method for manufacturing the printed wiring board will be described. First, a metal foil-clad laminate, such as the aforementioned copper foil-clad laminate, is prepared. Next, the surface of the metal foil-clad laminate is subjected to an etching treatment to form an inner layer circuit, thereby producing an inner layer substrate. The surface of the inner layer circuit of the inner layer substrate is subjected to a surface treatment for increasing adhesive strength, as necessary. Then, the required number of the aforementioned prepregs are stacked on the surface of the inner layer circuit, a metal foil for an outer layer circuit is further laminated on the outer side thereof, and they are integrally molded by heating and pressurization. In this way, a multilayer laminate is manufactured, in which an insulating layer including a substrate and the cured product of the resin composition is formed between the inner layer circuit and the metal foil for an outer layer circuit. Then, the multilayer laminate is subjected to a drilling process for a through hole or a via hole, and then a plated metal film, for electrically connecting the inner layer circuit and the metal foil for the outer layer circuit, is formed on the wall surfaces of the holes. Further, the metal foil for the outer layer circuit is subjected to an etching process to form the outer layer circuit, thereby manufacturing a printed wiring board.

The printed wiring board manufactured in the aforementioned manufacturing example is configured to include an insulating layer and a conductor layer formed on the surface of the insulating layer, in which the insulating layer is configured to contain the resin composition of the present embodiment described above and/or the cured product thereof. That is, the aforementioned prepreg of the present embodiment (e.g., a prepreg formed from a substrate and the resin composition of the present embodiment impregnated into or coated onto the substrate) and the layer formed from the resin composition on the metal foil-clad laminate of the present embodiment described above serve as the insulating layer of the present embodiment.

The present embodiment also relates to a semiconductor device including the printed wiring board. For details of the semiconductor device, reference can be made to the description in paragraphs from 0200 to 0202 of JP 2021-021027 A, the contents of which are incorporated herein.

In the insulating layer formed of the cured product of the resin composition of the present embodiment, it is preferable to reduce the surface roughness of the insulating layer after roughening treatment. Specifically, the arithmetic average roughness Ra of the surface of the insulating layer after the roughening treatment is preferably 200 nm or less, more preferably 150 nm or less, and particularly preferably 100 nm or less. The lower limit of the arithmetic average roughness Ra is not particularly limited, and can be, for example, 10 nm or more. The arithmetic average roughness Ra of the surface of the insulating layer is measured using a non-contact type surface roughness meter, a VSI mode, and a lens with a magnification of 50.

As the non-contact type surface roughness meter, WYKONT3300 available from Beacon Instruments is used.

### <<Resin Composite Sheet>>

The resin composite sheet of the present embodiment includes a support and a layer formed of the resin composition of the present embodiment disposed on a surface of the support. The resin composite sheet can be used as a build-up film or a dry film solder resist. The method of manufacturing the resin composite sheet is not particularly limited, and examples include a method in which a solution obtained by dissolving the resin composition of the present embodiment in a solvent is coated (applied) to a support and dried to obtain a resin composite sheet.

Examples of the support used herein include, but are not particularly limited to, organic-based film substrates, such as polyethylene films, polypropylene films, polycarbonate films, poly(ethylene terephthalate) films, ethylene-tetrafluoroethylene copolymer films, as well as release films formed by coating a surface of such a film with a release agent, and polyimide films; conductive foils, such as copper foils and aluminum foils; and plate-like supports, such as glass plates, steel use stainless (SUS) plates, and fiber-reinforced plastics (FRPs).

For example, the application method (coating method) involves application of a solution prepared by dissolving the resin composition of the present embodiment in a solvent onto a support by using a bar coater, a die coater, a doctor blade, or a Baker applicator. In addition, a monolayer sheet can also be formed by peeling or etching the support from the resin composite sheet in which the support and the resin composition are laminated after drying. A monolayer sheet can also be produced without using a support by, for example, feeding a solution prepared by dissolving the resin composition of the present embodiment in a solvent into a mold having a sheet-shaped cavity and drying the solution to mold into a sheet shape.

In the production of the monolayer sheet or the resin composite sheet of the present embodiment, the drying conditions for removing the solvent are not particularly limited. However, drying at a low temperature is likely to cause the solvent to remain in the resin composition, and drying at a high temperature allows the curing of the resin composition to proceed. Thus, the drying is preferably performed at a temperature from 20°C to 200°C for from 1 to 90 minutes. The monolayer sheet or resin composite sheet can also be used in an uncured state where the solvent is only dried or can also be used as necessary in a semi-cured (B-staged) state. Further, the thickness of the resin layer in the monolayer sheet or resin composite sheet of the present embodiment can be adjusted by the concentration of the solution of the resin composition used for coating (application) of the present embodiment and the coating thickness, and is not particularly limited. However, in general, as the thickness of the coating increases, the solvent is likely to remain during drying, and thus the thickness is preferably from 0.1 to 500 µm.

### Examples

Hereinafter, the present invention will be described more specifically with reference to Examples. Materials, usage amounts, proportions, processing details, and processing procedures described in the following Examples can be changed as appropriate without departing from the spirit of the invention. Thus, the scope of the present invention is not limited to the following specific examples.

If it is difficult to obtain the measuring instrument or the like used in Examples due to discontinuation or the like, another instrument with equivalent performance can be used for the measurement.

### <Synthesis Example 1 Synthesis of naphthol aralkyl-type cyanate ester compound (SNCN)>

300 g (1.28 mol in terms of OH group) of 1-naphthol aralkyl resin (available from Nippon Steel & Sumikin Chemical Co., Ltd.) and 194.6 g (1.92 mol) (1.5 mol with respect to 1 mol of hydroxy group) of triethyl amine were dissolved in 1800 g of dichloromethane to obtain a solution 1.

125.9 g (2.05 mol) (1.6 mol with respect to 1 mol of hydroxy group) of cyanogen chloride, 293.8 g of dichloromethane, 194.5 g (1.92 mol) (1.5 mol with respect to 1 mol of hydroxy group) of 36% hydrochloric acid, and 1205.9 g of water were stirred while the liquid temperature thereof was maintaining at from -2 to -0.5°C. During the stirring, the solution 1 was poured dropwise over 30 minutes. After the pouring of the solution 1 was completed and the mixture was stirred at the same temperature for 30 minutes, a solution (solution 2), prepared by dissolving 65 g (0.64 mol) (0.5 mol with respect to 1 mol of hydroxy group) of triethylamine in 65 g of dichloromethane, was poured dropwise over 10 minutes. After the pouring of the solution 2 was completed, the mixture was stirred at the same temperature for 30 minutes to complete the reaction.

Thereafter, the reaction solution was allowed to stand to separate an organic phase and an aqueous phase. The obtained organic phase was washed five times with 1300 g of water. The electric conductivity of the wastewater at the fifth washing was 5 µS/cm, and it was confirmed that the ionic compound to be removed was sufficiently removed by the washing with water.

The organic phase after the washing with water was concentrated under reduced pressure and finally concentrated to dryness at 90°C for 1 hour to obtain 331 g of the target naphthol aralkyl-type cyanate ester compound (SNCN) (orange viscous material). The weight average molecular weight of the obtained SNCN was 600. The IR spectrum of the SNCN showed absorption at 2250 cm⁻¹(cyanate ester group) and no absorption of a hydroxy group.

### <Measurement of weight average molecular weight and number average molecular weight>

The weight average molecular weight (Mw) and the number average molecular weight (Mn) were measured by a gel permeation chromatography (GPC) method. The measurement was conducted using a liquid feed pump (LC-20AD available from Shimadzu Corporation), a differential refractive index detector (RID-10A available from Shimadzu Corporation), and a GPC column (GPC KF-801, 802, 803, 804, available from Showa Denko Corporation), and also using tetrahydrofuran as a solvent, a flow rate of 1.0 ml/min, a column temperature of 40°C, and a calibration curve by monodispersed polystyrene.

### Example 1

50 parts by mass of the naphthol aralkyl-type cyanate ester compound (SNCN) obtained in Synthesis Example 1, 50 parts by mass of a polymaleimide compound (NE-X-9500 available from DIC Corporation, corresponding to the polymaleimide compound having the structural unit represented by the above-mentioned Formula (1)), 0.3 parts by mass of DISPERBYK (trade name)-2009 (available from BYK Japan KK) as a dispersant, 1.0 part by mass of DISPERBYK (trade name)-161 (available from BYK Japan KK), 100 parts by mass of fused silica (SC2050-MNU available from Admatechs, average particle diameter: 0.5 µm), and 0.18 parts by mass of manganese octylate (available from Nippon Chemical Industry Co., Ltd.) were mixed, and the solid content was diluted to 65 mass% with methyl ethyl ketone to obtain a varnish. The blending amount of each component indicates the solid content.

This varnish was further diluted with methyl ethyl ketone, applied by impregnation to an E-glass woven fabric (available from Arisawa Mfg. Co., Ltd., product number: 2116) having a thickness of 0.094 mm, and heated and dried at 165°C for 8 minutes to obtain a prepreg (thickness: 0.1 mm) having a resin composition content of 45 mass%.

8 sheets of the obtained prepregs were stacked, an electrolytic copper foil (3EC-M3-VLP, available from Mitsui Mining & Smelting Corporation) having a thickness of 12 µm was disposed on each surface, and vacuum pressing was performed at a pressure of 30 kgf/cm² and a temperature of 220°C for 120 minutes to obtain a copper foil-clad laminate having an insulating layer thickness of 0.8 mm as a metal foil-clad laminate.

The obtained copper foil-clad laminate was evaluated for thermal expansion coefficient, dielectric constant (Dk), dielectric loss tangent (Df), chemical resistance, and desmear resistance.

### (1) Coefficient of thermal expansion

According to the thermo-mechanical analysis (TMA) method defined in JIS C 6481, a coefficient of linear thermal expansion for a sample obtained by cutting the laminate A into a size of 30 mm × 30 mm were measured in the plane direction (CTE-XY@TMA) and the vertical direction (CTE-Z@TMA) of glass cloth. Specifically, a sample obtained by cutting the laminate A into 30 mm × 30 mm was heated in a thermostatic chamber at 220°C for 2 hours to remove the stress due to molding. Thereafter, the temperature was raised from 40°C to 320°C at 10°C per minute using a thermomechanical analyzer (available from TA Instruments), and the coefficient of linear thermal expansion (CTE) (ppm/°C) at from 60°C to 260°C was measured.

Evaluation was made as follows:
A: 18 or less
B: 18 or more and 28 or less
C: 28 or more

### (2) Dielectric constant (Dk) and dielectric loss tangent (Df)

The copper foil of the copper foil-clad laminate obtained above was removed by etching to obtain a laminate A.

The laminate A was dried at 120°C for 60 minutes, and then a dielectric constant (Dk) and a dielectric loss tangent (Df) after drying were measured at a frequency of 10 GHz using a perturbation method cavity resonator. The measurement temperature was 23°C.

The perturbation method cavity resonator was Agilent8722ES available from Agilent Technologies.

Evaluation was made as follows.
<Dk>
   A: 4.2 or less
   B: less than 4.2 and 4.5 or less
   C: less than 4.5
<Df>
   A: 0.0055 or less
   B: more than 0.0055 and 0.0085 or less
   C: more than 0.0085

### (3) Chemical resistance

A sample obtained by cutting the laminate A into 50 mm × 50 mm was immersed in a hydrochloric aqueous solution at 60°C, which was adjusted to 4N, for 60 minutes. A mass reduction amount (mass%) was calculated from the mass of the laminate A before and after the immersion. A lower absolute value indicates being more excellent in chemical resistance (acid resistance).

A sample obtained by cutting the laminate A into 50 mm × 50 mm was immersed in a sodium hydroxide aqueous solution at 70°C, which was adjusted to 1N, for 60 minutes. A mass reduction amount (mass%) was calculated from the mass of the laminate A before and after the immersion. A lower absolute value indicates being more excellent in chemical resistance (alkali resistance).

Evaluation was made as follows:
S: 0.15 mass% or less
A: more than 0.15 mass% and 0.4 mass% or less
B: more than 0.4 mass% and 1.0 mass% or less
C: more than 1.0 mass%

### (4) Desmear resistance

The laminate A was subjected to the following immersion treatment. First, the laminate A was immersed in a swelling solution (Swelling Dip Securigant P available from Atotech Japan Co., Ltd.) at 80°C for 10 minutes. Next, the immersed laminate A was immersed in a roughening solution (available from Atotech Japan Co., Ltd., Concentrate Compact CP) at 80°C for 5 minutes. Next, the immersed laminate A was immersed in a neutralizing solution (available from Atotech Japan Co., Ltd., Reduction Conditioner Securiganth P500) at 45°C for 10 minutes. After the immersion treatment was performed three times, a mass reduction rate (mass%) of the laminate A was measured.

Evaluation was made as follows:
A: 1.5 mass% or less
B: more than 1.5 mass% and 3.0 mass% or less
C: higher than 3.0 mass%

### Comparative Example 1

The same procedure as in Example 1 was carried out except that the polymaleimide compound (NE-X-9500 available from DIC Corporation, corresponding to the polymaleimide compound having the structural unit represented by Formula (1)) was replaced with the same amount of BMI-2300 (phenylmethane maleimide available from Daiwakasei Industry Co., LTD.), the blending amount of the manganese octylate was changed to 0.15 parts by mass, and the heating and drying time after the obtained varnish was impregnated and coated onto the NE-glass woven fabric having a thickness of 0.1 mm was changed to 10 minutes.

### Example 2

The same procedure as in Example 1 was carried out, except that the content of the naphthol aralkyl-type cyanate ester compound (SNCN) obtained in Synthesis Example 1 was changed from 50 parts by mass to 20 parts by mass, and the content of the polymaleimide compound (NE-X-9500 available from DIC Corporation, corresponding to the polymaleimide compound having the structural unit represented by the above-mentioned Formula (1)) was changed from 50 parts by mass to 80 parts by mass.

### Example 3

The same procedure as in Example 1 was carried out, except that the content of the naphthol aralkyl-type cyanate ester compound (SNCN) obtained in Synthesis Example 1 was changed from 50 parts by mass to 80 parts by mass, and the content of the polymaleimide compound (NE-X-9500 available from DIC Corporation, corresponding to the polymaleimide compound having the structural unit represented by Formula (1)) was changed from 50 parts by mass to 20 parts by mass.

**[Table 1]**

| | | Example 1 | Comparative Example 1 | Example 2 | Example 3 |
|---|---|---|---|---|---|
| CTE-XY@TMA (ppm/°C) | | A | A | A | A |
| CTE-Z@TMA (ppm/°C) | | A | B | A | A |
| Dk (-) | | A | A | A | A |
| Df (-) | | A | B | A | A |
| Chemical resistance (%) | 4N-HCl 60 min treatment | A | A | A | A |
| | 1N-NaOH 60 min treatment | S | B | S | A |
| Desmear resistance (%) | Three times | A | B | A | A |

## Claims

1. A resin composition comprising a polymaleimide compound (A) and a cyanate ester compound (B), the cyanate ester compound (B) comprising, in a molecule, two or more aromatic moieties substituted with at least one cyanato group,
wherein the polymaleimide compound (A) comprises, as reaction raw materials (1), an aromatic amine compound (a1) containing one or more and three or less alkyl groups on an aromatic ring, an aromatic divinyl compound (a2) containing two ethenyl groups, and maleic anhydride.

2. The resin composition according to claim 1, wherein, a content of the polymaleimide compound (A) is from 1 to 90 parts by mass, relative to 100 parts by mass of a resin solid content in the resin composition.

3. The resin composition according to claim 1 or 2, a content of the cyanate ester compound (B) comprising, in a molecule, two or more aromatic moieties substituted with at least one cyanato group is from 1 to 90 parts by mass, relative to 100 parts by mass of a resin solid content in the resin composition.

4. The resin composition according to claim 1 or 2, wherein the cyanate ester compound (B) comprising, in a molecule, two or more aromatic moieties substituted with at least one cyanato group comprises at least one selected from the group consisting of a phenol novolac-type cyanate ester compound, a naphthol aralkyl-type cyanate ester compound, a naphthylene ether-type cyanate ester compound, a biphenyl aralkyl-type cyanate ester compound, a xylene resin-type cyanate ester compound, a trisphenol methane-type cyanate ester compound, an adamantane skeleton-type cyanate ester compound, a bisphenol M-type cyanate ester compound, and a bisphenol A-type cyanate ester compound.

5. The resin composition according to claim 1 or 2, further comprising one or more additional resin components (C) selected from the group consisting of a maleimide compound other than the polymaleimide compound (A), a compound containing a polymerizable carbon-carbon unsaturated double bond, an epoxy compound, a phenolic compound, an oxetane resin, a benzoxazine compound, and a thermoplastic elastomer.

6. The resin composition according to claim 1 or 2, further comprising a filler (D).

7. The resin composition according to claim 6, wherein the filler (D) comprises one or more selected from the group consisting of silica, aluminum hydroxide, aluminum nitride, boron nitride, forsterite, titanium oxide, barium titanate, strontium titanate, and calcium titanate.

8. The resin composition according to claim 6, wherein a content of the filler (D) in the resin composition is from 1 to 1600 parts by mass, based on 100 parts by mass of a resin solid content in the resin composition.

9. The resin composition according to claim 1 or 2, further comprising from 0.5 to 30 parts by mass of a monomer or oligomer having an ethylenically unsaturated group, based on 100 parts by mass of a resin solid content in the resin composition.

10. The resin composition according to claim 1 or 2, further comprising a flame retardant, wherein the flame retardant contains a phosphorus-based flame retardant.

11. The resin composition according to claim 1,
wherein, a content of the polymaleimide compound (A) is from 1 to 90 parts by mass, relative to 100 parts by mass of a resin solid content in the resin composition;
a content of the cyanate ester compound (B) comprising, in a molecule, two or more aromatic moieties substituted with at least one cyanato group is from 1 to 90 parts by mass, relative to 100 parts by mass of a resin solid content in the resin composition;
the cyanate ester compound (B) comprising, in a molecule, two or more aromatic moieties substituted with at least one cyanato group comprises at least one selected from the group consisting of a phenol novolac-type cyanate ester compound, a naphthol aralkyl-type cyanate ester compound, a naphthylene ether-type cyanate ester compound, a biphenyl aralkyl-type cyanate ester compound, a xylene resin-type cyanate ester compound, a trisphenol methane-type cyanate ester compound, an adamantane skeleton-type cyanate ester compound, a bisphenol M-type cyanate ester compound, and a bisphenol A-type cyanate ester compound,
the resin composition further comprises a filler (D),
the filler (D) comprises one or more selected from the group consisting of silica, aluminum hydroxide, aluminum nitride, boron nitride, forsterite, titanium oxide, barium titanate, strontium titanate, and calcium titanate, and
a content of the filler (D) in the resin composition is from 1 to 1600 parts by mass, based on 100 parts by mass of the resin solid content in the resin composition.

12. The resin composition according to claim 1, 2, or 11, wherein the polymaleimide compound (A) comprises, as reaction raw materials (3), an intermediate amine compound (C) in which the aromatic amine compounds (a1) are linked to each other via the aromatic divinyl compound (a2), and the maleic anhydride.

13. The resin composition according to claim 12, wherein an amine equivalent of the intermediate amine compound (C) is in a range from 172 to 400 g/equivalent.

14. The resin composition according to claim 1, 2, or 11, wherein the reaction raw materials (1) further comprise an aromatic monovinyl compound (a3) having one ethenyl group.

15. The resin composition according to claim 1, 2, or 11, wherein the polymaleimide compound (A) comprises a structural unit represented by Formula (1) described below: 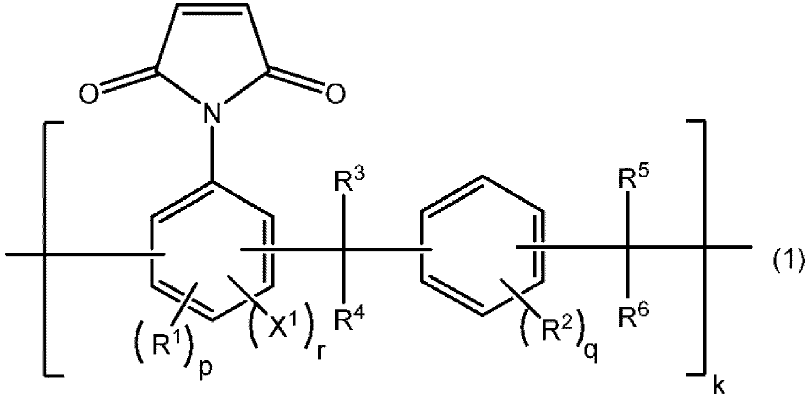 where in Formula (1), R¹ each independently represents an alkyl group having from 1 to 10 carbon atoms, R² each independently represents an alkyl group, an alkoxy group, or an alkylthio group each having from 1 to 10 carbon atoms; an aryl group, an aryloxy group, or an arylthio group each having from 6 to 10 carbon atoms; a cycloalkyl group having from 3 to 10 carbon atoms; a halogen atom; a hydroxyl group; or a mercapto group;
R³, R⁴, R⁵, and R⁶ each independently represent a hydrogen atom or a methyl group, for R³ and R⁴, one is a hydrogen atom and the other is a methyl group, and for R⁵ and R⁶, one is a hydrogen atom and the other is a methyl group;
X¹ represents a substituent represented by Formula (x) described below: 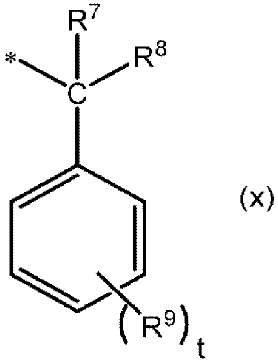 where in Formula (x), R⁷ and R⁸ each independently represent a hydrogen atom or a methyl group, for R⁷ and R⁸, one is a hydrogen atom and the other is a methyl group, R⁹ each independently represents an alkyl group, an alkoxy group, or an alkylthio group each having from 1 to 10 carbon atoms; an aryl group, an aryloxy group, or an arylthio group each having from 6 to 10 carbon atoms; a cycloalkyl group having from 3 to 10 carbon atoms; a halogen atom; a hydroxyl group; or a mercapto group, and t represents an integer from 0 to 4; and
r is an average value of the numbers of X¹ substituents per benzene ring to which X¹ is bonded and represents a number from 0 to 4, p represents an integer from 1 to 3, q represents an integer from 0 to 4, and k represents an integer from 1 to 100.

16. The resin composition according to claim 1, 2, or 11 for a printed wiring board.

17. A cured product of the resin composition according to claim 1, 2, or 11.

18. A prepreg formed from a substrate and the resin composition according to claim 1, 2, or 11.

19. A metal foil-clad laminate comprising at least one prepreg described in claim 18 and a metal foil disposed on one side or both sides of the prepreg.

20. A resin composite sheet comprising a support and a layer disposed on a surface of the support, the layer formed from a resin composition according to claim 1, 2, or 11.

21. A printed wiring board comprising an insulating layer and a conductor layer disposed on a surface of the insulating layer, wherein
the insulating layer includes a layer formed from a resin composition according to claim 1, 2, or 11.
